# EUROPEAN PATENT SPECIFICATION

(11) **EP 3 547 992 B1**
(45) Date of publication and mention of the grant of the patent: **30.12.2020**
(21) Application number: 17816569.2
(22) Date of filing: 30.11.2017
(51) Int. Cl.: A61K 8/34, A61Q 15/00, A61Q 19/10, A61Q 5/00, A61Q 5/12, A61Q 9/02, A61Q 17/00, A61Q 19/00, A61K 8/04, A61K 8/06

(54) **MIXTURES COMPRISING A PHENYL ALKANOL AND A LINEAR ALCOHOL WITH 8 TO 12 CARBON ATOMS**
PHENYLALKANOL UND LINEAR ALKOHOL MIT 8 BIS 12 KOHLENSTOFFATOMEN ENTHALTENDE GEMISCHE.
MELANGES À BASE DE PHÉNYL ALCANOLS ET D'ALCOOLS LINEAIRES AYANT DE 8 À 12 ATOMES DE CARBONES

(30) Priority: 01.12.2016 WO PCT/EP2016/079515; 06.12.2016 WO PCT/EP2016/079964
(43) Date of publication of application: 09.10.2019
(73) Proprietor: Symrise AG, 37603 Holzminden (DE)
(72) Inventor: PESARO, Manuel, 1032 Romanel-sur-Lausanne (CH); LANGE, Sabine, 37603 Holzminden (DE); SCHADE, Vanessa, 37627 Deensen (DE); DIESING, Beate, 37170 Uslar (DE); GRIEGER, Jessica, 37627 Lenne (DE); EBBECKE, Jan, Peter, 37620 Halle (DE)
(74) Representative: Fabry, Bernd
(86) International application number: PCT/EP2017/080895
(87) International publication number: WO 2018/100018

(56) References cited:
- EP-A1- 3 045 161
- WO-A1-2014/009157
- WO-A1-2016/123308
- DE-A1-102012 222 692
- US-A- 1 934 005
- DATABASE GNPD [Online] MINTEL; October 2014 (2014-10), "Comforting Shampoo with Silk Protein", XP002766376, Database accession no. 2709313
- ROBERT D.VOLD, KASHMIRI L.MITTAL: "The Effect of Lauryl Alcohol on the Stability of Oil-in-Water Emulsions", JOURNAL OF COLLOID AND INTERFACE SCIENCE, vol. 38, no. 2, February 1972 (1972-02), pages 451-459, XP002766377,

## Description

### FIELD OF INVENTION

The present invention belongs to the area of cosmetic and household applications and refers to improved stabilization of cosmetic and household formulations by addition of a mixture comprising at least a phenyl alkanol and at least a linear alcohol.

### STATE OF THE ART

Typically, cosmetic compositions comprise a multitude of components. Looking at the listing of ingredients for an average night cream one can find up to 20 positions and there has been a tendency to add as many ingredients as possible in order to address many different issues, all at the same time. As a matter of fact, many consumers equal complexity of a composition with benefit and are accepting high prices, since (s)he expects also high performance. On the other hand, the more complex a composition becomes, the more difficult it is to avoid negative interactions between the components. A major problem for cosmetic compositions, of course in particular for all types of emulsions, is still their stability, especially in case they are subjected to difficult storage conditions, like high or low temperatures. An improved emulsion stability can be achieved by reducing the oil droplets of emulsion.

Another object is related to the sensorial profile. Spreadability, afterfeel and smell are important parameters contributing to the overall liking of a cosmetic formulation after application to skin:

The faster the oil bodies of an emulsion are spread on the skin the better is the perception of the customer. The spreading behaviour of a formulation - and therefore its sensorial profile - is linked to the average particle size of the droplets in the composition. The smaller the droplets are, the faster the spreading is. As a consequence, there is still a need for additives allowing to shift the average particle size distribution to lower values.

Another problem especially for shampoo compositions is foam stability and viscosity.

In the context of the present invention reference is made to WO 2011 141110 A2 (MERCK) disclosing a composition for improved protection against photodecomposition using specific alicyclic compounds. Example 1 discloses a composition comprising 1 to 4 % 4-MAP in combination with oil bodies, emulsifiers and actives.

A further aspect refers to fragrances. Cosmetic and household products are often perfumed to meet the requirements of the consumers. The important functions of fragrances are to provide pleasant odours, to mask the base smell of the product, and also to give the product an odour identity.

However, since fragrances are usually poorly water-soluble or insoluble compounds it is often difficult to perfuming the products. The use of emulsifiers and surfactants is nowadays a standard solubilisation technique to improve the solubility of fragrances in preparations and formulations. But their use as solubilizing agents often leads to further different problems, such as causing cloudiness and turbidity in transparent formulations, skin irritation, and sensitization to light. Furthermore, another big problem in the use of fragrances in products is their high loss rate and the rapid decreases during storage due to volatilization, evaporation, oxidation and poor stability.

A further substance class, which shows the same solubility problems in cosmetic and household products like fragrances and flavors, are lipophilic compounds. A large number of lipophilic molecules have very low solubility in (cosmetic) solvents and have a tendency to recrystallize or separate rapidly. It is known in the art to combine certain compounds, like e.g. DHEA (Dehydroepiandrosteron), in order to solve the solubility problems of lipophilic compounds. Further, it is known practice to encapsulate lipophilic compounds in micelles of block copolymers, for example poly(ethylene oxide-propylene oxide) diblock or triblock copolymers to solubilize the lipophilic compounds. However, these block copolymers do not satisfactorily dissolve the lipophilic compound.

Thus, there is still a big demand for compounds and substances which show the ability to improve the solubility of fragrances and flavors and other lipophilic compounds in general, and thus improving the stability of the preparation, e.g. against flocculation, coalescence or creaming.

Therefore, the object of the present invention has been to identify a multi-functional additive for cosmetic and household formulations, which does not negatively interact with other ingredients while improving the stability, in particular in aspects of viscosity, foam stability, concerning soap bars the cracking stability and in overall the sensorial profile of the cosmetic or household compositions/formulations containing this additive.

The invention further objects to identify an additive which, in particular, have the mentioned properties in regard to flavors and fragrances and at the same time show additional improving characters concerning the solubility of lipophilic components in general, e.g. plant oils, neutral oils and fatty acid esters, especially in water-ethanol based or water-based formulation, and thus contribute to avoidance of phase separation and instability of the compositions/formulations.

### DESCRIPTION OF THE INVENTION

Object of the present invention is a mixture comprising or consisting essentially of
a) at least one phenyl alkanol as defined in claim 1
b) at least one primary, monohydric, linear alcohol with 8 to 12 carbon atoms, and optionally
c) one or more diols.

It has surprisingly found that the stability of cosmetic and household formulations can be improved by addition of one, two, three or more phenyl alkanol (compound a) and one, two, three or more linear alcohols (compound b) and optionally one, two, three or more diols (compound c).

Phenyl alkanols in the sense of the present invention are selected from the group consisting of 2-benzylheptanol (2-benzylheptan-1-ol), 2-methyl 5-phenylpentanol, dimethyl phenylbutanol (2-methyl-4-phenylbutan-2-ol), dimethyl phenylpropanol (2,2-dimethyl-3-phenylpropanol), 3-methyl-4-phenylbutan-2-ol (2-methyl-4-phenyl-2-butanol) or mixtures thereof. In particular preferred is 2-benzylheptanol.

Primary, monohydric, linear alcohols in the sense of the present invention are linear alcohols with 8 to 12 carbon atoms and are preferably selected from the group consisting of undecan-1-ol, decan-1-ol, nonan-1-ol, octan-1-ol, 1-dodecanol or mixtures thereof. Particularly preferred is 1-dodecanol.

Diols in the sense of the present invention are selected from the group consisting of 1,2-diols, derivatives of glyceryl esters or glyceryl ethers or mixtures thereof. Preferably, the diols are selected from the group consisting of 1,2-pentanediol, 1,2-hexanediol, 1,2-octanediol, 1,2-decanediol, 1,2-dodecanediol, glyceryl monocaprylate (2,3-dihydroxy-propyl octanoate), glyceryl monocaprate (2,3-dihydroxypropyl decanoate), glyceryl monolaurate (2,3-Dihydroxypropyl dodecanoate), ethylhexylglycerine (3-[(2-ethylhexyl)oxy]-1,2-propanediol) or mixtures thereof. In particular preferred is 1,2-decanediol and /or ethylhexylglycerine.

Surprisingly, it has been observed that the mixture according to the present invention serve the above mentioned needs simultaneously to improve stability:

Adjunction of the mixture according to the invention
- leads to the formation of smaller oil droplets and decreases particle sizes of oil droplets and thus improves stability of especially emulsions. Due to the fact that the oil or water droplets are more finely divided, the sensation on skin is improved. For example, the greasy afterfeel of creams and lotions can be reduced in that way. The deodorants can also have a smoother feeling and a more creamy feeling when applied to the skin.
- rises foam volume, thus stability and structure of the foam is improved and furthermore the viscosity of the formulation is increased, particularly in liquid surfactant-based formulations.
- solubilizes lipophilic components in water-alcohol, preferably water-ethanol based formulations or in water-based formulations and water-surfactant based products, wherein the lipophilic components are such as lipids, fatty acid esters, polymers, plant oils, neutral oils or any molecules, such as actives and emollients, which have lipophilic character. The lipophilic components may also be lipophilic flavors or fragrances (e.g. an essential oil) or any other lipophilic substances with a HLB value of lower than 20, preferably a HLB value lower than 15, more preferably lower than 10.
- especially prevents cracking of soap bars.

In a preferred embodiment the mixture of the present invention comprises or consists of one, two or three compounds a) selected from the group consisting of 2-benzylheptanol (2-benzylheptan-1-ol), 2-methyl 5-phenylpentanol, dimethyl phenylbutanol (2-methyl-4-phenylbutan-2-ol), dimethyl phenylpropanol (2,2-dimethyl-3-phenylpropanol).

In a preferred embodiment the mixture of the present invention comprises or consists of one, two or three compounds b) selected from the group consisting of decan-1-ol, nonan-1-ol, octan-1-ol, 1-dodecanol.

In a preferred embodiment the mixture of the present invention comprises or consists of one, two or three compounds c) selected from the group consisting of 1,2-pentanediol, 1,2-hexanediol, 1,2-octanediol, 1,2-decanediol, glyceryl monocaprylate (1,3-dihydroxy-2-propanyl octanoate), glyceryl monocaprate, glyceryl monolaurate (2,3-Dihydroxypropyl dodecanoate), ethylhexylglycerin (3-[(2-ethylhexyl)oxy]-1,2-propanediol).

In a preferred embodiment the mixture of the present invention comprises or consists essentially of 2-benzylheptanol (2-benzylheptan-1-ol) as compound a) 1-dodecanol as compound b). Advantageously, this combination of compounds a) and b) is sufficient to provide the above improvements to a series of cosmetic or household formulations.

In a preferred embodiment the mixture of the present invention comprises or consists essentially of 2-benzylheptanol (2-benzylheptan-1-ol) as compound a) 1-dodecanol as compound b) and 1,2-decanediol as compound c). Advantageously, this combination of compounds a), b) and c) provides a particular positive effect on the stability of a series of cosmetic or household formulations.

In a further preferred embodiment compound c) may be a mixture of at least two different 1,2-alkane diols selected from:
a) 1,2-pentanediol and 1,2- octanediol,
b) 1,2-pentanediol and 1,2- decanediol,
c) 1,2-hexanediol and 1,2- octanediol, or
d) 1,2-hexanediol and 1,2- decanediol.

In a particular preferred embodiment the mixture of the present invention comprises or consists essentially of 2-benzylheptanol (2-benzylheptan-1-ol) as compound a) 1-dodecanol as compound b) and as compound c) a combination selected from
a) 1,2-pentanediol and 1,2- octanediol,
b) 1,2-pentanediol and 1,2- decanediol,
c) 1,2-hexanediol and 1,2- octanediol, or
d) 1,2-hexanediol and 1,2- decanediol.

Advantageously, a combination of at least two different 1,2-alkane diols provides additional positive effects on the stability of cosmetic or household formulations.

In a preferred embodiment the mixture of the present invention comprises or consists essentially of:
i) from 1% by weight to 50% by weight, preferably from 5% by weight to 40% by weight, more preferred from 10% by weight to 35% by weight of compound a),
ii) from 50% by weight to 99% by weight, preferably from 60% by weight to 95% by weight, more preferred from 65% by weight to 90% by weight of compound b);
iii) in case compound c) is present: from 1% by weight to 50% by weight, preferably from 5% by weight to 40% by weight, more preferred from 10% by weight to 35% by weight of compound c),
on condition that the amounts of compound a) (phenyl alkanol) and compound b) (primary, monohydric, linear alcohol) and, if present compound c) (diol) add together to 100% b.w.

The mixture of the present invention shows to be particularly effective, in case the compounds are used in the above conventration ranges.

A further preferred mixture of the present invention comprises or consists of
a) at least one phenyl alkanol as described above,
b) at least one primary, monohydric, linear alcohol with 8 to 12 carbon atoms,
   and optionally
c) one or more diols,
d) one or more additional ingredient selected from e.g 2-phenoxyethanol or another suitable solvent or carrier like benzyl alcohol, propylene glycol, dipropylene glycol, 1,3 propanediol, glycerin, butylene glycol.

Such a preferred mixture of the present invention comprises or consists of
i) from 5% by weight to 15% by weight, preferably from 8% by weight to 12% by weight 2-benzylheptanol,
ii) from 30% by weight to 50% by weight, preferably from 35% by weight to 45% by weight 1-dodecanol,
iii) from 5% by weight to 15% by weight, preferably from 8% by weight to 12% by weight of 1,2-decanediol or a mixture of at least two different 1,2-alkane diols as described above,
   and
iv) from 30% by weight to 50% by weight, preferably from 35% by weight to 45% by weight of e.g 2-phenoxyethanol or another suitable solvent or carrier like benzyl alcohol, propylene glycol, dipropylene glycol, 1,3 propanediol, glycerin, butylene glycol
on condition that the amounts of the compounds add together to 100% b.w.

Advantageously, such a mixture serves to fulfil a series of all the above mentioned needs simultaneously to improve stability of cosmetic or household formulations

Another aspect of the invention is the use of a mixture according to the present invention in a cosmetic or household formulation to improve the stability of cosmetic or household formulation. It has been surprisingly shown, that the mixture of the present invention should essentially be present to stabilize cosmetic or household formulations.

The mixture of the present invention can be used in the form of a semi-finished product for the production of cosmetic and household compositions and formulations in order to achieve improved stability of the formulations. A semi-finished product is usually not the end-product, but could be easily mixed in the final product for benefits reasons.

In a preferred embodiment of the above use, the stability improvement is selected from
i) emulsion stability,
ii) foam and viscosity stability,
iii) solubility of lipophilic components,
iv) soap bar stability.

In particular, the present invention refers to the use of a combination of at least one phenyl alkanol and at least one primary, monohydric, linear alcohol with 8 to 12 carbon atoms, particularly the ones described above, for improving
i) emulsion stability,
ii) foam and viscosity stability,
iii) solubility of lipophilic components,
iv) soap bar stability
in a cosmetic or household formulation.

Preferably the stabilization effects based on the mixture of the present invention is not grounded through photostabilisation, antioxidant, antimicrobial or any chemical effects.The stabilization in the sense of the present invention is based on the synergistically properties of the compounds a) and b) and in some cases in the present of compound c).

In particular, the stabilization meant herein is directed to a physically stabilization by the use of the mixture of the present invention, particularly combination of compound a) and compound b), and optionally compound c) and not a chemical stabilization in which molecules used to undergo chemical reactions, such as functions as protective groups or the capture of radicals.

The mixture of the present invention used herein stabilizes the cosmetic or household formulations, as described above, by keeping the physical homogeneity.

In a preferred embodiment of the above use compound a) is 2-benzylheptanol (2-benzylheptan-1-ol), compound b) is 1-dodecanol and compound c) is, if present, 1,2-decanediol or a mixture of at least two different 1,2-alkane diol as described above.

Surprisingly, the mixture according to the present invention has been shown to be especially stabilising for water-based formulations, in particular, aqueous surfactant based formulations and o/w and w/o emulsions. Especially preferred water-based formulations are such as mouthwashes, after shaves, face cleaners, shampoos, shower gels, alcoholic and non-alcoholic deo sprays, household cleaners, and liquid detergents, body lotions, creams, deo microemulsions, skin cleansing emulsions.

### COSMETIC AND HOUSEHOLD COMPOSITIONS/FORMULATIONS

Another aspect of the present invention covers cosmetic or household compositions and formulations comprising a working amount of the mixture of the present invention.

The total amount of the mixture of the present invention in a cosmetic or household composition and formulation which is the working amount is preferably from 0.01% b.w. to 5% b.w., more preferably from 0.02% b.w. to 2% b.w., most preferred from 0.1% b.w. to 1% b.w.- calculated on the final cosmetic or household composition, respectively formulation.

Cosmetic and household formulation containing the mixture of the present invention comprises or consists essentially of total amounts of the individual compounds a), b) and c) preferably in amounts of:
i) from 0.01% by weight to 2% by weight, preferably 0.01% by weight to 0.5% by weight, more preferred 0.01% by weight to 0.1% by weight of compound a),
ii) from 0.02% by weight to 2% by weight, preferably 0.02% by weight to 1% by weight, more preferred 0,02 % by weight to 0.4% by weight of compound b);
iii) in case compound c) is present: from 0.01% by weight to 5% by weight, preferably 0.01% by weight to 3% by weight, more preferred 0.01% by weight to 0.5% by weight of compound c),
on condition that the amounts add - optionally together with water and other ingredients of the cosmetic and household composition, e.g. surfactants, solubilizers, alcohols, glycols, etc. - to 100% b.w.

Advantageously, using the compounds a) to c) in the concentration ranges described above has benefits for a series of the above mentioned needs, namely to improve stability for both cosmetic and household compositions and formulations.

Thus an aspect of the present invention is a cosmetic composition, respectively formulation comprising or consisting of
(a) from 0.1% b.w. to 5% b.w., preferably from 0.1% b.w. to 2% b.w., more preferably from 0.1% b.w. to 1% b.w. of the mixture of the present invention;
(b) from 0% b.w. to 5% b.w., preferably from 0.01% b.w. to 5% b.w. fragrance or flavouring agents,
(c) from 0% b.w. to 10% b.w., preferably from 0.05% b.w. to 8% b.w.lipophilic components,
(d) from 40% b.w. to 99% b.w., preferably from 50% b.w. to 98% b.w. water,
(e) from 0% b.w. to 25% b.w., preferably from 0.5% b.w. to 20% b.w. emulsifiers or surfactants,
   and optionally additionally
(f) from 0% b.w. to 50% b.w., preferably from 0.1% b.w. to 45% b.w., more preferably from 2% b.w. to 40% b.w. alcohol,
(g) from 0% b.w. to 45% b.w., preferably from 2% b.w. to 40% b.w. oil bodies and/or waxes;
(h) from 0% b.w. to 25% b.w., preferably from 0.01% b.w. to 20% b.w. active principles;
on condition that the amounts add - optionally together with fadditional ingredients - to 100% b.w.

The cosmetic compositions/formulations of the present invention may contain various additional ingredients, which are usable for the intended use. Such as abrasives, antiacne agents, agents against ageing of the skin, anticellulitis agents, antidandruff agents, anti-inflammatory agents, irritation-preventing agents, irritation-inhibiting agents, antioxidants, astringents, perspiration-inhibiting agents, antiseptic agents, antistatics, binders, buffers, carrier materials, chelating agents, cell stimulants, cleansing agents, care agents, depilatory agents, surface-active substances, deodorizing agents, antiperspirants, softeners, emulsifiers, enzymes, essential oils, fibres, film-forming agents, fixatives, foam-forming agents, foam stabilizers, substances for preventing foaming, foam boosters, gelling agents, gel-forming agents, hair care agents, hair-setting agents, hair-straightening agents, moisture-donating agents, moisturizing substances, moisture-retaining substances, bleaching agents, strengthening agents, stain-removing agents, optically brightening agents, impregnating agents, dirt-repellent agents, friction-reducing agents, lubricants, moisturizing creams, ointments, opacifying agents, plasticizing agents, covering agents, polish, gloss agents, polymers, powders, proteins, re-oiling agents, abrading agents, silicones, skin-soothing agents, skin-cleansing agents, skin care agents, skin-healing agents, skin-lightening agents, skin-protecting agents, skin-softening agents, cooling agents, skin-cooling agents, warming agents, skin-warming agents, stabilizers, UV-absorbing agents, UV filters, detergents, fabric conditioning agents, suspending agents, skin-tanning agents, thickeners, vitamins, oils, waxes, fats, phospholipids, saturated fatty acids, mono- or polyunsaturated fatty acids, α-hydroxy acids, polyhydroxyfatty acids, liquefiers, dyestuffs, colour-protecting agents, pigments, anticorrosives, aromas, flavouring substances, odoriferous substances, polyols, surfactants, electrolytes, organic solvents or silicone derivatives and the like as additional auxiliaries and additives.

However, every named (cosmetic, household) composition (or formulation) may further comprise various further ingredients, additives and auxiliaries. Some of them maybe use in cosmetic formulations and compositions as well as in household products and compositions/formulations. For some of the ingredients, additives and auxiliaries, there are no precise divisions which of them are only used in one kind of composition or formulation. Thus, a series of ingredients, additives and auxiliaries can be used in cosmetic formulations and compositions as well as in the household products and compositions / formulations as needed. The below listings of the ingredients, additives and auxiliaries are therefore not only limited to the named compositions/formulations underneath they are specified to.

Preferred compositions and formulations according to the present inventions are selected from the group of products for treatment, protecting, care and cleansing of the skin, mouth and/or hair or as a make-up product, preferably as a leave-on product (meaning that active ingredients stay on the skin and/or hair for a longer period of time, compared to rinse-off products, so that the moisturizing and/or anti-ageing and/or wound healing promoting action thereof is more pronounced) and preferably rinse-off products, such as shampoos, shower gels, hair tonics, face cleansers, mouthwashes.

The compositions and formulations according to the invention are preferably water-based formulations. Such formulations or preparations may comprise water in a quantity of up to 99 % b.w., preferably 50 to 99 % b.w., based on the total weight of the preparation.

Particular preferred compositions and formulations are mouthwashes, after shaves, face cleaners, shampoos, shower gels, soap bars, alcoholic and non-alcoholic deos (sprays or roll-on), household cleaners, liquid detergents.

In a preferred embodiment a mouth rinse preparation or formulation may preferably comprises or consisting of
i) from 0 to 26%b.w. ethylalcohol,
ii) from 0.2 to 3% b.w. Cremophor CO 40 (PEG 40 hydrogenated castor oil),
iii) from 0.05 to 0.50% b.w. flavor,
iv) from 0 to 10% % b.w., preferably from 2 to 10% b.w. sorbitol 70%,
v) from 0 to 0.5% b.w., preferably from 0.05 to 0.5% b.w. sodiumsaccharin 450,
vi) from 0 to 0.5% b.w., preferably from 0.05 to 0.5% b.w. sodiumfluoride,
vii) from 0 to 1.0% b.w., preferably from 0.01 to 1.0% % b.w. benzoic acid,
viii) from 0.1 to 1.0% b.w. mixture according to the invention,
ix) water (deionized),
on condition that the amounts add - optionally together with additional ingredients - to 100% b.w.

In a preferred embodiment a shampoo preparation or formulation may preferably comprises or consisting of
i) from 5 to 25% b.w. sodium lauryl ether sulfate (e.g. Texapon NSO),
ii) from 1.0 to 5% b.w. cocamidopropyl betaine (e.g. Dehyton K),
iii) from 0 to 5% b.w. plant oil, preferably from 0.1 to 5% b.w. (e.g. avocado oil),
iv) from 0 to 10% b.w., preferably from 0.1 to 10% b.w. fatty acid ester (e.g. Dragoxat 89: ethy hexyl isononanoate),
v) from 0 to 3% b.w., preferably from 0.1 to 2% b.w. sodium chloride,
vi) from 0 to 2% b.w. citric acid,
vii) from 0 to 2% b.w., preferably from 0.01 to 2% b.w. perfume oil,
viii) from 0 to 1.5% b.w., preferably from 0.5 to 1.5% b.w. phenoxyethanol, 4-hydroxy acetophenone,
ix) from 0.1 to 2% b.w. mixture according to the invention,
x) water (deionized),
on condition that the amounts add - optionally together with additional ingredients - to 100% b.w.

In a preferred embodiment a soap bar preparation and formulation may preferably comprises or consisting of
i) from 50 to 80% b.w. sodium soap from tallow,
ii) from 0 to 40% b.w. sodium soap from palm oil,
iii) from 0 to 10% b.w. glycerol,
iv) from 0 to 2% b.w. sodium chloride,
v) from 0 to 0,5% b.w. 1-hydroxyethane-1,1-diphosphonic acid, tetrasodium salt,
vi) from 0 to 0.5% b.w. ethylendiamin tetra acetic acid tetrasodium salt,
vii) from 0 to 3% b.w. fragrance,
viii) from 0.2 to 2% b.w. mixture according to the invention,
ix) water(deionized),
on condition that the amounts add - optionally together with additional ingredients - to 100% b.w.

In a preferred embodiment a deodorant preparation or formulation may preferably comprises or consisting of
i) from 0 to 90% b.w. ethylalcohol,
ii) from 0.2 to 3% b.w. Cremophor CO 40 (PEG 40 hydrogenated castor oil),
iii) from 0 to 2% b.w., preferably from 0.1 to 2% b.w. fragrance,
iv) from 0 to 20 % b.w., preferably from 0.1 to 18% b.w. glycerine,
v) from 0 to 30 % b.w., preferably from 1 to 30% b.w. aluminum chlorohydrate,
vi) from 0 to 80% b.w., preferably from 0.1 to 70% b.w. cyclomethicone,
vii) from 0 to 1% b.w., preferably from 0.01 to 1% b.w. phenoxyethanol,
viii) from 0 to 5% b.w., preferably from 0.1 to 5% b.w.fatty acid ester or caprylic, capric triglycerides
ix) from 0 to 1% b.w., preferably from 0.1 to 0.8% b.w. ethylhexyl glycerine,
x) from 0.1 to 1% b.w. mixture according to the invention,
xi) water (deionized)
on condition that the amounts add - optionally together with additional ingredients - to 100% b.w.

These water-based formulations advantageously serve to fulfil a series of all the above mentioned needs simultaneously to improve stability of cosmetic and household formulations.

The formulations according to the invention may also be in the form of an emulsion, e.g. W/O (water-in-oil), O/W (oil-in-water), W/O/W (water-in-oil-in-water), O/W/O (oil-in-water-in-oil) emulsion, PIT emulsion, Pickering emulsion, emulsion with a low oil content, micro- or nanoemulsion, a solution, e.g. in oil (fatty oils or fatty acid esters, in particular C6-C32 fatty acid C2-C30 esters) or silicone oil, dispersion, suspension, creme, lotion or milk, depending on the production method and ingredients, a gel (including hydrogel, hydrodispersion gel, oleogel), spray (e.g. pump spray or spray with propellant) or a foam or an impregnating solution for cosmetic wipes, a detergent, e.g. soap, synthetic detergent, liquid washing, shower and bath preparation, bath product (capsule, oil, tablet, salt, bath salt, soap, etc.), effervescent preparation, a skin care product such as e.g. an emulsion (as described above), ointment, paste, gel (as described above), oil, balsam, serum, powder (e.g. face powder, body powder), a mask, a pencil, stick, roll-on, pump, aerosol (foaming, non-foaming or post-foaming), a deodorant and/or antiperspirant, mouthwash and mouth rinse, a foot care product (including keratolytic, deodorant), an insect repellent, a sunscreen, after-sun preparation, a shaving product, aftershave balm, pre- and aftershave lotion, a depilatory agent, a hair care product such as e.g. shampoo (including 2-in-1 shampoo, anti-dandruff shampoo, baby shampoo, shampoo for dry scalps, concentrated shampoo), conditioner, hair tonic, hair water, hair rinse, styling creme, pomade, perm and setting lotion, hair spray, styling aid (e.g. gel or wax), hair smoothing agent (detangling agent, relaxer), hair dye such as e.g. temporary direct-dyeing hair dye, semi-permanent hair dye, permanent hair dye, hair conditioner, hair mousse, eye care product, make-up, make-up remover or baby product.

Further ingredients, additives and auxiliaries can be included in quantities of 5 to 99 % b.w., preferably 10 to 80 % b.w., based on the total weight of the formulation. The amounts of cosmetic auxiliary agents and additives and perfume to be used in each case depends on their function and intention to used, and can easily be determined by the person skilled in the art by simple trial and error, depending on the nature of the particular product.

### COSMETIC AND HOUSEHOLD PREPARATIONS

Cosmetic and household preparations according to the present invention may include similar additives, such as for example surfactants. Therefore, the border between cosmetic and household preparations is in flow and it should be understood that components cited for one application could in some cases also be recommended for the other mutatis-mutandis without literal repetition.

The cosmetic and household preparations accordingly may comprise typical auxiliaries and further additives as described aforementioned. Typical auxiliaries and further additives are such as mild surfactants, oil components, emulsifiers, pearlizing waxes, consistency-imparting agents, thickeners, superfatting agents, stabilizers, polymers, silicone compounds, fats, waxes, lecithins, phospholipids, moisturizers, biogenic agents, antioxidants, film-forming agents, expanding agents, insect repellents, self-tanning agents, tyrosine inhibitors (depigmenting agents), hydrotropes, solubilizers, preservatives, perfume oils, dyes and the like.

### SURFACTANTS

Examples of suitable surface-active substances that may be included are anionic, nonionic, cationic and/or amphoteric or zwitterionic surfactants, ordinarily contained in the agents in amounts of approx. 1 to 70, preferably 5 to 50, and particularly 10 to 30 wt%. Typical examples of anionic surfactants include soaps, alkylbenzene sulfonates, alkane sulfonates, olefin sulfonates, alkyl ether sulfonates, glycerol ether sulfonates, α-methyl ester sulfonates, sulfofatty acids, alkyl sulfates, alkylether sulfates, glycerol ether sulfates, fatty acid ether sulfates, hydroxy mixed ether sulfates, monoglyceride (ether) sulfates, fatty acid amide (ether) sulfates, mono- and dialkylsulfosuccinates, mono- and dialkylsulfosuccinamates, sulfotriglycerides, amide soaps, ether carboxylic acids and salts thereof, fatty acid isethionates, fatty acid sarcosinates, fatty acid taurides, N-acylamino acids such as acyl lactylates, acyl tartrates, acyl glutamates, and acyl aspartates, alkyl oligoglycoside sulfates, protein fatty acid condensates (particularly wheat-based vegetable products) and alkyl(ether)phosphates. If the anionic surfactants contain polyglycol ether chains, they may show a conventional homolog distribution, but preferably a narrow-range homolog distribution. Typical examples of nonionic surfactants are fatty alcohol polyglycol ethers, alkyl phenol polyglycol ethers, fatty acid polyglycol esters, fatty acid amide polyglycol ethers, fatty amine polyglycol ethers, alkoxylated triglycerides, mixed ethers or mixed formals, optionally partially oxidized alk(en)yl oligoglycosides or glucuronic acid derivatives, fatty acid N-alkyl glucamides, protein hydrolysates (particularly wheat-based vegetable products), polyol fatty acid esters, sugar esters, sorbitan esters, polysorbates, and amine oxides. If the nonionic surfactants contain polyglycol ether chains, they may show a conventional homolog distribution, but preferably a narrow-range homolog distribution. Typical examples of cationic surfactants are quaternary ammonium compounds such as dimethyl distearyl ammonium chloride, and esterquats, particularly quaternized fatty acid trialkanolamine ester salts. Typical examples of amphoteric or zwitterionic surfactants are alkylbetaines, alkylamidobetaines, aminopropionates, aminoglycinates, imidazolinium betaines, and sulfobetaines. The above-mentioned surfactants are exclusively known compounds. Typical examples of particularly suitable mild surfactants, i.e. those particularly well-tolerated by the skin, are fatty alcohol polylycolether sulfates, monoglyceride sulfates, mono- and/or dialkylsulfosuccinates, fatty acid isethionates, fatty acid sarcosinates, fatty acid taurides, fatty acid glutamates, α-olefin sulfonates, ether carboxylic acids, alkyl oligoglycosides, fatty acid glucamides, alkyl amidobetaines, and amphoacetal and/or protein fatty acid condensates, with the latter preferably being based on wheat proteins.

### Anionic (co-) surfactants

Preferably, surfactants of the sulfonate type, alk(en)yl sulfonates, alkoxylated alk(en)yl sulfates, ester sulfonates and/or soaps are used as the anionic surfactants. Suitable surfactants of the sulfonate type are advantageously C₉₋₁₃ alkylbenzene sulfonates, olefin sulfonates, i.e. mixtures of alkene- and hydroxyalkane sulfonates, and disulfonates, as are obtained, for example, by the sulfonation with gaseous sulfur trioxide of C₁₂₋₁₈ monoolefins having a terminal or internal double bond and subsequent alkaline or acidic hydrolysis of the sulfonation products.

**Alk(en)yl sulfates.** Preferred alk(en)yl sulfates are the alkali and especially the sodium salts of the sulfuric acid half-esters of the C₁₂-C₁₈ fatty alcohols, for example, from coconut butter alcohol, tallow alcohol, lauryl, myristyl, cetyl or stearyl alcohol or from C₈-C₂₀ oxo alcohols and those half-esters of secondary alcohols of these chain lengths. Alk(en)yl sulfates of the cited chain lengths that comprise a synthetic straight chain alkyl group manufactured petrochemically are also preferred. The C₁₂-C₁₆ alkyl sulfates and C₁₂-C₁₅ alkyl sulfates as well as C₁₄-C₁₅ alkyl sulfates and C₁₄-C₁₆ alkyl sulfates are particularly preferred on the grounds of laundry performance. The 2,3-alkyl sulfates, which can be obtained from Shell Oil Company under the trade name DAN™, are also suitable anionic surfactants.

**Alk(en)yl ether sulfates.** Sulfuric acid mono-esters derived from straight-chained or branched C₇-C₂₁ alcohols ethoxylated with 1 to 6 moles ethylene oxide are also suitable, such as 2-methyl-branched C₉-C₁₁ alcohols with an average of 3.5 mol ethylene oxide (EO) or C₁₂-C₁₈ fatty alcohols with 1 to 4 EO.

**Ester sulfonates.** The esters of alpha-sulfo fatty acids (ester sulfonates), e.g., the alpha-sulfonated methyl esters of hydrogenated coco-, palm nut- or tallow acids are likewise suitable.

**Soaps.** Soaps, in particular, can be considered as further anionic surfactants. Saturated fatty acid soaps are particularly suitable, such as the salts of lauric acid, myristic acid, palmitic acid, stearic acid, hydrogenated erucic acid and behenic acid, and especially soap mixtures derived from natural fatty acids such as coconut oil fatty acid, palm kernel oil fatty acid or tallow fatty acid. Those soap mixtures are particularly preferred that are composed of 50 to 100 wt. % of saturated C₁₂-C₂₄ fatty acid soaps and 0 to 50 wt. % of oleic acid soap.

**Ether carboxylic acids.** A further class of anionic surfactants is that of the ether carboxylic acids, obtainable by treating fatty alcohol ethoxylates with sodium chloroacetate in the presence of basic catalysts. They have the general formula: RO(CH₂CH₂O)ₚCH₂COOH with R = C₁-C₁₈ and p = 0.1 to 20. Ether carboxylic acids are insensitive to water hardness and possess excellent surfactant properties.

### Nonionic (co-)surfactants

**Alkohol alkoxylates.** The added nonionic surfactants are preferably alkoxylated and/or propoxylated, particularly primary alcohols having preferably 8 to 18 carbon atoms and an average of 1 to 12 mol ethylene oxide (EO) and/or 1 to 10 mol propylene oxide (PO) per mol alcohol. C₈-C₁₆-Alcohol alkoxylates, advantageously ethoxylated and/or propoxylated C₁₀-C₁₅-alcohol alkoxylates, particularly C₁₂-C₁₄ alcohol alkoxylates, with an ethoxylation degree between 2 and 10, preferably between 3 and 8, and/or a propoxylation degree between 1 and 6, preferably between 1.5 and 5, are particularly preferred. The cited degrees of ethoxylation and propoxylation constitute statistical average values that can be a whole or a fractional number for a specific product. Preferred alcohol ethoxylates and propoxylates have a narrowed homolog distribution (narrow range ethoxylates/propoxylates, NRE/NRP). In addition to these nonionic surfactants, fatty alcohols with more than 12 EO can also be used. Examples of these are (tallow) fatty alcohols with 14 EO, 16 EO, 20 EO, 25 EO, 30 EO or 40 EO.

**Alkylglycosides (APG®).** Furthermore, as additional nonionic surfactants, alkyl glycosides that satisfy the general Formula RO(G)ₓ, can be added, e.g., as compounds, particularly with anionic surfactants, in which R means a primary linear or methyl-branched, particularly 2-methyl-branched, aliphatic group containing 8 to 22, preferably 12 to 18 carbon atoms and G stands for a glycose unit containing 5 or 6 carbon atoms, preferably for glucose. The degree of oligomerization x, which defines the distribution of monoglycosides and oligoglycosides, is any number between 1 and 10, preferably between 1.1 and 1.4.

**Fatty acid ester alkoxylates.** Another class of preferred nonionic surfactants, which are used either as the sole nonionic surfactant or in combination with other nonionic surfactants, in particular, together with alkoxylated fatty alcohols and/or alkyl glycosides, are alkoxylated, preferably ethoxylated or ethoxylated and propoxylated fatty acid alkyl esters preferably containing 1 to 4 carbon atoms in the alkyl chain, more particularly the fatty acid methyl esters which are described, for example, in Japanese Patent Application JP-A-58/217598 or which are preferably produced by the process described in International Patent Application WO-A-90/13533. Methyl esters of C₁₂-C₁₈ fatty acids containing an average of 3 to 15 EO, particularly containing an average of 5 to 12 EO, are particularly preferred.

**Amine oxides.** Nonionic surfactants of the amine oxide type, for example, N-coco alkyl-N,N-dimethylamine oxide and N-tallow alkyl-N,N-dihydroxyethylamine oxide, and the fatty acid alkanolamides may also be suitable. The quantity in which these nonionic surfactants are used is preferably no more than the quantity in which the ethoxylated fatty alcohols are used and, particularly no more than half that quantity.

**Gemini surfactants.** The so-called gemini surfactants can be considered as further surfactants. Generally speaking, such compounds are understood to mean compounds that have two hydrophilic groups and two hydrophobic groups per molecule. As a rule, these groups are separated from one another by a "spacer". The spacer is usually a hydrocarbon chain that is intended to be long enough such that the hydrophilic groups are a sufficient distance apart to be able to act independently of one another. These types of surfactants are generally characterized by an unusually low critical micelle concentration and the ability to strongly reduce the surface tension of water. In exceptional cases, however, not only dimeric but also trimeric surfactants are meant by the term gemini surfactants. Suitable gemini surfactants are, for example, sulfated hydroxy mixed ethers according to German Patent Application DE 4321022 A1 or dimer alcohol bis- and trimer alcohol tris sulfates and ether sulfates according to International Patent Application WO 96/23768 A1. Blocked end group dimeric and trimeric mixed ethers according to German Patent Application DE 19513391 A1 are especially characterized by their bifunctionality and multifunctionality. Gemini polyhydroxyfatty acid amides or polyhydroxyfatty acid amides, such as those described in International Patent Applications WO 95/19953 A1, WO 95/19954 A1 and WO 95/19955 A1 can also be used.

### Cationic co-surfactants

**Tetraalkyl ammonium salts.** Cationically active surfactants comprise the hydrophobic high molecular group required for the surface activity in the cation by dissociation in aqueous solution. A group of important representatives of the cationic surfactants are the tetraalkyl ammonium salts of the general formula: (R¹R²R³R⁴N⁺) X⁻. Here R1 stands for C₁-C₈ alk(en)yl, R², R³ and R⁴, independently of each other, for alk(en)yl radicals having 1 to 22 carbon atoms. X is a counter ion, preferably selected from the group of the halides, alkyl sulfates and alkyl carbonates. Cationic surfactants, in which the nitrogen group is substituted with two long acyl groups and two short alk(en)yl groups, are particularly preferred.

**Esterquats.** A further class of cationic surfactants particularly useful as co-surfactants for the present invention is represented by the so-called esterquats. Esterquats are generally understood to be quaternised fatty acid triethanolamine ester salts. These are known compounds which can be obtained by the relevant methods of preparative organic chemistry. Reference is made in this connection to International patent application WO 91/01295 A1, according to which triethanolamine is partly esterified with fatty acids in the presence of hypophosphorous acid, air is passed through the reaction mixture and the whole is then quaternised with dimethyl sulphate or ethylene oxide. In addition, German patent DE 4308794 C1 describes a process for the production of solid esterquats in which the quaternisation of triethanolamine esters is carried out in the presence of suitable dispersants, preferably fatty alcohols.

Typical examples of esterquats suitable for use in accordance with the invention are products of which the acyl component derives from monocarboxylic acids corresponding to formula RCOOH in which RCO is an acyl group containing 6 to 10 carbon atoms, and the amine component is triethanolamine (TEA). Examples of such monocarboxylic acids are caproic acid, caprylic acid, capric acid and technical mixtures thereof such as, for example, so-called head-fractionated fatty acid. Esterquats of which the acyl component derives from monocarboxylic acids containing 8 to 10 carbon atoms, are preferably used. Other esterquats are those of which the acyl component derives from dicarboxylic acids like malonic acid, succinic acid, maleic acid, fumaric acid, glutaric acid, sorbic acid, pimelic acid, azelaic acid, sebacic acid and/or dodecanedioic acid, but preferably adipic acid. Overall, esterquats of which the acyl component derives from mixtures of monocarboxylic acids containing 6 to 22 carbon atoms, and adipic acid are preferably used. The molar ratio of mono and dicarboxylic acids in the final esterquat may be in the range from 1:99 to 99:1 and is preferably in the range from 50:50 to 90:10 and more particularly in the range from 70:30 to 80:20. Besides the quaternised fatty acid triethanolamine ester salts, other suitable esterquats are quaternized ester salts of mono-/dicarboxylic acid mixtures with diethanolalkyamines or 1,2-dihydroxypropyl dialkylamines. The esterquats may be obtained both from fatty acids and from the corresponding triglycerides in admixture with the corresponding dicarboxylic acids. One such process, which is intended to be representative of the relevant prior art, is proposed in European patent EP 0750606 B1. To produce the quaternised esters, the mixtures of mono- and dicarboxylic acids and the triethanolamine - based on the available carboxyl functions - may be used in a molar ratio of 1.1:1 to 3:1. With the performance properties of the esterquats in mind, a ratio of 1.2:1 to 2.2:1 and preferably 1.5:1 to 1.9:1 has proved to be particularly advantageous. The preferred esterquats are technical mixtures of mono-, di- and triesters with an average degree of esterification of 1.5 to 1.9.

### Amphoteric or zwitterionic co-surfactants

**Betaines.** Amphoteric or ampholytic surfactants possess a plurality of functional groups that can ionize in aqueous solution and thereby--depending on the conditions of the medium--lend anionic or cationic character to the compounds (see DIN 53900, July 1972). Close to the isoelectric point (around pH 4), the amphoteric surfactants form inner salts, thus becoming poorly soluble or insoluble in water. Amphoteric surfactants are subdivided into ampholytes and betaines, the latter existing as zwitterions in solution. Ampholytes are amphoteric electrolytes, i.e. compounds that possess both acidic as well as basic hydrophilic groups and therefore behave as acids or as bases depending on the conditions. Especially betaines are known surfactants which are mainly produced by carboxyalkylation, preferably carboxymethylation, of amine compounds. The starting materials are preferably condensed with halocarboxylic acids or salts thereof, more particularly sodium chloroacetate, one mole of salt being formed per mole of betaine. The addition of unsaturated carboxylic acids, such as acrylic acid for example, is also possible. Examples of suitable betaines are the carboxy alkylation products of secondary and, in particular, tertiary amines which correspond to formula R¹R²R³N-(CH₂)_{q}COOX where R¹ is a an alkyl radical having 6 to 22 carbon atoms, R² is hydrogen or an alkyl group containing 1 to 4 carbon atoms, R³ is an alkyl group containing 1 to 4 carbon atoms, q is a number of 1 to 6 and X is an alkali and/or alkaline earth metal or ammonium. Typical examples are the carboxymethylation products of hexylmethylamine, hexyldimethylamine, octyldimethylamine, decyldimethylamine, C_{12/14}-cocoalkyldimethylamine, myristyldimethylamine, cetyldimethylamine, stearyldimethylamine, stearylethylmethylamine, oleyldimethylamine, C_{16/18}-tallowalkyldimethylamine and their technical mixtures, and particularly dodecyl methylamine, dodecyl dimethylamine, dodecyl ethylmethylamine and technical mixtures thereof.

**Alkylamido betaines.** Other suitable betaines are the carboxyalkylation products of amidoamines corresponding to formula R¹CO(R³)(R⁴)-NH-(CH₂)ₚ-N-(CH₂)_{q}COOX in which R¹CO is an aliphatic acyl radical having 6 to 22 carbon atoms and 0 or 1 to 3 double bonds, R² is hydrogen or an alkyl radical having 1 to 4 carbon atoms, R³ is an alkyl radical having 1 to 4 carbon atoms, p is a number from 1 to 6, q is a number from 1 to 3 and X is an alkali and/or alkaline earth metal or ammonium. Typical examples are reaction products of fatty acids having 6 to 22 carbon atoms, like for example caproic acid, caprylic acid, caprinic acid, lauric acid, myristic acid, palmitic acid, palmoleic acid, stearic acid, isostearic acid, oleic acid, elaidic acid, petroselinic acid, linolic acid linoleic acid, elaeostearic acid, arachidonic acid, gadoleic acid, behenic acid, erucic acid and their technical mixtures with N,N-dimethylaminoethylamine, N,N-dimethylaminopropylamine, N,N-diethylaminoethylamine und N,N-diethylaminopropylamine, which are condensed with sodium chloroacetate. The commercially available products include Dehyton® K and Dehyton® PK (Cognis Deutschland GmbH & Co., KG) as well as Tego®Betaine (Goldschmidt).

**Imidazolines.** Other suitable starting materials for the betaines to be used for the purposes of the invention are imidazolines. These substances are also known and may be obtained, for example, by cyclizing condensation of 1 or 2 moles of C₆⁻C₂₂ fatty acids with polyfunctional amines, such as for example aminoethyl ethanolamine (AEEA) or diethylenetriamine. The corresponding carboxyalkylation products are mixtures of different open-chain betaines. Typical examples are condensation products of the above- mentioned fatty acids with AEEA, preferably imidazolines based on lauric acid, which are subsequently betainised with sodium chloroacetate. The commercially available products include Dehyton® G (Cognis Deutschland GmbH & Co., KG)

The amount of (co-)surfactant comprised in the inventive compositions is advantageously 0.1 wt. % to 90 wt. %, particularly 10 wt. % to 80 wt. % and particularly preferably 20 wt. % to 70 wt.-%.

### OIL COMPONENTS

Suitable oil components are, for example, Guerbet alcohols based on fatty alcohols containing 6 to 18, and preferably 8 to 10 carbon atoms, esters of linear C₆-C₂₂ fatty acids with linear or branched C₆-C₂₂ fatty alcohols or esters of branched C₆-C₁₃ carboxylic acids with linear or branched C₆-C₂₂ fatty alcohols, such as myristyl myristate, myristyl palmitate, myristyl stearate, myristyl isostearate, myristyl oleate, myristyl behenate, myristyl erucate, cetyl myristate, cetyl palmitate, cetyl stearate, cetyl isostearate, cetyl oleate, cetyl behenate, cetyl erucate, stearyl myristate, stearyl palmitate, stearyl stearate, stearyl isostearate, stearyl oleate, stearyl behenate, stearyl erucate, isostearyl myristate, isostearyl palmitate, isostearyl stearate, isostearyl isostearate, isostearyl oleate, isostearyl behenate, isostearyl oleate, oleyl myristate, oleyl palmitate, oleyl stearate, oleyl isostearate, oleyl oleate, oleyl behenate, oleyl erucate, behenyl myristate, behenyl palmitate, behenyl stearate, behenyl isostearate, behenyl oleate, behenyl behenate, behenyl erucate, erucyl myristate, erucyl palmitate, erucyl stearate, erucyl isostearate, erucyl oleate, erucyl behenate and erucyl erucate. Also suitable are esters of linear C₆-C₂₂ fatty acids with branched alcohols, particularly 2-ethyl hexanol, esters of C₁₈-C₃₈-alkyl hydroxycarboxylic acids with linear or branched C₆-C₂₂ fatty alcohols, particularly dioctyl malate, esters of linear and/or branched fatty acids with polyhydric alcohols (such as propylene glycol, dimer diol, or trimer triol) and/or Guerbet alcohols, triglycerides based on C₆-C₁₀ fatty acids, liquid mono-/di-/triglyceride mixtures based on C₆-C₁₈ fatty acids, esters of C₆-C₂₂ fatty alcohols and/or Guerbet alcohols with aromatic carboxylic acids, particularly benzoic acid, esters of C₂-C₁₂ dicarboxylic acids with linear or branched alcohols with 1 to 22 carbon atoms or polyols with 2 to 10 carbon atoms and 2 to 6 hydroxyl groups, vegetable oils, branched primary alcohols, substituted cyclohexanes, linear and branched C₆-C₂₂ fatty alcohol carbonates such as dicaprylyl carbonate (Cetiol® CC), Guerbet carbonates based on fatty alcohols containing 6 to 18, and preferably 8 to 10 carbon atoms, esters of benzoic acid with linear and/or branched C₆-C₂₂-alcohols (such as Finsolv® TN), linear or branched, symmetrical or asymmetrical dialkyl ethers containing 6 to 22 carbon atoms per alkyl group, such as dicaprylyl ether (Cetiol® OE), ring-opening products of epoxidized fatty acid esters with polyols, silicone oils (cyclomethicone, silicon methicones, etc.) and/or aliphatic or naphthenic hydrocarbons such as squalane, squalene, or dialkyl cyclohexane.

### EMULSIFIERS

Examples of suitable emulsifiers include nonionic surfactants from at least one of the following groups:
- addition products of 2 to 30 mol of ethylene oxide and/or 0 to 5 mol of propylene oxide to linear fatty alcohols with 8 to 22 carbon atoms, to fatty acids with 12 to 22 carbon atoms, to alkyl phenols with 8 to 15 carbon atoms in the alkyl group, as well as alkylamines with 8 to 22 carbon atoms in the alkyl residue;
- alkyl and/or alkenyl oligoglycosides with 8 to 22 carbon atoms the alk(en)yl residue and ethoxylated analogs thereof;
- addition products of 1 to 15 mol of ethylene oxide to castor oil and/or hardened castor oil;
- addition products of 15 to 60 mol of ethylene oxide to castor oil and/or hardened castor oil;
- partial esters of glycerol and/or sorbitan with unsaturated, linear or saturated, branched fatty acids with 12 to 22 carbon atoms and/or hydroxycarboxylic acids with 3 to 18 carbon atoms, as well as adducts thereof with 1 to 30 mol of ethylene oxide;
- partial esters of polyglycerol (average degree of self-condensation 2 to 8), polyethylene glycol (molecular weight 400 to 5000), trimethylolpropane, pentaerythrite, sugar alcohols (such as sorbite), alkyl glycosides (such as methyl glycoside, butyl glycoside, lauryl glycoside), as well as polyglycosides (such as cellulose) with saturated and/or unsaturated, linear or branched fatty acids with 12 to 22 carbon atoms and/or hydroxycarboxylic acids with 3 to 18 carbon atoms, as well as adducts thereof with 1 to 30 mol of ethylene oxide;
- mixed esters of pentaerythrite, fatty acids, citric acid and fatty alcohols and/or mixed esters of fatty acids containing 6 to 22 carbon atoms, methyl glucose and polyols, preferably glycerol or polyglycerol.
- mono, di- and trialkylphosphates, as well as mono, di- and/or tri-PEG-alkyl phosphates and salts thereof;
- wool wax alcohols;
- polysiloxane/polyalkyl/polyether copolymers or corresponding derivatives;
- block copolymers such as polyethylene glycol-30 dipolyhydroxystearate;
- polymer emulsifiers, such as Pemulen polymers (TR-1, TR-2) from Goodrich or Cosmedia® SP from Cognis;
- polyalkylene glycols, and
- glycerol carbonates.

In the following, particularly suitable emulsifiers are described in further detail:
**Alkoxylates.** The addition products of ethylene oxide and/or propylene oxide to fatty alcohols, fatty acids, alkyl phenols, or castor oil constitute known, commercially available products. These are homolog mixtures whose average degree of alkoxylation corresponds to the ratio of the amounts of ethylene oxide and/or propylene oxide to the substrates with the addition reaction was carried out. C_{12/18}-fatty acid mono and diesters of addition products of ethylene oxide to glycerol are known as refatting agents for cosmetic preparations.
**Alkyl and/or alkenyl oligoglycosides.** Alkyl and/or alkenyl oligoglycosides and the production and use thereof are known from prior art. In particular they are produced by reacting glucose or oligosaccharides with primary alcohols having 8 to 18 carbon atoms. With the respect to the glycoside residue, both monoglycosides, in which a cyclic sugar residue is glycosidically bonded to the fatty alcohol, and oligomeric glycosides, preferably having a degree of oligomerization of approx. 8, are suitable. In this case, the degree of oligomerization is an average statistical value based on the usual homolog distribution for such technical products.
**Partial glycerides.** Typical examples of suitable partial glycerides are hydroxystearic acid monoglyceride, hydroxystearic acid diglyceride, isostearic acid monoglyceride, isostearic acid diglyceride, oleic acid monoglyceride, oleic acid diglyceride, ricinoleic acid monoglyceride, ricinoleic acid diglyceride, linoleic acid monoglyceride, linoleic acid diglyceride, linolenic acid monoglyceride, linolenic acid diglyceride, erucic acid monoglyceride, erucic acid diglyceride, tartaric acid monoglyceride, tartaric acid diglyceride, citric acid monoglyceride, citric diglyceride, malic acid monoglyceride, malic acid diglyceride, and technical mixtures thereof that can secondarily contain small amounts of triglycerides from the production process. Addition products of 1 to 30, and preferably 5 to 10 mol of ethylene oxide to the above-mentioned partial glycerides are also suitable.
**Sorbitan esters.** Examples of suitable sorbitan esters include sorbitan monoisostearate, sorbitan sesquiisostearate, sorbitan diisostearate, sorbitan triisostearate, sorbitan monooleate, sorbitan sesquioleate, sorbitan dioleate, sorbitan trioleate, sorbitan monoerucate, sorbitan sesquierucate, sorbitan dierucate, sorbitan trierucate, sorbitan monoricinoleate, sorbitan sesquiricinoleate, sorbitan diricinoleate, sorbitan triricinoleate, sorbitan monohydroxystearate, sorbitan sesquihydroxystearate, sorbitan dihydroxystearate, sorbitan trihydroxystearate, sorbitan monotartrate, sorbitan sesquitartrate, sorbitan ditartrate, sorbitan tritartrate, sorbitan monocitrate, sorbitan sesquicitrate, sorbitan dicitrate, sorbitan tricitrate, sorbitan monomaleate, sorbitan sesquimaleate, sorbitan dimaleate, sorbitan trimaleate, and technical mixtures thereof. Addition products of 1 to 30 and preferably 5 to 10 mol of ethylene oxide to the above-mentioned sorbitan esters are also suitable.
**Polyglycerol esters.** Typical examples of suitable polyglycerol esters are polyglyceryl-2 dipolyhydroxystearate (Dehymuls® PGPH), polyglyceryl-3-diisostearate (Lameform® TGI), polyglyceryl-4 isostearate (Isolan® GI 34), polyglyceryl-3 oleate, diisostearoyl polyglyceryl-3 diisostearate (Isolan® PDI), polyglyceryl-3 methylglucose distearate (Tego Care® 450), polyglyceryl-3 beeswax (Cera Bellina®), polyglyceryl-4 caprate (polyglycerol caprate T2010/90), polyglyceryl-3 cetyl ether (Chimexane® NL), polyglyceryl-3 distearate (Cremophor® GS 32), polyglyceryl polyricinoleate (Admul® WOL 1403), polyglyceryl dimerate isostearate, and mixtures thereof. Examples of further suitable polyol esters are mono, di, and triesters, optionally reacted with 1 to 30 mol of ethylene oxide, of trimethylol propane or pentaerythrite with lauric acid, coconut fatty acid, tallow fatty acid, palmitic acid, stearic acid, oleic acid, behenic acid and the like.
**Anionic emulsifiers.** Typical anionic emulsifiers are aliphatic fatty acids with 12 to 22 carbon atoms, such as palmitic acid, stearic acid or behenic acid, as well as dicarboxylic acids with 12 to 22 carbon atoms, such as azelaic acid or sebacic acid.
**Amphoteric and cationic emulsifiers.** Zwitterionic surfactants can also be used as emulsifiers. Zwitterionic surfactants are surface-active compounds that carry at least one quaternary ammonium group and at least one carboxylate and a sulfonate group in the molecule. Particularly suitable zwitterionic surfactants are the so-called betaines, including N-alkyl-N,N-dimethylammonium glycinates such as coconut alkyl dimethylammonium glycinate, N-acylaminopropyl-N,N-dimethylammonium glycinates such as coconut acyl-aminopropyldimethyl ammoniumglycinate, and 2-alkyl-3-carboxylmethyl-3-hydroxyethylimidazolines containing 8 to 18 carbon atoms in their alkyl or acyl groups, as well as coconut acylaminoethyl hydroxyethyl carboxymethyl glycinate. Particularly preferred is the fatty acid amide derivative known under the CTFA name cocamidopropyl betaine. Ampholytic surfactants are also suitable emulsifiers. Ampholytic surfactants are surface-active compounds that, in addition to a C_{8/18} alkyl or acyl group, contain at least one free amino group and at least one -COOH- or -SO₃H group in the molecule and are capable of forming inner salts. Examples of suitable ampholytic surfactants include N-alkyl glycines, N-alkyl propionic acids, N-alkyl aminobutyric acids, N-alkyl iminodipropionic acids, Nhydroxyethyl-N-alkyl amidopropylglycine, N-alkyl taurine, N-alkyl sarcosine, 2-alkyl aminopropionic acids and alkyl aminoacetic acids with approx. 8 to 18 carbon atoms in their alkyl groups. Particularly preferred ampholytic surfactants are N-coconut alkyl aminopropionate, coconut acyl aminoethylaminopropionate, and C_{12/18} acyl sarcosine. Finally, cationic surfactants are also suitable as emulsifiers, with those of the esterquat type, preferably methyl quaternized difatty acid triethanolamine ester salts, being particularly preferred.

### FATS AND WAXES

Typical examples of fats are glycerides, i.e. solid or liquid vegetable or animal products consisting essentially of mixed glycerol esters of higher fatty acids; examples of suitable waxes include natural waxes, such as candelilla wax, carnauba wax, Japan wax, esparto grass wax, cork wax, guaruma wax, rice germ oil wax, sugar cane wax, ouricury wax, montan wax, beeswax, shellac wax, spermaceti, lanolin (wool wax), uropygial fat, ceresin, ozocerite (earth wax), petrolatum, paraffin waxes, and microwaxes; chemically modified waxes (hard waxes), such as montan ester waxes, sasol waxes, hydrogenated jojoba waxes, as well as synthetic waxes such as polyalkylene waxes and polyethylene glycol waxes. In addition to the fats, fatlike substances such as lecithins and phospholipids are also suitable as additives. The person skilled in the art understands the term lecithins to refer to glycero-phospholipids formed from fatty acids, glycerol, phosphoric acid, and choline by esterification. Lecithins are therefore frequently referred to by specialists as phosphatidyl cholines (PC). Examples of suitable natural lecithins include the kephalins, also referred to as phosphatidic acids, and which are derivatives of 1,2-diacyl-sn-glycerol-3-phosphoric acids. In contrast, phospholipids are ordinarily understood to be mono- and preferably diesters of phosphoric acid with glycerol (glycerol phosphates) that are generally classified as fats. In addition, sphingosines or sphingolipids are also suitable.

Examples of suitable pearlizing waxes include alkylene glycol esters, particularly ethylene glycol distearate; fatty acid alkanolamides, particularly coconut fatty acid diethanolamide; partial glycerides, particularly stearic acid monoglyceride; esters of polyvalent, optionally hydroxy-substituted carboxylic acids with fatty alcohols containing 6 to 22 carbon atoms, particularly long-chain esters of tartaric acid; fatty substances such as fatty alcohols, fatty ketones, fatty aldehydes, fatty ethers, and fatty carbonates that have a total of at least 24 carbon atoms, particularly laurone and distearyl ether; fatty acids such as stearic acid, hydroxystearic acid, or behenic acid, ring opening products of olefin epoxides having 12 to 22 carbon atoms with fatty alcohols having 12 to 22 carbon atoms and/or polyols having 2 to 15 carbon atoms and 2 to 10 hydroxyl groups, as well as mixtures thereof.

### COOLANTS

Coolants are compounds that produce a feeling of coolness on the skin. As a rule, these are menthol compounds, which-in addition to the base component menthol itself-contain substances selected from the group comprising menthol methyl ether, menthone glyceryl acetal (FEMA GRAS 3807), menthone glyceryl ketal (FEMA GRAS 3808), menthyl lactate (FEMA GRAS 3748), menthol ethylene glycol carbonate (FEMA GRAS 3805), menthol propylene glycol carbonate (FEMA GRAS 3806), menthyl-N-ethyloxamate, monomethyl succinate (FEMA GRAS 3810), monomenthyl glutamate (FEMA GRAS 4006), menthoxy-1,2-propane diol (FEMA GRAS 3784), menthoxy-2-methyl-1,2-propane diol (FEMA GRAS 3849), and the methane carboxylic acid esters and amides WS-3, WS-4, WS-5, WS-12, WS-14, and WS-30, as well as mixtures thereof.

A first important representative of these substances is monomenthyl succinate (FEMA GRAS 3810). Both the succinate and the analogous monomenthyl glutarate (FEMA GRAS 4006) constitute important representatives of monomenthyl esters based on di- and polycarboxylic acids:

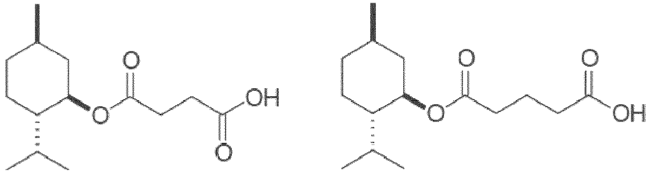

Examples of uses of these substances can be found for example in the documents WO 2003 043431 (Unilever) or EP 1332772 A1 (IFF).

The next important group of preferred menthol compounds within the meaning of the invention comprises carbonate esters of menthol and polyols, including glycols, glycerol, or carbohydrates, such as menthol ethylene glycol carbonate (FEMA GRAS 3805 = Frescolat® MGC), menthol propylene glycol carbonate (FEMA GRAS 3784 = Frescolat® MPC), menthol 2-methyl-1,2-propane diol carbonate (FEMA GRAS 3849) or the corresponding sugar derivatives. Also preferred are the menthol compounds menthyl lactate (FEMA GRAS 3748 = Frescolat® ML), and particularly menthone glyceryl acetal (FEMA GRAS 3807) or menthone glyceryl ketal (FEMA GRAS 3808), which is marketed under the name Frescolat® MGA, menthyl ethylamide oxalate, which is marketed under the name Frescolat® X-Cool. Among these substances, menthone glyceryl acetal/ketal, menthyl lactate, menthol ethylene glycol carbonate, menthyl ethylamide oxalate or menthol propylene glycol carbonate have been found to be particularly advantageous, and are marketed by the Applicant under the names Frescolat® MGA, Frescolat® ML, Frescolat® MGC, Frescolat® X-cool and Frescolat® MPC.

Menthol compounds having a C-C bond at position 3 and from which a series of representatives can also be used was first developed in the 1970s. These substances are generally referred to as WS types. The base component is a menthol derivative in which the hydroxyl group has been replaced with a carboxyl group (WS-1). All other types of WS, such as the preferred species WS-3, WS-4, WS-5, WS-12, WS-14 and WS-30, are derived from this structure.

### CONSISTENCY-IMPARTING AGENTS AND THICKENERS

Suitable consistency-imparting agents are primarily fatty alcohols or hydroxy fatty alcohols with 12 to 22, and preferably 16 to 18 carbon atoms, as well as partial glycerides, fatty acids, or hydroxy fatty acids. A combination of these substances with alkyl oligoglycosides and/or fatty acid-N-methylglucamides of the same chain length and/or polyglyceryl poly-12-hydroxystearates is preferred. Examples of suitable thickeners are aerosil types (hydrophilic silicic acids), polysaccharides, particularly xanthan gum, guar-guar, agar-agar, alginates and tyloses, carboxymethylcellulose and hydroxyethyl- and hydroxypropylcellulose, as well as higher-molecular polyethylene glycol mono- and diesters of fatty acids, polyacrylates (such as Carbopole® and Pemulen products from Goodrich; Synthalene® from Sigma; Keltrol products from Kelco; Sepigel products from Seppic; Salcare products from Allied Colloids) polyacrylamides, polymers, polyvinyl alcohol, and polyvinyl pyrrolidone. Bentonites such as Bentone® Gel VS-5PC (Rheox) have also been found to be particularly effective, comprising a mixture of cyclopentasiloxane, disteardimonium hectorite, and propylene carbonate. Also suitable are surfactants such as ethoxylated fatty acid glycerides, esters of fatty acids with polyols such as pentaerythrite or trimethylol propane, fatty alcohol ethoxylates having narrow-range homolog distribution, or alkyl oligoglycosides, as well as electrolytes such as table salt and ammonium chloride.

### SUPERFATTING AGENTS

Examples of suitable superfatting agents are substances such as lanolin and lecithin, as well as polyethoxylated or acylated lanolin and lecithin derivatives, polyol fatty acid esters, monoglycerides, and fatty acid alkanolamides, wherein the latter simultaneously serve as foam stabilizers.

### POLYMERS

Examples of suitable cationic polymers include cationic cellulose derivatives such as a quaternized hydroxyethylcellulose available under the name Polymer JR 400® from Amerchol, cationic starch, copolymers of diallyl ammonium salts and acrylamides, quaternized vinyl pyrrolidone/vinyl imidazole polymers such as Luviquat® (BASF), condensation products of polyglycols and amines, quaternized collagen polypeptides such as lauryldimonium hydroxypropyl hydrolyzed collagen (Lamequat®L/Grünau), quaternized wheat polypeptides, polyethylene imine, cationic silicone polymers such as amodimethicone, copolymers of adipic acid and dimethylaminohydroxypropyldiethylene triamine (Cartaretine®/Sandoz), copolymers of acryl acid with dimethyl diallyl ammonium chloride (Merquat® 550/Chemviron), polyaminopolyamides and crosslinked water-soluble polymers thereof, cationic chitin derivatives such as quaternized chitosan, optionally distributed in microcrystalline form, condensation products of dihalogen alkylene such as dibromobutane with bisdialkylamines such as bis-dimethylamino-1,3-propane, cationic guar-gums such as Jaguar® CBS, Jaguar® C-17, and Jaguar® C-16 from Celanese, and quaternized ammonium salt polymers such as Mirapol® A-15, Mirapol® AD-1, and Mirapol® AZ-1 from Miranol.

Examples of suitable anionic, zwitterionic, amphoteric, and nonionic polymers include vinyl acetate/crotonic acid copolymers, vinyl pyrrolidone/vinyl acrylate copolymers, vinyl acetate/butyl maleate/isobornyl acrylate copolymers, methylvinyl ether/maleic acid anhydride copolymers and esters thereof, non-crosslinked polyacrylic acids and polyacrylic acids crosslinked with polyols, acrylamidopropyl trimethylammonium chloride/acrylate copolymers, octylacrylamide/methyl methacrylate/tert-butyl aminoethyl methacrylate/2-hydroxypropyl methacrylate copolymers, polyvinyl pyrrolidone, vinyl pyrrolidone/vinyl acetate copolymers, vinyl pyrrolidone/dimethylaminoethyl methacrylate/vinyl caprolactam terpolymers, and optionally, derivatized cellulose ethers and silicones.

### THICKENING AGENTS AND RHEOLOGY ADDITIVES

Suitable thickeners are polymeric thickeners, such as Aerosil® types (hydrophilic silicas), polysaccharides, more especially xanthan gum, guar-guar, agar-agar, alginates and tyloses, carboxymethyl cellulose and hydroxyethyl cellulose, also relatively high molecular weight polyethylene glycol monoesters and diesters of fatty acids, polyacrylates (for example Carbopols® [Goodrich] or Synthalens® [Sigma]), polyacrylamides, polyvinyl alcohol and polyvinyl pyrrolidone, surfactants such as, for example, ethoxylated fatty acid glycerides, esters of fatty acids with polyols, for example pentaerythritol or trimethylol propane, narrow-range fatty alcohol ethoxylates and electrolytes, such as sodium chloride and ammonium chloride.

### SILICONE COMPOUNDS

Suitable silicone compounds are for example dimethyl polysiloxane, methylphenyl polysiloxane, cyclic silicones, as well as amino, fatty acid, alcohol, polyether, epoxy, fluorine, glycoside, and/or alkyl-modified silicone compounds, which can be present at room temperature either in liquid or resinous form. Also suitable are simethicones, which are mixtures of dimethicones having an average chain length of 200 to 300 dimethylsiloxane units and hydrogenated silicates.

### MOISTURIZERS

Moisturizers are used for further optimization of the sensory properties of the composition and for moisture regulation of the skin. At the same time, the cold stability of the preparations according to the invention is increased, particularly in the case of emulsions. The moisturizers are ordinarily contained in an amount of 0.1 to 15 wt%, preferably 1 to 10 wt%, and particularly preferably 5 to 10 wt%.

Examples of suitable moisturizers according to the invention include amino acids, pyrrolidone carboxylic acid, lactic acid and salts thereof, lactitol, urea and urea derivatives, uric acid, glucosamine, creatinine, cleavage products of collagen, chitosan or chitosan salt derivatives, and particularly polyols and polyol derivatives (such as glycerol, diglycerol, triglycerol, ethylene glycol, propylene glycol, butylene glycol, erythrite, 1,2,6-hexane triol, polyethylene glycols such as PEG-4, PEG-6, PEG-7, PEG-8, PEG-9, PEG-10, PEG-12, PEG-14, PEG-16, PEG-18, and PEG-20), sugar and sugar derivatives (including fructose, glucose, maltose, maltitol, mannite, inosite, sorbite, sorbityl silane diol, sucrose, trehalose, xylose, xylite, glucuronic acid and salts thereof), ethoxylated sorbite (sorbeth-6, sorbeth-20, sorbeth-30, sorbeth-40), honey and hardened honey, hardened starch hydrolysates, as well as mixtures of hardened wheat protein and PEG-20/acetate copolymer. Preferred suitable moisturizers according to the invention are glycerol, diglycerol, triglycerol, and butylene glycol.

### PRIMARY SUN PROTECTION FACTORS

Primary sun protection factors in the context of the invention are, for example, organic substances (light filters) which are liquid or crystalline at room temperature and which are capable of absorbing ultraviolet radiation and of releasing the energy absorbed in the form of longer-wave radiation, for example heat.

The formulations according to the invention advantageously contain at least one UV-A filter and/or at least one UV-B filter and/or a broadband filter and/or at least one inorganic pigment. Formulations according to the invention preferably contain at least one UV-B filter or a broadband filter, more particularly preferably at least one UV-A filter and at least one UV-B filter.

Preferred cosmetic compositions, preferably topical formulations according to the present invention comprise one, two, three or more sun protection factors selected from the group consisting of 4-aminobenzoic acid and derivatives, salicylic acid derivatives, benzophenone derivatives, dibenzoylmethane derivatives, diphenyl acrylates, 3-imidazol-4-yl acrylic acid and esters thereof, benzofuran derivatives, benzylidene malonate derivatives, polymeric UV absorbers containing one or more organosilicon radicals, cinnamic acid derivatives, camphor derivatives, trianilino-s-triazine derivatives, 2-hydroxyphenylbenzotriazole derivatives, phenylbenzimidazole sulfonic acid derivatives and salts thereof, anthranilic acid menthyl esters, benzotriazole derivatives and indole derivatives.

The UV filters cited below which can be used within the context of the present invention are preferred but naturally are not limiting.

### UV-A AND UV-B FILTERS

Ordinarily, UV filters are contained in amounts of 0.05 wt% to 50 wt% and preferably 0.5 wt% to 40 wt%. UVB filters can be oil-soluble or water-soluble. Examples of suitable oil-soluble substances include:
- 3-benzylidene camphor or 3-benzylidene norcamphor and derivatives thereof, such as 3-(4-methylbenzylidene)camphor;
- 4-aminobenzoic acid derivatives, preferably 4-(dimethylamino)benzoic acid-2-ethylhexyl ester, 4-(dimethylamino)benzoic acid-2-octyl ester, and 4-(dimethylamino)-benzoic acid amyl ester;
- esters of cinnamic acid, preferably 4-methoxycinnamic acid-2-ethylhexyl ester, 4-methoxycinnamic acid propyl ester, 4-methoxycinnamic acid isoamyl ester, and 2-cyano-3,3-phenylcinnamic acid-2-ethylhexyl ester (octocrylene);
- esters of salicylic acid, preferably salicylic acid-2-ethylhexyl ester, salicylic acid-4-isopropyl benzyl ester, and salicylic acid homomenthyl ester;
- benzophenone derivatives, preferably 2-hydroxy-4-methoxybenzophenone, 2-hydroxy-4-methoxy-4'-methylbenzophenone, and 2,2'-dihydroxy-4-methoxybenzophenone;
- esters of benzylmalonic acid, preferably 4-methoxybenzylmalonic acid di-2-ethylhexyl ester;
- triazine derivatives such as 2,4,6-trianilino-(p-carbo-2'-ethyl-1'-hexyloxy)-1,3,5-triazine and octyl triazone or dioctyl butamidotriazone (Uvasorb® HEB);
- propane-1,3-diones such as 1-(4-tert-butylphenyl)-3-(4'-methoxyphenyl)propane-1,3-dione; and
- ketotricyclo (5.2.1.0) decane derivatives.

Examples of suitable water-soluble substances include:
- 2-phenylbenzimidazole-5-sulfonic acid and alkali, alkaline earth, ammonium, alkylammonium, alkanolammonium, and glucammonium salts thereof;
- 1H-benzimidazole-4,6-disulfonic acid, 2,2'-(1,4-phenylene)bis-disodium salt (Neo Heliopan® AP);
- sulfonic acid derivatives of benzophenones, preferably 2-hydroxy-4-methoxybenzophenone-5-sulfonic acid and salts thereof;
- sulfonic acid derivatives of 3-benzylidene camphor such as 4-(2-oxo-3-bornylidene methyl)benzene sulfonic acid, 2-methyl-5-(2-oxo-3-bornylidene)sulfonic acid, and salts thereof.

Typical examples of particularly suitable UV-A filters include benzoyl methane derivatives such as 1-(4'-tert-butylphenyl)-3-(4'-methoxyphenyl)propane-1,3-dione, 4-tert-butyl-4'-methoxy-dibenzoyl methane (Parsol® 1789), 2-(4-diethylamino-2-hydroxybenzoyl)-benzoic acid hexyl ester (Uvinul® A Plus), 1-phenyl-3-(4'-isopropylphenyl)-propane-1,3-dione, as well as enamine compounds. Of course, the UV-A and UV-B filters can also be used in mixtures. Particularly suitable combinations consist of benzoyl methane derivatives such as 4-tert-butyl-4'-methoxydibenzoyl methane (Parsol® 1789) and 2-cyano-3,3-phenylcinnamic acid-2-ethyl-hexyl ester (octocrylene) in combination with esters of cinnamic acid, preferably 4-methoxycinnamic acid-2-ethylhexyl ester and/or 4-methoxycinnamic acid propyl ester and/or 4-methoxycinnamic acid-isoamyl ester. Such combinations have been advantageous combined with water-soluble filters such as 2-phenylbenzimidazole-5-sulfonic acid and alkali, alkaline earth, ammonium, alkylammonium, alkanolammonium, and glucammonium salts thereof.

In a preferred embodiment the (cosmetic) preparation of the present invention comprises at least an additional UV absorbing substance selected from the group consisting of:
- 3-(4'-trimethylammonium)benzylidenebornan-2-one methyl sulphate
- homomenthyl salicylate (Neo Heliopan®HMS)
- terephthalylidenedibornanesulphonic acid and salts (Mexoryl®SX)
- 3-(4'-sulpho)benzylidenebornan-2-one and salts
- 2-ethylhexyl 2-cyano-3,3-diphenylacrylate (Neo Heliopan®303)
- N-[(2 and 4)-[2-(oxoborn-3-ylidene)methyl]benzyl]acrylamide polymer
- 2-ethylhexyl p-methoxycinnamate (Neo Heliopan®AV)
- ethyl p-aminobenzoate (25 mol) ethoxylated
- isoamyl p-methoxycinnamate (Neo Heliopan®E1000)
- 2-phenylbenzimidazole sulfonic acid (Neo Heliopan® Hydro) and its salts
- 2,4,6-trianilino(p-carbo-2'-ethylhexyl-1'-oxy)-1,3,5-triazine (Uvinul®T150)
- phenol, 2-(2H-benzotriazol-2-yl)-4-methyl-6-(2-methyl-3(1,3,3,3-tetramethyl-1-(trimethylsilyl)oxy)disiloxyanyl)propyl), (Mexoryl®XL)
- 4,4'-[(6-[4-(1,1-dimethyl)aminocarbonyl)phenylamino]-1,3,5-triazin-2,4-diyl)-diimino]bis(benzoic acid 2-ethylhexyl ester), (Uvasorb® HEB)
- 3-(4'-methylbenzylidene)-d,l-camphor (Neo Helipan®MBC)
- 2-ethylhexyl salicylate (Neo Helipan®OS)
- 2-ethylhexyl 4-dimethylaminobenzoate (Padimate O)
- 4-hydroxy-4-methoxybenzophenone - 5-sulfonate (Benzophenone-4, Sulisobenzone) and its salts,
- benzylidenemalonate-polysiloxane (Parsol®SLX)
- menthyl anthranilate (Neo Heliopan®MA)
or mixtures thereof.

### LIGHT PROTECTION PIGMENTS

In addition to the above-mentioned soluble substances, insoluble light protection pigments, specifically finely-dispersed metal oxides or salts, are also suitable for this purpose. Examples of particularly suitable metal oxides are zinc oxide and titanium dioxide, as well as iron, zirconium, silicon, manganese, aluminum, and cerium oxides and mixtures thereof. Silicates (talc), barium sulfate, or zinc stearate can be used as examples of suitable salts. The oxides and salts are used in the form of pigments for skin care and skin protection emulsions and decorative cosmetics. In this case, the particles should have an average diameter of less than 100 nm, preferably 5 to 50 nm, and particularly preferably 15 to 30 nm. They can be spherical in shape, but particles can also be used that are ellipsoid or whose shape is other than spherical The pigments may also be surface-treated, i.e. in a hydrophilized or hydrophobized form. Typical examples are coated titanium dioxides such as titanium dioxide T 805 (Degussa), Eusolex® T2000, Eusolex® T, Eusolex® T-ECO, Eusolex® T-S, Eusolex® T-Aqua, Eusolex® T-45D (all Merck), and Uvinul TiO₂ (BASF). Examples of suitable hydrophobic coating agents in this case are primarily silicones, particularly trialkoxyoctyl silane or simethicone. So-called micro- or nanopigments are preferably used in sun protection agents. Micronized zinc oxides such as Z-COTE® or Z-COTE HP1® are preferably used.

In a preferred embodiment the light protection pigment is selected from microfine titanium dioxide, Zinc oxide, Microfine zinc oxide. When titanium dioxide is chosen as the light protection pigment, it is advantageous that its total amount ranges from 0.1% to 10.0 wt.% of the formulation. When Zinc Oxide is chosen as the light protection pigment it is advantageous that its total amount ranges from 0.1 wt.% to 10.0 wt.% of the formulation and when one or more triazine organic pigment(s) are chosen it is advantageous that its total amount ranges from 0.1% to 10.0 wt.% based on the total amount of the formulation.

### SECONDARY SUN PROTECTION FACTORS

Besides the groups of primary sun protection factors mentioned above, secondary sun protection factors of the antioxidant type may also be used. Secondary sun protection factors of the antioxidant type interrupt the photochemical reaction chain which is initiated when UV rays penetrate into the skin. Typical examples are amino acids (for example glycine, histidine, tyrosine, tryptophane) and derivatives thereof, imidazoles (for example urocanic acid) and derivatives thereof, peptides, such as D,L-carnosine, D-carnosine, L-carnosine and derivatives thereof (for example anserine), carotinoids, carotenes (for example alpha-carotene, beta-carotene, lycopene) and derivatives thereof, chlorogenic acid and derivatives thereof, liponic acid and derivatives thereof (for example dihydroliponic acid), aurothioglucose, propylthiouracil and other thiols (for example thioredoxine, glutathione, cysteine, cystine, cystamine and glycosyl, N-acetyl, methyl, ethyl, propyl, amyl, butyl and lauryl, palmitoyl, oleyl, alpha-linoleyl, cholesteryl and glyceryl esters thereof) and their salts, dilaurylthiodipropionate, distearylthiodipropionate, thiodipropionic acid and derivatives thereof (esters, ethers, peptides, lipids, nucleotides, nucleosides and salts) and sulfoximine compounds (for example butionine sulfoximines, homocysteine sulfoximine, butionine sulfones, penta-, hexa- and hepta-thionine sulfoximine) in very small compatible dosages, also (metal) chelators (for example alpha-hydroxyfatty acids, palmitic acid, phytic acid, lactoferrine), alpha-hydroxy acids (for example citric acid, lactic acid, malic acid), humic acid, bile acid, bile extracts, bilirubin, biliverdin, EDTA, EGTA and derivatives thereof, unsaturated fatty acids and derivatives thereof (for example linoleic acid, oleic acid), folic acid and derivatives thereof, ubiquinone and ubiquinol and derivatives thereof, vitamin C and derivatives thereof (for example ascorbyl palmitate, Mg ascorbyl phosphate, ascorbyl acetate), tocopherols and derivatives (for example vitamin E acetate), vitamin A and derivatives (vitamin A palmitate) and coniferyl benzoate of benzoin resin, rutinic acid and derivatives thereof, glycosyl rutin, ferulic acid, furfurylidene glucitol, carnosine, butyl hydroxytoluene, butyl hydroxyanisole, nordihydroguaiac resin acid, nordihydroguaiaretic acid, trihydroxybutyrophenone, uric acid and derivatives thereof, mannose and derivatives thereof, superoxide dismutase, titanium dioxide (for example dispersions in ethanol), zinc and derivatives thereof (for example ZnO, ZnSO₄), selenium and derivatives thereof (for example selenium methionine), stilbenes and derivatives thereof (for example stilbene oxide, trans-stilbene oxide) and derivatives of these active substances suitable for the purposes of the invention (salts, esters, ethers, sugars, nucleotides, nucleosides, peptides and lipids).

Advantageous inorganic secondary light protection pigments are finely dispersed metal oxides and metal salts which are also mentioned in WO 2005 123101 A1. The total quantity of inorganic pigments, in particular hydrophobic inorganic micro-pigments in the finished cosmetic preparation according to the present invention is advantageously from 0.1 to 30% by weight, preferably 0.5 to 10.0% by weight, in each case based on the total weight of the preparation.

Also preferred are particulate UV filters or inorganic pigments, which can optionally be hydrophobed, can be used, such as the oxides of titanium (TiO₂), zinc (ZnO), iron (Fe₂O₃), zirconium (ZrO₂), silicon (SiO₂), manganese (e.g. MnO), aluminium (Al₂O₃), cerium (e.g. Ce₂O₃) and/or mixtures thereof.

### ANTI-AGEING ACTIVES

In the context of the invention, anti-ageing or biogenic agents are, for example antioxidants, matrix-metalloproteinase inhibitrors (MMPI), skin moisturizing agents, glycosaminglycan stimulkators, anti-inflammatory agents, TRPV1 antagonists and plant extracts.

**Antioxidants.** amino acids (preferably glycine, histidine, tyrosine, tryptophane) and derivatives thereof, imidazoles (preferably urocanic acid) and derivatives thereof, peptides, preferably D,L-carnosine, D-carnosine, L-carnosine and derivatives thereof (preferably anserine), carnitine, creatine, matrikine peptides (preferably lysyl-threonyl-threonyl-lysyl-serine) and palmitoylated pentapeptides, carotenoids, carotenes (preferably alpha-carotene, beta-carotene, lycopene) and derivatives thereof, lipoic acid and derivatives thereof (preferably dihydrolipoic acid), aurothioglucose, propyl thiouracil and other thiols (preferably thioredoxine, glutathione, cysteine, cystine, cystamine and glycosyl, N-acetyl, methyl, ethyl, propyl, amyl, butyl and lauryl, palmitoyl, oleyl, gamma-linoleyl, cholesteryl, glyceryl and oligoglyceryl esters thereof) and salts thereof, dilauryl thiodipropionate, distearyl thiodipropionate, thiodipropionic acid and derivatives thereof (preferably esters, ethers, peptides, lipids, nucleotides, nucleosides and salts) and sulfoximine compounds (preferably buthionine sulfoximines, homocysteine sulfoximine, buthionine sulfones, penta-, hexa-, heptathionine sulfoximine) in very small tolerated doses (e.g. pmol to µmol/kg), also (metal) chelators (preferably alpha-hydroxy fatty acids, palmitic acid, phytic acid, lactoferrin, alpha-hydroxy acids (preferably citric acid, lactic acid, malic acid), humic acid, bile acid, bile extracts, tannins, bilirubin, biliverdin, EDTA, EGTA and derivatives thereof), unsaturated fatty acids and derivatives thereof (preferably gamma-linolenic acid, linoleic acid, oleic acid), folic acid and derivatives thereof, ubiquinone and derivatives thereof, ubiquinol and derivatives thereof, vitamin C and derivatives (preferably ascorbyl palmitate, Mg ascorbyl phosphate, ascorbyl acetate, ascorbyl glucoside), tocopherols and derivatives (preferably vitamin E acetate), vitamin A and derivatives (vitamin A palmitate) and coniferyl benzoate of benzoic resin, rutinic acid and derivatives thereof, flavonoids and glycosylated precursors thereof, in particular quercetin and derivatives thereof, preferably alpha-glucosyl rutin, rosmarinic acid, carnosol, carnosolic acid, resveratrol, caffeic acid and derivatives thereof, sinapic acid and derivatives thereof, ferulic acid and derivatives thereof, curcuminoids, chlorogenic acid and derivatives thereof, retinoids, preferably retinyl palmitate, retinol or tretinoin, ursolic acid, levulinic acid, butyl hydroxytoluene, butyl hydroxyanisole, nordihydroguaiac acid, nordihydroguaiaretic acid, trihydroxybutyrophenone, uric acid and derivatives thereof, mannose and derivatives thereof, zinc and derivatives thereof (preferably ZnO, ZnSO₄), selenium and derivatives thereof (preferably selenium methionine), superoxide dismutase, stilbenes and derivatives thereof (preferably stilbene oxide, trans-stilbene oxide) and the derivatives (salts, esters, ethers, sugars, nucleotides, nucleosides, peptides and lipids) of these cited active ingredients which are suitable according to the invention or extracts or fractions of plants having an antioxidant effect, preferably green tea, rooibos, honeybush, grape, rosemary, sage, melissa, thyme, lavender, olive, oats, cocoa, ginkgo, ginseng, liquorice, honeysuckle, sophora, pueraria, pinus, citrus, Phyllanthus emblica or St. John's wort, grape seeds, wheat germ, Phyllanthus emblica, coenzymes, preferably coenzyme Q10, plastoquinone and menaquinone. Preferred antioxidants are selected from the group consisting of vitamin A and derivatives, vitamin C and derivatives, tocopherol and derivatives, preferably tocopheryl acetate, and ubiquinone.

**Matrix-Metalloproteinase inhibitors (MMPI).** Preferred compositions comprise matrix-metalloproteinase inhibitors, especially those inhibiting matrix-metalloproteinases enzymatically cleaving collagen, selected from the group consisting of: ursolic acid, retinyl palmitate, propyl gallate, precocenes, 6-hydroxy-7-methoxy-2,2-dimethyl-1(2H)-benzopyran, 3,4-dihydro-6-hydroxy-7-methoxy-2,2-dimethyl-1(2H)-benzopyran, benzamidine hydrochloride, the cysteine proteinase inhibitors N-ethylmalemide and epsilon-amino-n-caproic acid of the serinprotease inhibitors: phenylmethylsufonylfluoride, collhibin (company Pentapharm; INCI: hydrolysed rice protein), oenotherol (company Soliance; INCI: propylene glycol, aqua, Oenothera biennis root extract, ellagic acid and ellagitannins, for example from pomegranate), phosphoramidone hinokitiol, EDTA, galardin, EquiStat (company Collaborative Group; apple fruit extract, soya seed extract, ursolic acid, soya isoflavones and soya proteins), sage extracts, MDI (company Atrium; INCI: glycosaminoglycans), fermiskin (company Silab/Mawi; INCI: water and lentinus edodes extract), actimp 1.9.3 (company Expanscience/Rahn; INCI: hydrolysed lupine protein), lipobelle soyaglycone (company Mibelle; INCI: alcohol, polysorbate 80, lecithin and soy isoflavones), extracts from green and black tea and further plant extracts, which are listed in WO 02 069992 A1 (see tables 1-12 there, incorporated herein by reference), proteins or glycoproteins from soya, hydrolysed proteins from rice, pea or lupine, plant extracts which inhibit MMPs, preferably extracts from shitake mushrooms, extracts from the leaves of the Rosaceae family, sub-family Rosoideae, quite particularly extracts of blackberry leaf (preferably as described in WO 2005 123101 A1**,** incorporated herein by reference) as e.g. SymMatrix (company Symrise, INCI: Maltodextrin, Rubus Fruticosus (Blackberry) Leaf Extract). Preferred actives of are selected from the group consisting of retinyl palmitate, ursolic acid, extracts from the leaves of the Rosaceae family, sub-family Rosoideae, genistein and daidzein.

**(Skin-moisturizing agents.** Preferred skin moisturizing agents are selected from the group consisting of alkane diols or alkane triols comprising 3 to 12 carbon atoms, preferably C₃-C₁₀-alkane diols and C₃-C₁₀-alkane triols. More preferably the skin moisturizing agents are selected from the group consisting of: glycerol, 1,2-propylene glycol, 1,2-butylene glycol, 1,3-butylene glycol, 1,2-pentanediol, 1,2-hexanediol, 1,2-octanediol and 1,2-decanediol.

**Glycosaminoglycan stimulators.** Preferred compositions comprise substances stimulating the synthesis of glycosaminoglycans selected from the group consisting of hyaluronic acid and derivatives or salts, Subliskin (Sederma, INCI: Sinorhizobium Meliloti Ferment Filtrate, Cetyl Hydroxyethylcellulose, Lecithin), Hyalufix (BASF, INCI: Water, Butylene Glycol, Alpinia galanga leaf extract, Xanthan Gum, Caprylic/Capric Triglyceride), Stimulhyal (Soliance, INCI: Calcium ketogluconate), Syn-Glycan (DSM, INCI: Tetradecyl Aminobutyroylvalylaminobutyric Urea Trifluoroacetate, Glycerin, Magnesium chloride), Kalpariane (Biotech Marine), DC Upregulex (Distinctive Cosmetic Ingredients, INCI: Water, Butylene Glycol, Phospholipids, Hydrolyzed Sericin), glucosamine, N-acetyl glucosamine, retinoids, preferably retinol and vitamin A, Arctium lappa fruit extract, Eriobotrya japonica extract, Genkwanin, N-Methyl-L-serine, (-)-alpha-bisabolol or synthetic alpha-bisabolol such as e.g. Dragosantol and Dragosantol 100 from Symrise, oat glucan, Echinacea purpurea extract and soy protein hydrolysate. Preferred actives are selected from the group consisting of hyaluronic acid and derivatives or salts, retinol and derivatives, (-)-alpha-bisabolol or synthetic alpha-bisabolol such as e.g. Dragosantol and Dragosantol 100 from Symrise, oat glucan, Echinacea purpurea extract, Sinorhizobium Meliloti Ferment Filtrate, Calcium ketogluconate, Alpinia galanga leaf extract and tetradecyl aminobutyroylvalylaminobutyric urea trifluoroacetate.

**Anti-inflammatory agents.** The compositions may also contain anti-inflammatory and/or redness and/or itch ameliorating ingredients, in particular steroidal substances of the corticosteroid type selected from the group consisting of hydrocortisone, dexamethasone, dexamethasone phosphate, methyl prednisolone or cortisone, are advantageously used as anti-inflammatory active ingredients or active ingredients to relieve reddening and itching, the list of which can be extended by the addition of other steroidal anti-inflammatories. Non-steroidal anti-inflammatories can also be used. Examples which can be cited here are oxicams such as piroxicam or tenoxicam; salicylates such as aspirin, disalcid, solprin or fendosal; acetic acid derivatives such as diclofenac, fenclofenac, indomethacin, sulindac, tolmetin or clindanac; fenamates such as mefenamic, meclofenamic, flufenamic or niflumic; propionic acid derivatives such as ibuprofen, naproxen, benoxaprofen or pyrazoles such as phenylbutazone, oxyphenylbutazone, febrazone or azapropazone. Anthranilic acid derivatives, in particular avenanthramides described in WO 2004 047833 A1**,** are preferred antiitch ingredients in a composition according to the present invention.

Also useful are natural or naturally occurring anti-inflammatory mixtures of substances or mixtures of substances that alleviate reddening and/or itching, in particular extracts or fractions from camomile, Aloe vera, Commiphora species, Rubia species, willow, willow-herb, oats, calendula, arnica, St John's wort, honeysuckle, rosemary, Passiflora incarnata, witch hazel, ginger or Echinacea; preferably selected from the group consisting of extracts or fractions from camomile, Aloe vera, oats, calendula, arnica, honeysuckle, rosemary, witch hazel, ginger or Echinacea, and/or pure substances, preferably alpha-bisabolol, apigenin, apigenin-7-glucoside, gingerols, shogaols, gingerdiols, dehydrogingerdiones, paradols, natural or naturally occuring avenanthramides, preferably tranilast, avenanthramide A, avenanthramide B, avenanthramide C, non-natural or non-naturally occuring avenanthramides, preferably dihydroavenanthramide D, dihydroavenanthramide E, avenanthramide D, avenanthramide E, avenanthramide F, boswellic acid, phytosterols, glycyrrhizin, glabridin and licochalcone A; preferably selected from the group consisting of alpha-bisabolol, natural avenanthramides, non-natural avenanthramides, preferably dihydroavenanthramide D (as described in WO 2004 047833 A1), boswellic acid, phytosterols, glycyrrhizin, and licochalcone A, and/or allantoin, panthenol, lanolin, (pseudo-)ceramides [preferably Ceramide 2, hydroxypropyl bispalmitamide MEA, cetyloxypropyl glyceryl methoxypropyl myristamide, N-(1-hexadecanoyl)-4-hydroxy-L-proline (1-hexadecyl) ester, hydroxyethyl palmityl oxyhydroxypropyl palmitamide], glycosphingolipids, phytosterols, chitosan, mannose, lactose and β-glucans, in particular 1,3-1,4-β-glucan from oats.

**TRPV1 antagonists.** Suitable compounds which reduce the hypersensitivity of skin nerves based on their action as TRPV1 antagonists, encompass e.g. trans-4-tert-butyl cyclohexanol as described in WO 2009 087242 A1**,** or indirect modulators of TRPV1 by an activation of the µ-receptor, e.g. acetyl tetrapeptide-15, are preferred.

**Botanical extracts.** The compositions may also contain various extracts of plants, such as for example extracts of *Ginkgo biloba, Oleacea europensis, Glyzyrrhiza glabra, Vaccinium myrtillus, Trifolium pratense, Litchi sinensis, Vitis, vinifera, Brassica oleracea, Punica granatum, Petroselinium crispum, Centella asiatica, Passiflora incarnata, Medicago sativa, Melissa officinalis, Valeriana officinalis, Castanea sativa, Salix alba* and *Hapagophytum procumbens.*

### PRESERVATIVES

Examples of suitable preservatives include phenoxyethanol, formaldehyde solution, parabens, pentane diol, or sorbic acid, as well as the silver complexes known under the name Surfacine® and the additional substance classes listed in Appendix 6, sections A and B of the Cosmetics Ordinance.

Preference is made to preservatives which are selected from the group consisting of o-cymen-5-ol, benzoic acid and para-hydroxybenzoic acid, their esters and salts, Benzyl benzoate, propionic acid and its salts, salicylic acid and its salts, 2,4-hexadienoic acid (sorbic acid) and its salts, levulinic acid and its salts, anisic acid and its salts, perillic acid and its salts, cinnamic acid and its salts, formaldehyde and paraformaldehyde, 4-hydroxy benzaldehyde, ortho-, meta-, and para-anisic aldehyde, cinnamic aldehyde, cinnamic alcohol, 2-hydroxybiphenyl ether and its salts, 2-zinc-sulfidopyridine N-oxide, inorganic sulfites and bisulfites, sodium iodate, chlorobutanolum, 4-ethylmercury-(II)5-amino-1,3-bis(2-hydroxybenzoic acid), its salts and esters, dehydracetic acid, formic acid, 1,6-bis(4-amidino-2-bromophenoxy)-n-hexane and its salts, the sodium salt of ethylmercury-(II)-thiosalicylic acid, phenylmercury and its salts, 10-undecylenic acid and its salts, 5-amino-1,3-bis(2-ethylhexyl)-5-methyl-hexahydropyrimidine, 5-bromo-5-nitro-1,3-dioxane, 2-bromo-2-nitro-1,3-propanediol, 2,4-dichlorobenzyl alcohol, N-(4-chlorophenyl)-N'-(3,4-dichlorophenyl)-urea, 4-chloro-m-cresol, 2,4,4'-trichloro-2'-hydroxy-diphenyl ether, 4-chloro-3,5-dimethylphenol, 1,1'-methylene-bis(3-(1-hydroxymethyl-2,4-dioximidazolidin-5-yl)urea), poly-(hexame-thylenediguanide) hydrochloride, (Benzyloxymethoxy)-methanol hexamethylenetetramine, 1-(3-chloroallyl)-3,5,7-triaza-1-azonia-adamantane chloride, 1-(4-chlorophenoxy)-1-(1H-imidazol-1-yl)-3,3-dimethyl-2-butanone, 1,3-bis-(hydroxyme-thyl)-5,5-dimethyl-2,4-imidazolidinedione, 1,2-dibromo-2,4-dicyanobutane, 2,2'-methylene-bis(6-bromo-4-chlorophenol), bromochlorophene, mixture of 5-chloro-2-methyl-3(2H)-isothiazolinone, 2-methyl-3(2H)-isothiazolinone and with magnesium chloride and magnesium nitrate, 2-Octyl-2H-isothiazol-3-one, 1,2-benzisothiazol-3(2H)-one, 2-benzyl-4-chlorophenol, 3-(4-Chlorphenoxy)-1,2-propanediol (Chlorphenesin), 2-chloroacetamide, chlorhexidine, chlorhexidine acetate, chlorhexidine gluconate, chlorhexidine hydrochloride, N-alkyl(C12-C22)trimethyl-ammonium bromide and chloride, 4,4-dimethyl-1,3-oxazolidine, N-hydroxymethyl-N-(1,3-di(hydroxymethyl)-2,5-dioxoimidazolidin-4-yl)-N'-hydroxymethylurea, 1,6-bis(4-amidino-phenoxy)-n-hexane and its salts, glutaraldehyde, 5-ethyl-1-aza-3,7-dioxabicyclo(3.3.0)octane, 3-(4-chlorophenoxy)-1,2-propanediol, hyamines, alkyl-(C8-C18)-dimethyl-benzyl-ammonium chloride, alkyl-(C8-C18)-dimethyl-benzylammonium bromide, alkyl-(C8-C18)-dimethyl-benzyl-ammonium saccharinate, benzyl hemiformal, 3-iodo-2-propynyl butylcarbamate, sodium hydroxymethyl-aminoacetate or sodium hydroxymethyl-aminoacetate, imidazolidinylurea, diazolidinylurea, sodium hydroxymethylglycinate, DMDM hydantoin, Tropolone, (Ethylendioxy)dimethanol, 2-Brom-2-(brommethyl)pentandinitril, N-(3-Aminopropyl)-N-dodecylpropan-1,3-diamin, α,α',α"-trimethyl-1,3,5-triazine-1,3,5(2H,4H,6H)-triethanol, pyridine-2-thiol-1-oxide, sodium salt, Tetrahydro-1,3,4,6-tetrakis(hydroxymethyl)imidazo[4,5-d]imidazol-2,5(1H,3H)-dion, 1,3-bis(hydroxymethyl)-1-(1,3,4-tris(hydroxymethyl)-2,5-dioxoimidazolidin-4-yl)urea (Diazolidinyl Urea), 1,3-Bis(hydroxymethyl)-5,5-dimethylimidazolidine-2,4-dione, 3-Acetyl-2-hydroxy-6-methyl-4H-pyran-4-one, cetyl pyridium chloride, ethyl-N-alpha-dodecanoyl-L-arginate hydrochloride, caprylhydroxamic acid, sorbohydroxamic acid, and their mixtures.

### ANTIMICROBIAL AGENTS

Suitable anti-microbial agents are, in principle, all substances effective against Gram-positive bacteria, such as, for example, 4- hydroxybenzoic acid and its salts and esters, N-(4-chlorophenyl)-N'-(3,4- dichlorophenyl)urea, 2,4,4'-trichloro-2'-hydroxy-diphenyl ether (triclosan), 4-chloro-3,5-dimethyl-phenol, 2,2'-methylenebis(6-bromo-4- chlorophenol), 3-methyl-4-(1-methylethyl)phenol, 2-benzyl-4-chloro-phenol, 3-(4-chlorophenoxy)-1,2-propanediol, 3-iodo-2-propynyl butylcarbamate, chlorhexidine, 3,4,4'-trichlorocarbanilide (TTC), antibacterial fragrances, thymol, thyme oil, eugenol, oil of cloves, menthol, mint oil, farnesol, phenoxyethanol, glycerol monocaprate, glycerol monocaprylate, glycerol monolaurate (GML), diglycerol monocaprate (DMC), salicylic acid N-alkylamides, such as, for example, n-octylsalicylamide or n- decylsalicylamide.

### ENZYME INHIBITORS

Suitable enzyme inhibitors are, for example, esterase inhibitors. These are preferably trialkyl citrates, such as trimethyl citrate, tripropyl citrate, triisopropyl citrate, tributyl citrate and, in particular, triethyl citrate (Hydagen CAT). The substances inhibit enzyme activity, thereby reducing the formation of odour. Other substances which are suitable esterase inhibitors are sterol sulfates or phosphates, such as, for example, lanosterol, cholesterol, campesterol, stigmasterol and sitosterol sulfate or phosphate, dicarboxylic acids and esters thereof, such as, for example, glutaric acid, monoethyl glutarate, diethyl glutarate, adipic acid, monoethyl adipate, diethyl adipate, malonic acid and diethyl malonate, hydroxycarboxylic acids and esters thereof, such as, for example, citric acid, malic acid, tartaric acid or diethyl tartrate, and zinc glycinate.

### FILM-FORMING AGENTS, ANTIDANDRUFF AGENTS, AND EXPANDING AGENTS

Examples of common film-forming agents include chitosan, microcrystalline chitosan, quaternized chitosan, polyvinyl pyrrolidone, vinyl pyrrolidone-vinyl acetate copolymerisates, polymers of the acrylic acid series, quaternary cellulose derivatives, collagen, hyaluronic acid or salts thereof, and similar compounds.

Examples of suitable antidandruff active ingredients include piroctone olamine (1-hydroxy-4-methyl-6-(2,4,4-trimethylpentyl)-2-(1H)-pyridinone monoethanolamine salt), Baypival® (climbazole), Ketoconazol®, (4-acetyl-1-{-4-[2-(2,4-dichlorophenyl)r-2-(1H-imidazol-1-ylmethyl)-1,3-dioxylan-c-4-ylmethoxyphenyl}piperazine, ketoconazole, Elubiol, selenium disulfide, colloidal sulfur, sulfur polyethylene glycol sorbitan monooleate, sulfur rizinol polyethoxylate, sulfur-tar distillates, salicylic acid (or in combination with hexachlorophene), undecylenic acid monoethanolamide sulfosuccinate Na salt, Lamepon® UD (protein-undecylenic acid condensate), zinc pyrithione, aluminum pyrithione, and magnesium pyrithione/dipyrithione magnesium sulfate.

Examples of suitable expanding agents for aqueous phases are montmorillonite, clay mineral substances, Pemulen, as well as alkyl-modified carbopol products (Goodrich). Further suitable polymers or expanding agents can be seen in the overview of R. Lochhead in Cosm. Toil. 108, 95 (1993**).**

### CARRIERS

Suitable carriers or solvents for the present invention may be selected from the group consisting of water, alcohols, esters, butylene glycol, dipropylene glycol, pentylene glycol, 1,2-hexane diol, caprylyl glycol, decylene glycol, ethanol, ethoxydiglycol, ethyl acetate, glycerol, propanol, isopropanol, macrogols, propyl propylene glycol(2) methyl ether, propyl propylene glycol(3) methyl ether, propylene carbonate, propylene glycol, triethylene glycol, isoparaffin, amyl acetate, amyl benzoate, benzyl acetate, butyl acetate, butylene glycol, butyl lactate, butooctyl benzoate, butooctyl salicylate, C10-C13 alkanes, C14-C17 alkanes, C11-C15 cycloalkanes, caprylyl butyrate, isoparaffins, diacetin, triacetin dicaprylyl ether, dicaprylyl maleate, and mixtures thereof. Most preferred are glycerol, propylene glycol, butylene glycol, dipropylene glycol, pentylene glycol, 1,2-hexane diol, caprylyl glycol, decylene glycolas.

Preferred solid carrier materials, which may be a component of a preparation according to the invention are hydrocolloids, such as starches, degraded starches, chemically or physically modified starches, dextrins, (powdery) maltodextrins (preferably with a dextrose equivalent value of 5 to 25, preferably of 10 - 20), lactose, silicon dioxide, glucose, modified celluloses, gum arabic, ghatti gum, traganth, karaya, carrageenan, pullulan, curdlan, xanthan gum, gellan gum, guar flour, carob bean flour, alginates, agar, pectin and inulin and mixtures of two or more of these solids, in particular maltodextrins (preferably with a dextrose equivalent value of 15 - 20), lactose, silicon dioxide and/or glucose.

### HYDROTROPES

Moreover, hydrotropes, such as ethanol, isopropyl alcohol, or polyols can be used in order to improve flow properties; these substances largely correspond to the carriers described at the outset. In this case, suitable polyols preferably have 2 to 15 carbon atoms, and at least two hydroxyl groups. The polyols can also include other functional groups, particularly amino groups, or be modified with nitrogen. Typical examples are
- glycerol;
- alkylene glycols, such as ethylene glycol, diethylene glycol, propylene glycol, butylene glycol, hexylene glycol, as well as polyethylene glycols with an average molecular weight of 100 to 1,000 daltons;
- technical oligoglycerol mixtures having a degree of self-condensation of 1.5 to 10 such as technical diglycerol mixtures with a diglycerol content of 40 to 50 wt%;
- methylol compounds, particularly trimethylol ethane, trimethylol propane, trimethylol butane, pentaerythrite, and dipentaerythrite;
- lower alkyl glycosides, particularly those with 1 to 8 carbon atoms in the alkyl residue, such as methyl and butyl glycoside;
- sugar alcohols with 5 to 12 carbon atoms, such as sorbite or mannite,
- sugars with 5 to 12 carbon atoms, such as glucose or saccharose;
- amino sugars, such as glucamine;
- dialcoholamines, such as diethanolamine or 2-amino-1,3-propane diol.

### ORGANIC SOLVENT

The household preparations of the present inventions, in particular such as liquid light or heavy duty detergents may comprise organic solvents, preferably those miscible with water. Polydiols, ethers, alcohols, ketones, amides and/or esters are preferably used as the organic solvent for this in amounts of 0 to 90 wt. %, preferably 0.1 to 70 wt. %, particularly 0.1 to 60 wt. %. Low molecular weight polar substances, such as for example, methanol, ethanol, propylene carbonate, acetone, acetonylacetone, diacetone alcohol, ethyl acetate, 2-propanol, ethylene glycol, propylene glycol, glycerin, diethylene glycol, dipropylene glycol monomethyl ether and dimethylformamide or their mixtures are preferred.

### ENZYMES

Suitable enzymes include, in particular, those from the classes of hydrolases, such as proteases, esterases, lipases or lipolytic enzymes, amylases, cellulases or other glycosyl hydrolases and mixtures thereof. In the wash, all these hydrolases contribute to removing stains such as protein, fat or starchy stains and against graying. Moreover, cellulases and other glycosyl hydrolases can contribute to increased softness of the textile and to color retention by removing pilling and micro fibrils. Oxidoreductases can also be added to the bleaches or to inhibit the color transfer. Enzymatic active materials obtained from bacterial sources or fungi such as bacillus subtilis, bacillus licheniformis, streptomyceus griseus and humicola insolens are particularly well suited. Proteases of the subtilisin type and particularly proteases that are obtained from bacillus lentus are preferably used. Here, mixtures of enzymes are of particular interest, for example, proteases and amylases or proteases and lipases or lipolytic enzymes or proteases and cellulases or cellulases and lipase or lipolytic enzymes or proteases, amylases and lipases or lipolytic enzymes or proteases, lipases or lipolytic enzymes and cellulases, in particular, however proteases and/or lipase-containing mixtures or mixtures with lipolytic enzymes. Examples of such lipolytic enzymes are the known cutinases. Peroxidases or oxidases have also proved to be suitable in certain cases. The suitable amylases particularly include .alpha.-amylases, iso-amylases, pullulanases and pectinases. Cellobiohydrolases, endoglucanases and beta-glucosidases or mixtures thereof, which are also known as cellobiases, are preferred cellulases. As the different cellulase types differ in their CMCase and avicelase activities, the required activities can be adjusted by controlled mixtures of the cellulases. The content of the enzymes or enzyme mixtures may be, for example, about 0.1 to 5% by weight and is preferably 0.1 to about 3% by weight.

### BUILDERS

**Zeolites.** Fine crystalline, synthetic zeolites containing bound water can be used as builders, for example, preferably zeolite A and/or P. Zeolite MAP.RTM. (commercial product of the Crosfield company), is particularly preferred as the zeolite P. However, zeolite X and mixtures of A, X, Y and/or P are also suitable. A co-crystallized sodium/potassium aluminum silicate from Zeolite A and Zeolite X, which is available as Vegobond® RX. (commercial product from Condea Augusta S.p.A.), is also of particular interest. Preferably, the zeolite can be used as a spray-dried powder. For the case where the zeolite is added as a suspension, this can comprise small amounts of nonionic surfactants as stabilizers, for example, 1 to 3 wt. %, based on the zeolite, of ethoxylated C₁₂-C₁₈ fatty alcohols with 2 to 5 ethylene oxide groups, C₁₂-C₁₄ fatty alcohols with 4 to 5 ethylene oxide groups or ethoxylated isotridecanols. Suitable zeolites have an average particle size of less than 10µm (test method: volumetric distribution Coulter counter) and preferably comprise 18 to 22 wt. %, particularly 20 to 22 wt. % of bound water. Apart from this, phosphates can also be used as builders.

**Layered silicates.** Suitable substitutes or partial substitutes for phosphates and zeolites are crystalline, layered sodium silicates. These types of crystalline layered silicates are described, for example, in European Patent Application EP 0164514 A1. Preferred crystalline layered silicates are those obtained for example, from the process described in International Patent Application WO 91/08171 A1.

**Amorphous silicates.** Preferred builders also include amorphous sodium silicates with a modulus (Na₂O:SiO₂ ratio) of 1:2 to 1:3.3, preferably 1:2 to 1:2.8 and more preferably 1:2 to 1:2.6, which dissolve with a delay and exhibit multiple wash cycle properties. The delay in dissolution compared with conventional amorphous sodium silicates can have been obtained in various ways, for example, by surface treatment, compounding, compressing/compacting or by over-drying. In the context of this invention, the term "amorphous" also means "X-ray amorphous". In other words, the silicates do not produce any of the sharp X-ray reflexions typical of crystalline substances in X-ray diffraction experiments, but at best one or more maxima of the scattered X-radiation, which have a width of several degrees of the diffraction angle. However, particularly good builder properties may even be achieved where the silicate particles produce indistinct or even sharp diffraction maxima in electron diffraction experiments. This is to be interpreted to mean that the products have microcrystalline regions between 10 and a few hundred nm in size, values of up to at most 50 nm and especially up to at most 20 nm being preferred. This type of X-ray amorphous silicates, which similarly possess a delayed dissolution in comparison with the customary water glasses, are described, for example, in German Patent Application DE 4400024 A1. Compacted/densified amorphous silicates, compounded amorphous silicates and over dried X-ray-amorphous silicates are particularly preferred.

**Phosphates.** Also the generally known phosphates can also be added as builders, in so far that their use should not be avoided on ecological grounds. The sodium salts of the orthophosphates, the pyrophosphates and especially the tripolyphosphates are particularly suitable. Their content is generally not more than 25 wt. %, preferably not more than 20 wt. %, each based on the finished composition. In some cases it has been shown that particularly tripolyphosphates, already in low amounts up to maximum 10 wt. %, based on the finished composition, in combination with other builders, lead to a synergistic improvement of the secondary washing power. Preferred amounts of phosphates are under 10 wt. %, particularly 0 wt. %.

### COBUILDERS

**Polycarboxylic acids.** Useful organic cobuilders are, for example, the polycarboxylic acids usable in the form of their sodium salts of polycarboxylic acids, wherein polycarboxylic acids are understood to be carboxylic acids that carry more than one acid function. These include, for example, citric acid, adipic acid, succinic acid, glutaric acid, malic acid, tartaric acid, maleic acid, fumaric acid, sugar acids, aminocarboxylic acids, nitrilotriacetic acid (NTA) and its derivatives and mixtures thereof. Preferred salts are the salts of polycarboxylic acids such as citric acid, adipic acid, succinic acid, glutaric acid, tartaric acid, sugar acids and mixtures thereof.

**Organic acids.** Acids per se can also be used. Besides their building effect, the acids also typically have the property of an acidifying component and, hence also serve to establish a relatively low and mild pH in detergents or cleansing compositions. Citric acid, succinic acid, glutaric acid, adipic acid, gluconic acid and any mixtures thereof are particularly mentioned in this regard. Further suitable acidifiers are the known pH regulators such as sodium hydrogen carbonate and sodium hydrogen sulfate.

**Polymers.** Particularly suitable polymeric cobuilders are polyacrylates, which preferably have a molecular weight of 2,000 to 20,000 g/mol. By virtue of their superior solubility, preferred representatives of this group are again the short-chain polyacrylates, which have molecular weights of 2,000 to 10,000 g/mol and, more particularly, 3,000 to 5,000 g/mol. Suitable polymers can also include substances that consist partially or totally of vinyl alcohol units or its derivatives.

Further suitable copolymeric polycarboxylates are particularly those of acrylic acid with methacrylic acid and of acrylic acid or methacrylic acid with maleic acid. Copolymers of acrylic acid with maleic acid, which comprise 50 to 90 wt. % acrylic acid and 50 to 10 wt. % maleic acid, have proven to be particularly suitable. Their relative molecular weight, based on free acids, generally ranges from 2,000 to 70,000 g/mol, preferably 20,000 to 50,000 g/mol and especially 30,000 to 40,000 g/mol. The (co)polymeric polycarboxylates can be added either as an aqueous solution or preferably as powder. In order to improve the water solubility, the polymers can also comprise allylsulfonic acids as monomers, such as, for example, allyloxybenzene sulfonic acid and methallyl sulfonic acid as in the EP 0727448 B1.

Biodegradable polymers comprising more than two different monomer units are particularly preferred, examples being those comprising, as monomers, salts of acrylic acid and of maleic acid, and also vinyl alcohol or vinyl alcohol derivatives, as in DE 4300772 A1, or those comprising, as monomers, salts of acrylic acid and of 2-alkylallyl sulfonic acid, and also sugar derivatives. Further preferred copolymers are those that are described in German Patent Applications DE 4303320 A1 and DE 4417734 A1 and preferably include acrolein and acrylic acid/acrylic acid salts or acrolein and vinyl acetate as monomers.

Similarly, other preferred builders are polymeric aminodicarboxylic acids, salts or precursors thereof. Those polyaspartic acids or their salts and derivatives disclosed in German Patent Application DE 19540086 A1 as having a bleach-stabilizing action in addition to cobuilder properties are particularly preferred.

Further suitable builders are polyacetals that can be obtained by treating dialdehydes with polyol carboxylic acids that possess 5 to 7 carbon atoms and at least 3 hydroxyl groups, as described in European Patent Application EP 0280223 A1. Preferred polyacetals are obtained from dialdehydes like glyoxal, glutaraldehyde, terephthalaldehyde as well as their mixtures and from polycarboxylic acids like gluconic acid and/or glucoheptonic acid.

**Carbohydrates.** Further suitable organic cobuilders are dextrins, for example, oligomers or polymers of carbohydrates that can be obtained by the partial hydrolysis of starches. The hydrolysis can be carried out using typical processes, for example, acidic or enzymatic catalyzed processes. The hydrolysis products preferably have average molecular weights in the range of 400 to 500,000 g/mol. A polysaccharide with a dextrose equivalent (DE) of 0.5 to 40 and, more particularly, 2 to 30 is preferred, the DE being an accepted measure of the reducing effect of a polysaccharide in comparison with dextrose, which has a DE of 100. Both maltodextrins with a DE between 3 and 20 and dry glucose syrups with a DE between 20 and 37 and also so-called yellow dextrins and white dextrins with relatively high molecular weights of 2,000 to 30,000 g/mol may be used. A preferred dextrin is described in British Patent Application 94 19 091.

The oxidized derivatives of such dextrins concern their reaction products with oxidizing compositions that are capable of oxidizing at least one alcohol function of the saccharide ring to the carboxylic acid function. Such oxidized dextrins and processes for their manufacture are known for example, from European Patent Applications EP 0232202 A1. A product oxidized at C6 of the saccharide ring can be particularly advantageous.

**Oxydisuccinates** and other derivatives of disuccinates, preferably ethylenediamine disuccinate are also further suitable cobuilders. Here, ethylene diamine-N,N'-disuccinate (EDDS), the synthesis of which is described for example, in US 3,158,615, is preferably used in the form of its sodium or magnesium salts. In this context, glycerine disuccinates and glycerine trisuccinates are also particularly preferred, such as those described in US 4,524,009. Suitable addition quantities in zeolite-containing and/or silicate-containing formulations range from 3 to 15% by weight.

**Lactones.** Other useful organic co-builders are, for example, acetylated hydroxycarboxylic acids and salts thereof which optionally may also be present in lactone form and which contain at least 4 carbon atoms, at least one hydroxyl group and at most two acid groups. Such cobuilders are described, for example, in International Patent Application WO 95/20029 A1.

### OILS REPELLANTS

In addition, the compositions can also comprise components that positively influence the oil and fat removal from textiles during the wash (so-called soil repellents). This effect is particularly noticeable when a textile is dirty and had been previously already washed several times with an inventive detergent that comprised this oil- or fat-removing component. The preferred oil and fat removing components include, for example, nonionic cellulose ethers such as methyl cellulose and methyl hydroxypropyl cellulose with a content of methoxy groups of 15 to 30 wt. % and hydroxypropoxy groups of 1 to 15 wt. %, each based on the nonionic cellulose ether, as well as polymers of phthalic acid and/or terephthalic acid or their derivatives known from the prior art, particularly polymers of ethylene terephthalates and/or polyethylene glycol terephthalates or anionically and/or nonionically modified derivatives thereof. From these, the sulfonated derivatives of the phthalic acid polymers and the terephthalic acid polymers are particularly preferred.

### INORGANIC SALTS

Further suitable ingredients of the composition are water-soluble inorganic salts such as bicarbonates, carbonates, amorphous silicates or mixtures of these; alkali carbonate and amorphous silicate are particularly used, principally sodium silicate with a molar ratio Na₂O:SiO₂ of 1:1 to 1:4.5, preferably of 1:2 to 1:3.5. Preferred compositions comprise alkaline salts, builders and/or cobuilders, preferably sodium carbonate, zeolite, crystalline, layered sodium silicates and/or trisodium citrate, in amounts of 0.5 to 70 wt. %, preferably 0.5 to 50 wt. %, particularly 0.5 to 30 wt. % anhydrous substance.

### FOAM INHIBITORS

In some cases it is required that no foam formation arises. Especially when used in automatic washing processes, it can be advantageous to add conventional foam inhibitors to the compositions. Suitable foam inhibitors include for example, soaps of natural or synthetic origin, which have a high content of C₁₈-C₂₄ fatty acids. Suitable non-surface-active types of foam inhibitors are, for example, organopolysiloxanes and mixtures thereof with microfine, optionally silanised silica and also paraffins, waxes, microcrystalline waxes and mixtures thereof with silanised silica or bis-stearyl ethylenediamide. Mixtures of various foam inhibitors, for example, mixtures of silicones, paraffins or waxes, are also used with advantage. Preferably, the foam inhibitors, especially silicone-containing and/or paraffin-containing foam inhibitors, are loaded onto a granular, water-soluble or dispersible carrier material. Especially in this case, mixtures of paraffins and bis-stearylethylene diamides are preferred.

### FRAGRANCES, FLAVOURS, AROMA COMPOUNDS AND PERFUME OILS

Fragrances, flavours, aroma compounds and perfume oils are well known in the art can be added to the compositions of the invention. The Fragrances, flavours, aroma compounds and perfume oils may be obtained from natural sources or prepared by organic synthesis. According to the present invention the terms "fragrances, flavours, aroma compounds and perfume oils" are herewith used equivalent to each.

Flavours and fragrances can be chosen from synthetic flavouring liquid and/or oils derived from plants leaves, flowers, fruits and so forth, and combinations thereof. Representative flavouring liquids include: artificial, natural or synthetic fruit flavours such as eucalyptus, lemon, orange, banana, grape, lime, apricot and grapefruit oils and fruit essences including apple, strawberry, cherry, orange, pineapple and so forth; bean and nut derived flavours such as coffee, cocoa, cola, peanut, almond and so forth; and root derived flavours such as licorice or ginger, flower extracts (lily, lavender, rose, jasmine, neroli, ylang-ylang), stems and leaves (geranium, patchouli, petitgrain), fruits (anise, coriander, caraway, juniper), fruit peels (bergamot, lemon, orange), roots (nutmeg, angelica, celery, cardamom, costus, iris, calmus), woods (pinewood, sandalwood, guaiac wood, cedarwood, rosewood), herbs and grasses (tarragon, lemon grass, sage, thyme), needles and branches (spruce, fir, pine, dwarf pine), resins and balsams (galbanum, elemi, benzoin, myrrh, olibanum, opoponax). Animal raw materials such as civet and beaver may also be used. Typical synthetic perfume compounds are products of the ester, ether, aldehyde, ketone, alcohol and hydrocarbon type. Examples of perfume compounds of the ester type are benzyl acetate, phenoxyethyl isobutyrate, p-tert-butyl cyclohexylacetate, linalyl acetate, dimethyl benzyl carbinyl acetate, phenyl ethyl acetate, linalyl benzoate, benzyl formate, ethylmethyl phenyl glycinate, allyl cyclohexyl propionate, styrallyl propionate and benzyl salicylate. Ethers include benzyl ethyl ether, while aldehydes include linear alkanals containing 8 to 18 carbon atoms, citral, citronellal, citronellyl oxyacetaldehyde, cyclamen aldehyde, hydroxycitronellal, lilial and bourgeonal; examples of suitable ketones are the ionones, α-isomethylionone, and methyl cedryl ketone. Suitable alcohols are anethol, citronellol, eugenol, isoeugenol, geraniol, linalool, phenylethyl alcohol, and terpineol. The hydrocarbons chiefly include the terpenes and balsams. However, mixtures of different perfume compounds are preferred that produce an agreeable fragrance together. Other suitable perfume oils include essential oils of low volatility that are mostly used as aroma components, such as sage oil, camomile oil, clove oil, melissa oil, mint oil, cinnamon leaf oil, lime-blossom oil, juniper berry oil, vetiver oil, olibanum oil, galbanum oil, ladanum oil, and lavendin oil. Preferably, bergamot oil, dihydromyrcenol, lilial, lyral, citronellol, phenylethyl alcohol, α-hexylcinnamaldehyde, geraniol, benzyl acetone, cyclamen aldehyde, linalool, boisambrene forte, ambroxan, indole, hedione, sandelice, citrus oil, mandarin oil, orange oil, allyl amyl glycolate, cyclovertal, lavendin oil, clary oil, β-damascone, geranium oil bourbon, cyclohexyl salicylate, Vertofix Coeur, Iso-E-Super, Fixolide NP, evernyl, iraldein gamma, phenylacetic acid, geranyl acetate, benzyl acetate, rose oxide, romillate, irotyl, and floramate are used either individually or in mixtures.

It is of particular advantage if the flavour and fragrance compositions according to the invention comprise at least one flavour and / or fragrance, preferably two, three, four, five, six, seven, eight or more flavours and / or fragrances chosen from the following flavours and fragrances:

### Aldehydes

Examples for suitable flavours and fragrances showing an aldehyde structure encompass melonal, triplal, ligustral, adoxal, anisaldehyde, cymal, ethylvanillin, florhydral, floralozon, helional, heliotropin, hydroxycitronellal, koavon, laurinaldehyde, canthoxal, lyral, lilial, adoxal, anisaldehyde, cumal, methyl-nonyl-acetaldehyde, citronellal, citronellyloxyacetaldehyde, cyclamenaldehyde, bourgeonal, p-tert.-bucinal, phenylacetaldehyde, undecylenaldehyde, vanillin; 2,6,10-trimethyl-9-undecenal, 3-dodecen-I-al, α-n-Amylzimtaldehyde, 4-methoxy-benz-aldehyde, benzaldehyde, 3-(4-tert-butylphenyl)-propanal,2-methyl-3-(para-methoxy-phe-nylpropanal), 2-methyl-4-(2,6,6-trimethyl-2(I)-cyclohexen-1-yl)butanal,3-phenyl-2-pro-penal, cis-/trans-3,7-dimethyl-2,6-octadien-I-al, 3,7-dimethyl-6-octen-I-al,[(3,7-dimethyl-6-octenyl)-xy]-cetaldehyde, 4-isopropylbenzyaldehyde, 1,2,3,4,5,6,7,8-octahydro-8,8-dimethyl-2-naphthaldehyde, 2,4-dimethyl-3-cyclohexen-1-carboxyaldehyde, 2-methyl-3-(isopropyl-phenyl)propanal, decyl aldehyde, 2,6-dimethyl-5-heptenal; 4-(tricyclo[5.2.1.0 (2,6)]-decylidene-8)-butanal; octahydro-4,7-methano-IH-indenecarboxaldehyde; 3-ethoxy-4-hydroxybenzaldehyde, para-ethyl-alpha,alpha-dimethylhydrozimtaldehyde, α-methyl-3,4-(methylenedioxy)-hydrocinnamaldehyde, 3,4-methylenedioxybenzaldehyde, α-n-hexyl-cinnamaldehyde, m-cymene-7-carboxaldehyde, α-methylphenylacetaldehyde, 7-hydroxy-3,7-dimethyl octanal, undecenal, 2,4,6-trimethyl-3-cyclohexene-1-carboxalde-hyde,4-(3)(4-methyl-3-pentenyl)-3-cyclohexen-carboxaldehyde, 1-dodecanal, 2,4-dimethyl-cyclohexene-3-carboxaldehyde,4-(4-hydroxy-4-methylpentyl)-3-cylohexene-I-carboxal-dehyde, 7-methoxy-3,7-dimethyloctan-1-al, 2-methyl undecanal, 2-methyl decanal, 1-nonanal, 1-octanal, 2,6,10-trimethyl-5,9-undecadienal, 2-methyl-3-(4-tertbutyl)propanal, 3-(4-ethylphenyl)-2,2-dimethylpropanal, 3-(4-methoxyphenyl)-2-methylpropanal, methylno-nylacetaldehyde, 2-phenylpropan-1-al, 3-phenylprop-2-en-1-al, 3-phenyl-2-pentylprop-2-en-1-al, 3-phenyl-2-hexylprop-2-enal, 3-(4-isopropylphenyl)-2-methylpropan-1-al, 3-(4-ethylphenyl)-2,2-dimethylpropan-1-al, 3-(4-tert-butylphenyl)-2-methyl-propanal, 3-(3,4-Methylendioxy-phenyl)-2-methylpropan-1-al,3-(4-Ethylphenyl)-2,2-dimethylpropanal, 3-(3-Isopropylphenyl)-butan-1-al, 2,6-Dimethylhept-5-en-1-al,Dihydrozimtaldehyde, 1-methyl-4-(4-methyl-3-pentenyl)-3-cyclohexene-1-carboxaldehyde, 5- or 6-Methoxyhexahydro-4,7-methanoindan-1 or 2-carboxyaldehyde, 3,7-dimethyloctan-1-al, 1-undecanal, 10-undecen-1-al, 4-hydroxy-3-methoxybenzaldehyde, 1-methyl-3-(4-methylpentyl)-3-cyclohexene-carboxyaldehyde, 7-hydroxy-3,7-dimethyl-octanal; trans-4-decenal, 2,6-nonadienal, p-tolylacetaldehyde; 4-methylphenylacetaldehyde, 2-methyl-4-(2,6,6-trimethyl-1-cyclohexen-1-yl)-2-butenal, o-methoxyzimtaldehyde, 3,5,6-trimethyl-3-cyclohexenecarboxaldehyde, 3,7-dimethyl-2-methylene-6-octenal, phenoxyacetaldehyde; 5,9-dimethyl-4,8-decadienal, peony aldehyde (6,10-dimethyl-3-oxa-5,9-undecadien-1-al), hexahydro-4,7-methanoindan-1-carboxaldehyde, octanal, 2-methyl octanal, alpha-methyl-4-(I-methylethyl)benzene-acetaldehyde, 6,6-dimethyl-2-norpinene-2-propionaldehyde, p-methyl phenoxy acetaldehyde, 2-methyl-3-phenyl-2-propen-1-al, 3,5,5-trimethylhexanal, hexahydro-8,8-dimethyl-2-naphthaldehyde, 3-propyl-bicyclo[2.2.1]-hept-5-ene-2-carbaldehyde, 9-decenal, 3-methyl-5-phenyl-1-pentanal, methylnonyl acetaldehyde, 1-p-menthene-q-carboxaldehyde, citral or its mixtures, lilial citral, 1-decanal, n-undecanal, n-dodecanal, hlorhydral, 2,4-dimethyl-3-cyclohexen-1-carboxaldehyde 4-methoxybenzaldehyde, 3-methoxy-4-hydroxy-benzalde-hyde, 3-ethoxy-4-hydroxybenzaldehyde, 3,4-methylendioxybenzaldehyde, 3,4-dimethoxybenzaldehyde and their mixtures.

As explained above, said ketones or said aldehydes may show an aliphatic, cycloaliphatic, aromatic, ethylenically unsaturated structure or a mixture of these elements. The components may also include heteroatoms or show a polycyclic structure. Suitable substituents for all these structures are hydroxyl and/or amino groups. Further fragrances are compiled in the following document: Steffen Arctander "Published 1960 and 1969 respectively, Reprinted2000 ISBN: Aroma Chemicals Vol. 1: 0-931710-37-5, Aroma Chemicals Vol. 2: 0-931710-38-3", which is hereby incorporated by reference.

### Ketones

Examples for suitable flavours and fragrances showing a ketone structure encompass buccoxime, iso jasmone, methyl beta naphthyl ketone, moschus indanone, tonalid/moschus plus, α-damascone, β-damascon, δ-damascone, Iso-damascone, damascenone, damarose, methyl-dihydrojasmonate, menthone, carvone, campher, fenchone, alphalonen, β-iononw, dihydro-β-Ionone, γ-methylionone, fleuramone, dihydrojasmone, cis-Jasmon, iso-E-Super, methyl cedrenylk etone, or methyl cedrylon, acetophenone, methyl aceto phenone, p-methoxyacetophenone, methyl-β-naphtyl ketone, benzylacetone, benzophenone, p-hydroxy phenylbutanone, celery Ketone or livescon, 6-osopropyl-deca-hydro-2-naphtone, dimethyloctenone, freskomenth, 4-(I-ethoxyvinyl)-3,3,5,5,-tetramethylv cyclohexanone, methylheptenone, 2-(2-(4-Methyl-3-cyclohexen-1-yl)propyl)-cyclopentanone, 1-(p-men-thene-6(2)-yl)-1-propanone,4-(4-Hydroxy-3-methoxyphenyl)-2-butanone, 2-Acetyl-3,3-di-methyl-norbornan, 6,7-dihydro-1,1,2,3,3-pentamethyl-4(5H)-indanone, 4-damascol, dulcinyl or cassione, gelsone, hexalone, isocyclemone E, Methylcyclocitrone, methyl lavender ketone, orivone, p-tert-butyl cyclohexanone, verdone, delphone, muscone, neobutenone, plicatone, veloutone, 2,4,4,7-tetramethyl-oct-6-en-3-one, tetrameran, hedion and their mixtures. The preferred ketones are selected from the group comprising α-damascone, δ-damascone, iso-damascone, carvone, γ-methyl ionone, Iso-E-Super, 2,4,4,7-tetramethyl-oct-6-en-3-one, benzylacetone, β-damascone, damascenone, methyl dihydrojasmonate, methyl cedrylone, hedione and their mixtures

### Alcohols

Suitable flavour and fragrance alcohols encompass for example 10-undecen-1-ol, 2,6-dimethylheptan-2-ol, 2-methylbutanol, 2-methylpentanol,2-phenoxyethanol, 2-phenylpropanol, 2-tert-Butycyclohexanol, 3,5,5-trimethylcyclohexanol, 3-hexanol, 3-methyl-5-phenylpentanol, 3-octanol, 1-octen-3-ol, 3-phenylpropanol,4-heptenol, 4-isopropylcyclohexanol, 4-tert-butycyclohexanol, 6,8-dimethyl-2-nonanol,6-nonen-1-ol, 9-decen-1-ol, α-methyl benzylalcohol, α-terpineol, amylsalicylat, benzyl alcohol, benzyl salicylate, β-terpineol, butyl salicylate, citronellol, cyclohexyl salicylate, decanol, dihydromyrcenol, dimethyl benzylcarbinol, dimethyl heptanol, dimethyl octanol, ethyl salicylate, ethyl vanilin, anethol, eugenol, geraniol, heptanol, hexyl salicylat, isoborneol, isoeugenol, isopulegol, linalool, menthol, myrtenol, n-hexanol, nerol, nonanol, octanol, para-menthan-7-ol, phenylethylalkohol, phenol, phenyl salicylat, tetrahydro geraniol, tetrahydro linalool, thymol, trans-2-cis-6-nonadienol, trans-2-nonen-1-ol, trans-2-octenol, undecanol, vanillin, cinnamalcohol and their mixtures.

### Esters

Examples for suitable flavours and fragrances showing a ketone structure encompass benzyl acetate, phenoxyisobutyrate, p-tert.-butylcyclohexylacetate, linalylacetate, dimethylbenzylcarbinylacetate (DMBCA), phenylethylacetate, benzylacetate, ethylmethylphenylglycinate, allylcyclohexylpropionate, styrallylpropionate, benzylsalicylate, cyclohexylsalicylate, floramat, melusat, jasmacyclatat and their mixtures.

### Ethers

Examples for suitable flavours and fragrances showing a ketone structure encompass benzylethyl ether or ambroxan

### Perfume oils

Suitable flavours and fragrances may also be on the base of perfume oils, which are mixtures of natural and synthetic perfumes. Natural perfumes include the extracts of blossoms (lily, lavender, rose, jasmine, neroli, ylang-ylang), stems and leaves (geranium, patchouli, petitgrain), fruits (anise, coriander, caraway, juniper), fruit peel (bergamot, lemon, orange), roots (nutmeg, angelica, celery, cardamom, costus, iris, calmus), woods (pinewood, sandalwood, guaiac wood, cedarwood, rosewood), herbs and grasses (tarragon, lemon grass, sage, thyme), needles and branches (spruce, fir, pine, dwarf pine), resins and balsams (galbanum, elemi, benzoin, myrrh, olibanum, opoponax). Animal raw materials, for example civet and beaver, may also be used. Typical synthetic perfume compounds are products of the ester, ether, aldehyde, ketone, alcohol and hydrocarbon type. Examples of perfume compounds of the ester type are benzyl acetate, phenoxyethyl isobutyrate, p-tert.butyl cyclohexylacetate, linalyl acetate, dimethyl benzyl carbinyl acetate, phenyl ethyl acetate, linalyl benzoate, benzyl formate, ethylmethyl phenyl glycinate, allyl cyclohexyl propionate, styrallyl propionate and benzyl salicylate. Ethers include, for example, benzyl ethyl ether while aldehydes include, for example, the linear alkanals containing 8 to 18 carbon atoms, citral, citronellal, citronellyloxyacetaldehyde, cyclamen aldehyde, hydroxycitronellal, lilial and bourgeonal. Examples of suitable ketones are the ionones, α-isomethylionone and methyl cedryl ketone. Suitable alcohols are anethol, citronellol, eugenol, isoeugenol, geraniol, linalool, phenylethyl alcohol and terpineol. The hydrocarbons mainly include the terpenes and balsams. However, it is preferred to use mixtures of different perfume compounds which, together, produce an agreeable perfume. Other suitable perfume oils are essential oils of relatively low volatility which are mostly used as aroma components. Examples are sage oil, camomile oil, clove oil, melissa oil, mint oil, cinnamon leaf oil, lime-blossom oil, juniper berry oil, vetiver oil, olibanum oil, galbanum oil, ladanum oil and lavendin oil. The following are preferably used either individually or in the form of mixtures: bergamot oil, dihydromyrcenol, lilial, lyral, citronellol, phenylethyl alcohol, hexylcinnamaldehyde, geraniol, benzyl acetone, cyclamen aldehyde, linalool, Boisambrene Forte, Ambroxan, indole, hedione, sandelice, citrus oil, mandarin oil, orange oil, allylamyl glycolate, cyclovertal, lavendin oil, clary oil, damascone, geranium oil bourbon, cyclohexyl salicylate, Vertofix Coeur, Iso-E-Super, Fixolide NP, evernyl, iraldein gamma, phenylacetic acid, geranyl acetate, benzyl acetate, rose oxide, romillat, irotyl and floramat, peppermint oil, spearmint oil, anise oil, star anise oil, caraway oil, eucalyptus oil, fennel oil, citrus oil, wintergreen oil, clove oil, menthol.

### DYES

Suitable dyes that can be used are those suitable and approved for cosmetic purposes, such as those listed in the publication **"Cosmetic Dyes" of the** Farbstoffkommission der Deutschen Forschungsgemeinschaft [Dyes Commission of the German Research Foundation], Verlag Chemie, Weinheim, 1984, pp. 81-106**.** Examples are cochineal red A (C.I. 16255), patent blue V (C.I.42051), indigotin (C.1.73015), chlorophyllin (C.1.75810), quinoline yellow (C.1.47005), titanium dioxide (C.I.77891), indanthrene blue RS (C.I. 69800) and alizarin red (C.I.58000). Luminol can also be included as a luminescent dye. These dyes are ordinarily used in concentrations of 0.001 to 0.1 wt% relative to the entire mixture.

### INDUSTRIAL APPLICATION

The mixture of the present invention is especially useful in cosmetic and household formulations and compositions (preparations), such as personal care compositions or detergents and cleaners.

Therefore, an important object of the invention is a cosmetic or household composition, comprising the above described mixture according to the invention.

A cosmetic composition according to the present invention preferably comprises
(a) from 0.1 to 5% b.w. mixture of the present invention;
(b) from 0 to 5% b.w. flavours or fragrances,
(c) from 0 to 10% b.w. lipophilic components,
(d) from 50 to 99% b.w. water,
(e) from 0.5 to 25% b.w. emulsifiers or surfactants,
(f) from 0 to 50% b.w. alcohols,
   and optionally
(g) from 0 to 45% b.w. oil bodies and/or waxes;
(h) 0 to about 25% b.w. active principles;
   on condition that the amounts add - optionally together with additional ingredients - to 100% b.w.

The inventive compositions are preferably water-based, up to 99 % b.w., preferably up to 95 % b.w. water, based on the total amount of the end product.

The compositions according to the invention may represent o/w or w/o or multiple o/w/o or w/o/w emulsion. They can be used as an intermediate or a final product for example in the form of a lotion, a cream or a stick.

A further object of the present invention is a method of
i) stabilizing a formulation, which is preferably an emulsion, against separation,
ii) reducing the size of water or oil droplets in a formulation, which is preferably an emulsion,
iii) improving foam stability and viscosity of a formulation,
iv) preventing soap cracking
   by adding of a working amount of a mixture of the present invention to a cosmetic or household formulation, wherein the working amount is used to be preferably from 0.01% b.w. to 5% b.w., more preferably 0.02% b.w. to 2% b.w., most preferred 0.1% b.w. to 1% b.w.

Another object of the present invention relates to the process to produce a cosmetic or household composition, comprising the steps of
i) providing at least a mixture according to the invention,
ii) providing a cosmetic or household base formulation, and
iii) mixing the composition from step (i) with the formulation of step (ii) together.

Additional embodiments of the invention concern:

A method of stabilizing an emulsion against separation whereby a working amount of the present mixture of the invention is added.

A further method of reducing the size of water or oil droplets in an emulsion whereby a working amount of the present mixture of the invention is added.

A further method of improving foam stability and viscosity of shampoos whereby a working amount of the present mixture of the invention is added.

The use the present mixture of the invention as stabilizer system against separation for cosmetic or househols compositions.

A further use of the present mixture of the invention for reducing the size of water or oil droplets in an emulsion.

A further use of the present mixture of the invention for improving foam stability and viscosity of shampoos.

### EXAMPLES

### Examples

### Example 1, emulsion stability

O/W emulsions were prepared by heating a lipid phase A and an aqueous phase B separately to approximately 80 °C. Then the aqueous phase B was added to the lipid phase A and mixed by using an Ultra-Turrax stirrer for 2 minutes at 5.000 rpm. The emulsion thus obtained was allowed to cool down for 10 minutes using a vane stirrer at 150 rpm. Finally, the pH value was adjusted to about 6.0.

**Table 1, o/w emulsions**

| | | | | |
|---|---|---|---|---|
| Formulation B is according to the invention, formulation A serves as comparison (Placebo). | | | | |

| | Raw Material | INCI | **% (w/w)** | |
|---|---|---|---|---|
| | | | **Placebo A** | **B** |
| A. | Emulsiphos® (PN677660) | Potassium Cetyl Phosphate, Hydrogenated Palm Glycerides | 2.0 | 2.0 |
| | Lanette O, ex BASF | Cetearyl Alcohol | 0.7 | 0.7 |
| | Neutral Oil | Caprylic/Capric Triglyceride | 4.0 | 4.0 |
| | PCL Liquid 100 (PN660089) | Cetearyl Ethylhexanoate | 2.0 | 2.0 |
| | Abil 350, ex Evonik | Dimethicone | 0.1 | 0.1 |
| | **I-dodecanol** | **Lauryl Alcohol** | - | **0.8** |
| | **Jasmol** | **2-Benzylheptanol** | - | **0.2** |
| B. | Water, demin. | Water (Aqua) | Ad 100 | |
| | Glycerin 99% | Glycerin | 1.5 | 1.5 |

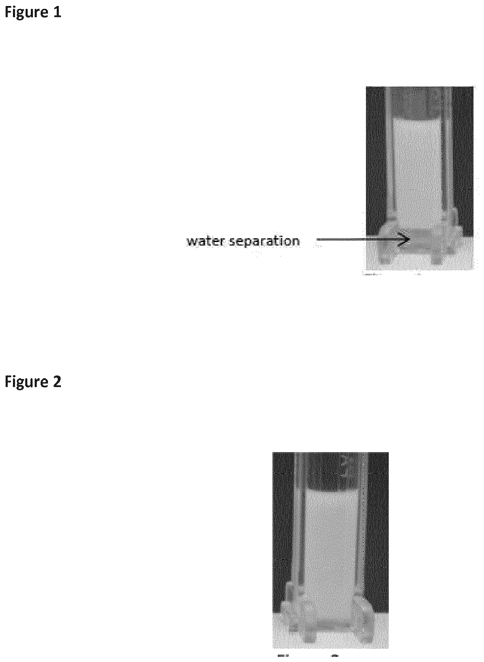

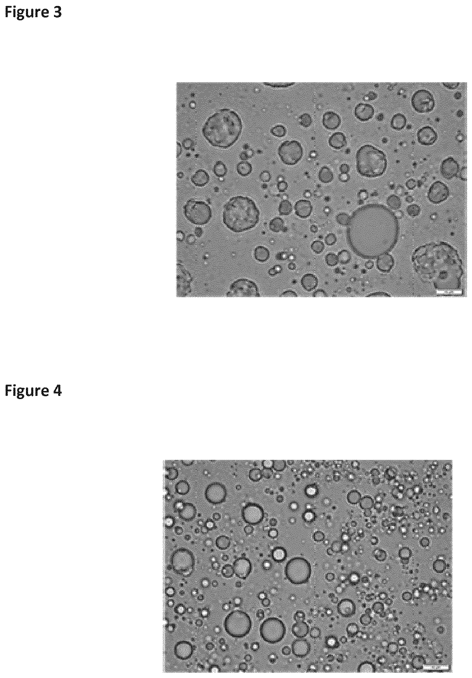

The stability of emulsions A and B were determined by using an analytical photo-centrifuge (LUMiSizer LS6102-41), the results are included in **table 2,** images of the cuvettes after centrifugation are shown in **figure 1** and **figure 2****.**
Furthermore microscopic images were done (see **figure 3** and **figure 4**).

### LUMiSizer LS6102-41, analytical photo-centrifuge

Lumisizer® (ex L.U.M. GmbH) is an instrument, which determines demixing processes of suspensions and emulsions, using a multi-sample analytical photo-centrifuge by measuring the change of transmission during measuring time. An instability index is calculated (Instability Index= value without a unit, calculated by change of transmission over the whole cuvette; 0= very stable; 1= complete separation). The smaller the instability index, the lower the water separation of the emulsion in the cuvette. Smaller values indicate better emulsion stability.

The tests were performed 1 day after preparation of the emulsions A and B.

### Method:

| | |
|---|---|
| Measuring time: | 2.25 h |
| Temperature: | 25°C |
| Speed: | 1250 rpm |

This method simulates a storage time (real-time) for 1 month at 25°C.

**Table 2**

| | **Placebo A** | **B** |
|---|---|---|
| **Instability Indices** | 0.227 | 0.021 |

### Images of cuvettes after centrifugation

**Figure 1** and **figure 2** are images of cuvettes containing emulsions A and B after centrifugation process.

Emulsion B (containing 0.8 % 1-dodecanol and 0.2% 2-benzylheptanol) showed less water separation in comparison to emulsion A (without active) and therefore improved stability after centrifugation according to the method described above. Furthermore the instability index of emulsion B (containing 0.8 % 1-dodecanol and 0.2% 2-benzylheptanol) is much lower versus instability index of emulsion A, which also indicates improved stability.

### Microscopic images (Olympus IX 70, 60 fold enlargement)

Emulsion B (containing 0,8 % 1-dodecanol and 0,2% 2-benzylheptanol, see **Figure 4**) showed smaller oil droplets (particle sizes) in comparison to emulsion A (without active see **Figure 3**). Smaller oil droplets indicate a more homogeneous appearance and better stability of the emulsion.

### Example 2, foam stability in shampoo

Shampoos were prepared with the following production method according to the shampoo formulation of table 3:
Blend Phase A by stirring slowly with a vane stirrer, stop the process when foaming starts. Swell Ucare Poylmer JR-400 in water by stirring and warming up to 50°C. (The dispersion becomes clear and slightly viscous, when swelling process is completed) Add Phases B, C, D, E and F one after the other by stirring with a vane stirrer. pH value should be adjusted to approx. 6.0.

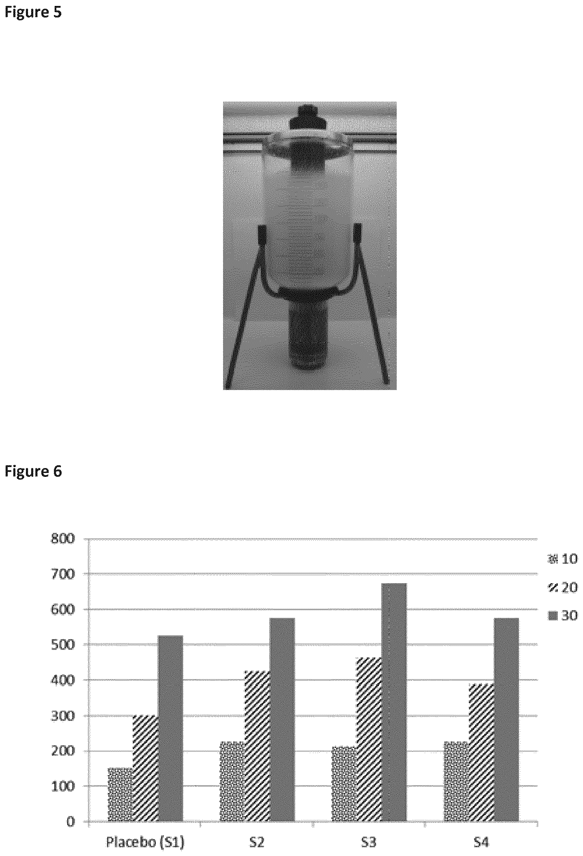

Foam volume and stability measurements (drainage formation) of formulations S1-S4 were performed with a foam and drainage tester. (Ernst Haage, see **figure 5**).

**Table 3, shampoo formulations**

| | | | | | | |
|---|---|---|---|---|---|---|
| Formulations S2-S4 are according to the invention, formulation S1 serves as comparison. (Placebo) | | | | | | |

| A. | **Raw Material** | **INCI** | **w/w%** | | | |
|---|---|---|---|---|---|---|
| | | | **Placebo S1** | **S2** | **S3** | **S4** |
| | Plantacare PS10 | Sodium Laureth Sulfate, Lauryl Glycoside | 17.0 | 17.0 | 17.0 | 17.0 |
| | Citric Acid | Citric Acid | 0.15 | 0.15 | 0.15 | 0.15 |
| | SymDiol 68 | 1,2 Hexandiol, Caprylyl Glycol | 1.0 | 1.0 | 1.0 | 1.0 |
| | EDTA BD | Disodium EDTA | 0.1 | 0.1 | 0.1 | 0.1 |
| | Water, demin. | Water (Auqa) | Ad 100 | | | |
| B. | Ucare Polymer JR-400 | Polyquaternium-10 | 0.2 | 0.2 | 0.2 | 0.2 |
| | Potassium Sorbate | Potassium Sorbate | 0.15 | 0.15 | 0.15 | 0.15 |
| C. | Tego Betain F50 | Cocoamidoproyl Betaine | 5.0 | 5.0 | 5.0 | 5.0 |
| D. | Sodium Chloride | Sodium Chloride | 0.4 | 0.4 | 0.4 | 0.4 |
| E. | **I-dodecanol** | **Lauryl Alcohol** | **-** | **0.08** | **0.24** | **0.4** |
| F. | **Jasmol** | **2-Benzylheptanol** | **-** | **0.02** | **0.06** | **0.1** |

### Test procedure, foam volume :

1% w/w of the shampoos S1-S4 was diluted with demineralized water up to 100%. The samples were homogenized by slowly stirring with a vane stirrer at 50 rpm for 2 minutes (to avoid premature foam formation). 200ml of these diluted formulations S1-S4 were transferred into the foam volume and drainage measuring instrument (Ernst Haage). The stamp of the instrument is pulled up and pushed down for 30 seconds. The volume of the formed foam was recorded after 10, 20 and 30 seconds. The results are included in **table 4.** The forming of drainage liquid was observed from 0-300 seconds in regular intervals. The drainage liquid is released during foam breakage. The results of drainage formation were recorded in **table 5.**

### Results of foam volume and drainage formation measurements

**Table 4, results of foam volume measurements**

| | **time (sec)** | | |
|---|---|---|---|
| **Foam volume (ml)** | **10** | **20** | **30** |
| **Placebo (S1)** | 150 | 300 | 525 |
| **S2** | 225 | 425 | 575 |
| **S3** | 213 | 463 | 675 |
| **S4** | 225 | 388 | 575 |

Samples S2, S3 and S4, containing 0.1-0.5% in sum of 1-dodecanol and 2-benzylheptanol showed more foam volume in comparison to sample S1 (without active, Placebo).

**Figure 6** shows the foam formation in ml of shampoo formulations S2, S3 and S4 in comparison to shampoo S1 (Placebo) after 10, 20 and 30 seconds of testing time. It could be demonstrated that foam volume of formulations S2, S3 and S4 is much higher at all tested time points than foam volume of shampoo S1 (Placebo). The values of foam volume measurements in ml are depicted on the y-axis.

**Table 5, results of drainage liquid formation**

| **[sec] time** | | **0** | **15** | **30** | **60** | **180** | **300** |
|---|---|---|---|---|---|---|---|
| **Volume [ml]** | **Placebo S1** | 80 | 115 | 130 | 150 | 180 | 180 |
| | **S2** | 60 | 100 | 120 | 140 | 170 | 185 |
| | **S3** | 60 | 100 | 115 | 130 | 155 | 165 |
| | **S4** | 50 | 90 | 105 | 120 | 150 | 155 |

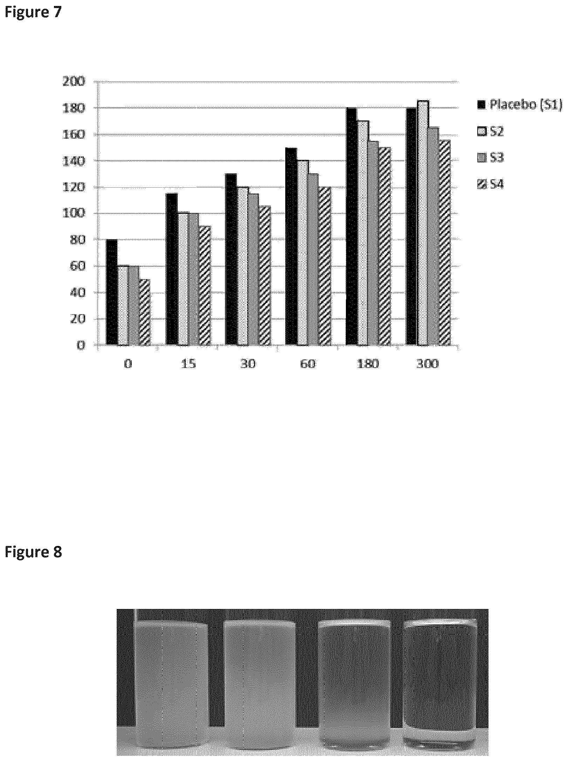

**Figure 7** shows the formation of liquid drainage in ml of shampoo formulations S2, S3 and S4 in comparison to shampoo S1 (Placebo) at test start and after 15, 30, 60, 180 and 300 seconds. It could be demonstrated that drainage formation of formulations S2, S3 and S4 (containing 0.1, 0.3 and 0.5% of 1-dodecanol and 2-benzylheptanol) is clearly lower at all testing times in comparison to shampoo S1 (Placebo). Lower drainage formation indicates improved foam stability and structure for a longer time. The values of liquid drainage in ml were depicted on the y-axis.

### Example 3, solubility improvement of lipophilic ingredients a.) Solubility of fragrance in aqueous/ethanolic solutions

Aqueous/ethanolic solutions were prepared by the formulation according to **table 6.** A fragrance was pre-solved with 1-dodecanol and 2-benzylheptanol. In a next step ethanol and water were added one after the other by stirring. Turbidity measurements (Turb 430 IR, ex WTW) of the test solutions were performed. The higher the value of turbidity units, the more turbid/cloudy the solution appears. These measurements were done with the equipment Turb 430 IR, which determines the nephelometric turbidity units. These results were summarized in **table 7.** Additionally images of the test solutions were done.

**Table 6, aqueous/ethanolic solution**

| | | | | | |
|---|---|---|---|---|---|
| Formulations 3B-3D are according to the invention, formulation 3A serves as comparison (Placebo). | | | | | |

| **Raw Material w/w%** | **INCI** | **3A Placebo** | **3B** | **3C** | **3D** |
|---|---|---|---|---|---|
| **Ethanol** | Ethanol | 50.0 | 50.0 | 50.0 | 50.0 |
| **I-dodecanol** | **Lauryl Alcohol** | - | **0.8** | **1.2** | **1.6** |
| **Jasmol** | **2-Benzylheptanol** | **-** | **0.2** | **0.3** | **0.4** |
| **Water demin.** | Water (Aqua) | Ad 100 | | | |
| **Fragrance (836720)** | - | 4.0 | 4.0 | 4.0 | 4.0 |
| **Comment** | | turbid | turbid | less turbid | clear |

**Table 7, results of turbidity measurements of aqueous/ethanolic solutions**

| | **3A Placebo** | **3B** | **3C** | **3D** |
|---|---|---|---|---|
| **Nephelometric Turbidity Unit (NTU), Turb 430 IR ex WTW** | 662 | 492 | 49 | 0,3 |
| | 676 | 518 | 49 | 0.5 |
| | 684.0 | 581.0 | 48.0 | 0.7 |
| Ø (mean value) | **674** | **530** | **49** | **0.5** |

**Figure 8** shows the images of the test solutions 3A (Placebo), 3B, 3C and 3D (from left to right). It could be demonstrated that with increasing concentration of 1-dodecanol and
2-benzylheptanol the test solutions get more transparent. This indicates a solubility improving effect of 1-dodecanol and 2-benzylheptanol.

**Figure 9** demonstrates the results of turbidity measurements in NTU units (**N**ephelometric **T**urbidity **U**nits) of aqueous/ethanolic solutions 3A (Placebo), 3B, 3C and 3D containing 1.0, 1.5 and 2.0% in sum of 1-dodecanol and 2-benzylheptanol (according to **table** 6). The NTU values were depicted on the y-axis.

It could be seen that with increasing concentration of 1-dodecanol and 2-benzylheptanol the NTU values were decreasing. Decreasing NTU values indicate more transparency, the lower the NTU value, the clearer the solution appears.

### b.) Solubility of fatty acid ester Diisopropyl Adipate in surfactant based solutions.

Diisopropyl Adipate is a lipophilic ingredient and is used in rinse off formulations as a refatting ingredient. Mostly a solubilizer is required to incorporate these kinds of ingredients into a shampoo or shower gel formulation.
1-dodecanol and 2-benzylheptanol were dissolved in sodium laureth sulfate solution by warming up to 40°C. Diisopropyl adipate was added by slightly stirring with a van stirrer.

**Table 8, sodium laureth sulfate/aqueous solution**

| | | | | |
|---|---|---|---|---|
| Formulations 2 and 3 are according to the invention, formulation 1 serves as comparison (Placebo). | | | | |

| **w/w%** | **INCI** | **1** | **2** | **3** |
|---|---|---|---|---|
| **Genapol LRO liquid (28% active content)** | Sodium Laureth Sulfate | 35 | 35 | 35 |
| **Water, demin.** | Water (Aqua) | Ad 100 | | |
| **Isoadipate** | Diisopropyl Adipate | 2.9 | 2.9 | 2.9 |
| **I-dodecanol** | **Lauryl Alcohol** | **-** | **0.4** | **1.2** |
| **Jasmol** | **2-Benzylheptanol** | **-** | **0.1** | **0.3** |

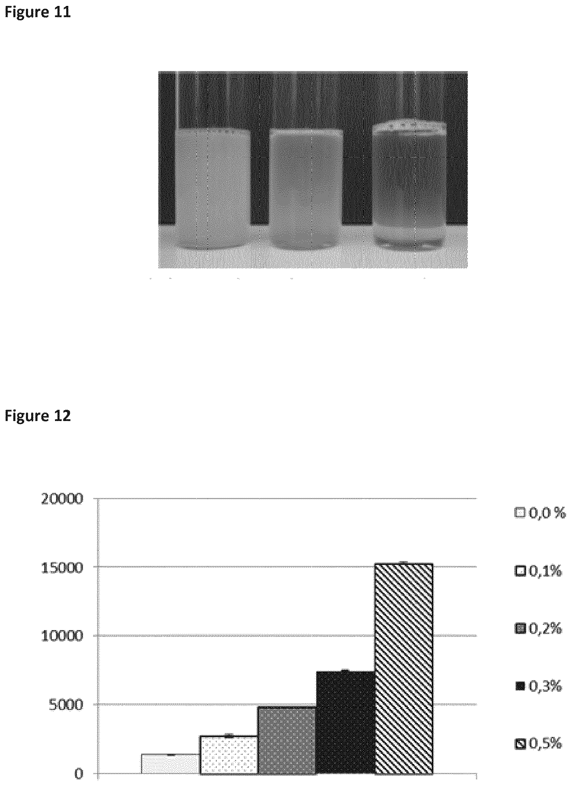

Turbidity measurements (Turb 430 IR, ex WTW) of the test solutions were performed and the results were summarized in **table 9.** Additionally images of the test solutions were taken **(****Figure 11****).**

**Table 9, results of turbidity measurements of solutions 1-3**

| | **1** | **2** | **3** |
|---|---|---|---|
| **Nephelometric Turbidity Unit (NTU), Turb 430 IR ex WTW** | ≥1100 | 152 | 20.7 |
| | ≥1100 | 150 | 21.2 |
| | ≥1100 | 147 | 20.3 |
| **Ø (mean value)** | ≥1100 | 150 | 21 |

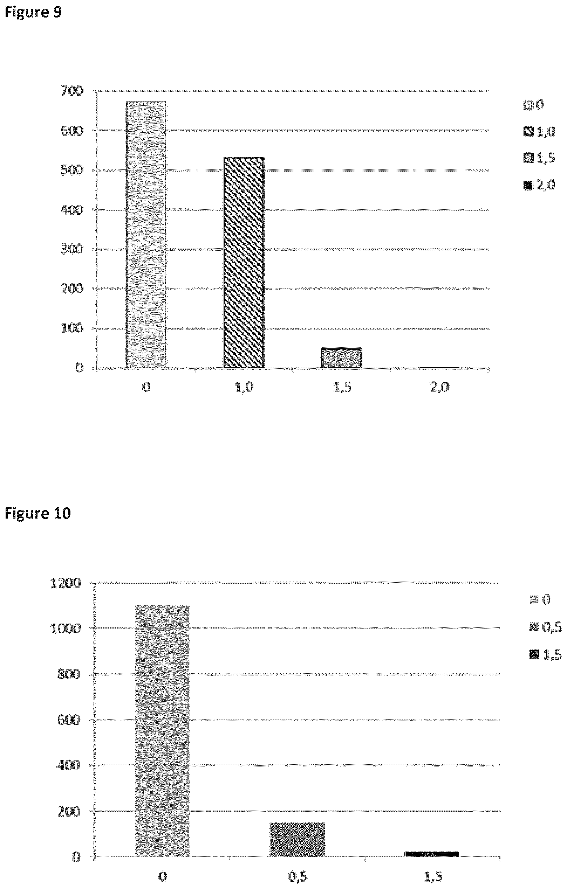

**Figure 10** demonstrates the results of turbidity measurements in NTU units (**N**ephelometric **T**urbidity **U**nits) of sodium laureth sulfate solutions 1, 2 and 3 according to the formulations of table 8. Formulations 2 and 3 contain 0.5 and 1.5% in sum of 1-dodecanol and 2-benzylheptanol. Formulation 1 serves as comparison (Placebo). NTU values were depicted on the y-axis.

**Figure 11** **shows the images of test solutions 1-3. From** left to right: solution 1, 2 and 3. With increasing concentration of 1-dodecanol and 2-benzylheptanol the NTU values were decreasing. This is also illustrated by the images of the test solutions which get more transparent with rising concentrations of 1-dodecanol and 2-benzylheptanol.

### Example 4, viscosity in shampoo

**Table 10, shampoo formulation**

| | | | | | | | |
|---|---|---|---|---|---|---|---|
| Formulations 11-14 are according to the invention, formulation 10 serves as comparison (Placebo). | | | | | | | |

| | **Raw Material w/w%** | **INCI** | **10** | **11** | **12** | **13** | **14** |
|---|---|---|---|---|---|---|---|
| **A.** | Plantacare PS10 | Sodium Laureth Sulfate, Lauryl Glycoside | 17.0 | 17.0 | 17.0 | 17.0 | 17.0 |
| | Citric Acid | Citric Acid | 0.15 | 0.15 | 0.15 | 0.15 | 0.15 |
| | SymDiol 68 | 1,2 Hexandiol, Caprylyl Glycol | 1.0 | 1.0 | 1.0 | 1.0 | 1.0 |
| | EDTA BD | Disodium EDTA | 0.1 | 0.1 | 0.1 | 0.1 | 0.1 |
| | Sodium Chloride | Sodium Chloride | 0.4 | 0.4 | 0.4 | 0.4 | 0.4 |
| **B.** | Water, demin. | Water (Auqa) | Ad 100,0 | | | | |
| | Ucare Polymer JR-400 | Polyquaternium-10 | 0.2 | 0.2 | 0.2 | 0.2 | 0.2 |
| **C.** | Potassium Sorbate | Potassium Sorbate | 0.15 | 0.15 | 0.15 | 0.15 | 0.15 |
| **D.** | Tego Betain F50 | Cocoamidoproyl Betaine | 5.0 | 5.0 | 5.0 | 5.0 | 5.0 |
| **E.** | **I-dodecanol** | **Lauryl Alcohol** | - | **0.08** | **0.16** | **0.24** | **0.4** |
| | **Jasmol** | **2-Benzylheptanol** | - | **0.02** | **0.04** | **0.06** | **0.1** |

### Production method:

Blend Phase A by stirring slowly with a vane stirrer, stop the process when foaming starts. Swell Ucare Poylmer JR-400 in water by stirring and warming up to 50°C. (The dispersion becomes clear and slightly viscous, when swelling process is completed). Add Phases B, C, D and E to A one after the other by stirring with a vane stirrer. pH value should be adjusted to approx. 5.8.

Shampoo formulations were prepared as described above and 24h after production rheology measurements were performed.

### Method:

Haake Rheostress1
Measuring geometry: L12096
Temperature: 23°C
Rotation speed: 5 u/s

**Table 11, results of rheological measurements**

| **Trial** | **Viscosity (mPas)** |
|---|---|
| **10** | 1380.0 |
| **11** | 2739.4 |
| **12** | 4825.5 |
| **13** | 7493.3 |
| **14** | 15285.0 |

**Figure 12** demonstrates the results of viscosity measurements of shampoo formulations 11-14, containing 0.1 - 0.5% is sum of 1-dodecanol and 2-benzylheptanol and formulation 10 (without active, Placebo) according to table 11.
A strong dose dependent increase of viscosity could be observed for formulations 11-14, containing 1-dodecanol and 2-benzylheptanol in comparison to formulation 10 (Placebo). The viscosity of formulation 11-14 was increasing with raising concentrations of 1-dodecanol and 2-benzylheptanol. The viscosity values in mPas were depicted on the y-axis.

### Example 5, bar soap cracking & foam formation

Bar soap base (ex Hirtler), ingredients (INCI):
Sodium Palmate, Sodium Cocoate, Aqua, Glycerin, Sodium Chloride

### Bar soap cracking

2% w/w in sum of 1-dodecanol and 2-benzylheptanol (ratio 4:1) were incorporated into a soap base ex Hirtler by using a soap extruder. The samples were stored at 23°C for 6 months and visually observed.

**Table 12, bar soap (ex Hirtler)**

| | | | |
|---|---|---|---|
| Formulation 2 is according to the invention, formulation 1 serves as comparison (Placebo). | | | |

| **w/w%** | **INCI** | **1** | **2** |
|---|---|---|---|
| **1-dodecanol** | **Lauryl Alcohol** | **-** | **1.6** |
| **Jasmol** | **2-Benzylheptanol** | **-** | **0.4** |
| **Soap base (ex Hirtler)** | Sodium Palmate, Sodium Cocoate, Aqua, Glycerin, Sodium Chloride | Ad 100,0 | |

The samples were stored at 23°C for 6 months and visually observed.

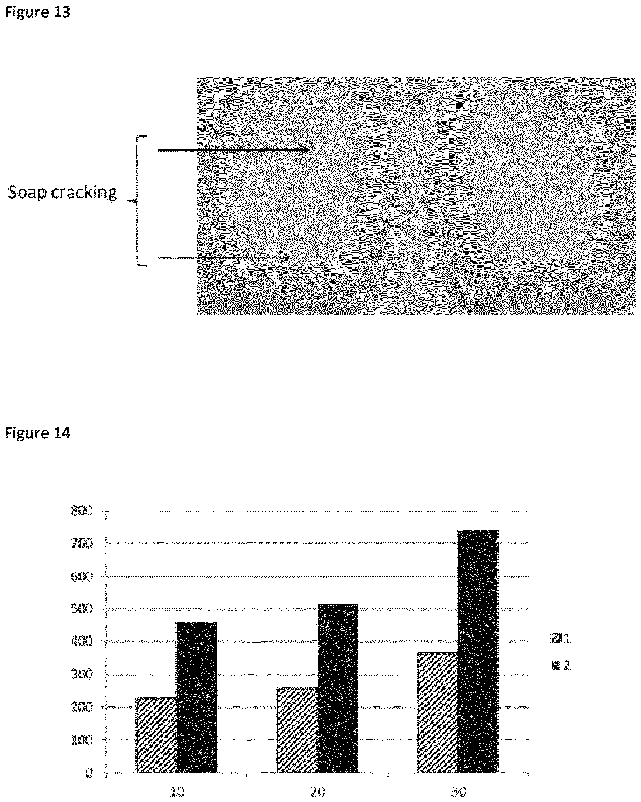

### Figure 13 shows images of soap bars 1 and 2 after storage test of 6 months storage at 23C.

**Left:** Soap formula 1 (without active, Placebo)
**Right:** Soap formula 2, containing 2% 1-dodecanol and 2-benzylheptanol (ratio 4:1)

Soap bar formula 1 (without active, Placebo) showed significant cracking, whereas sample 2 (containing 2% in sum of 1-dodecanol and 2-benzylheptanol 4:1) showed a plain surface without crack formation after storage. The addition of 1-dodecanol and 2-benzylheptanol leads to improved stability and reduced cracking of the bar soap.

### Bar soap, foam and drainage formation

### Test procedure:

1% w/w of the soap bars of formula 1 and 2 were diluted with demineralized water up to 100%. The samples were homogenized by slowly stirring with a vane stirrer at 50 rpm for 2 minutes at 40°C (to avoid premature foam formation). 200ml of these liquids were transferred into the foam volume and drainage measuring instrument (Ernst Haage). The stamp of the instrument is pulled up and pushed down for 30 seconds. The volume of the formed foam was recorded after 10, 20 and 30 seconds. The results are included in **table 13** and demonstrated in **figure 14****.** The forming of drainage liquid was observed from 0-300 seconds in regular intervals. The drainage liquid is released during foam breakage. The results of drainage formation were recorded in **table 14.**

**Table 13, foam volume formation**

| **Foam volume (ml)** | **time (sec)** | | |
|---|---|---|---|
| | **10** | **20** | **30** |
| **soap 1 (Placebo)** | 220 | 250 | 250 |
| | 230 | 260 | 480 |
| **mean value Ø** | **225** | **255** | **365** |
| **soap 2** | 460 | 510 | 730 |
| | 460 | 520 | 750 |
| **mean value Ø** | **460** | **515** | **740** |

**Figure 14** demonstrates the results of foam value measurements of soap bars formula 1 (Placebo) and formula 2 (containing 2% in sum of 1-dodecanol and 2-benzylheptanol 4:1) after 10, 20 and 30 seconds of treatment in the Haage foam and drainage tester. The foam volume of soap formula 2 (containing 1-dodecanol and 2-benzylheptanol) was much higher at every time point in comparison to soap formula 1 (Placebo, without active). The foam volume values in ml were depicted on the y-axis.

**Table 14, drainage formation (ml)**

| | **drainage (ml)** | | | | | |
|---|---|---|---|---|---|---|
| **time (sec)** | **0** | **15** | **30** | **60** | **180** | **300** |
| **Placebo 1** | 80 | 100 | 140 | 160 | 200 | 220 |
| **2** | 75 | 90 | 100 | 125 | 170 | 190 |

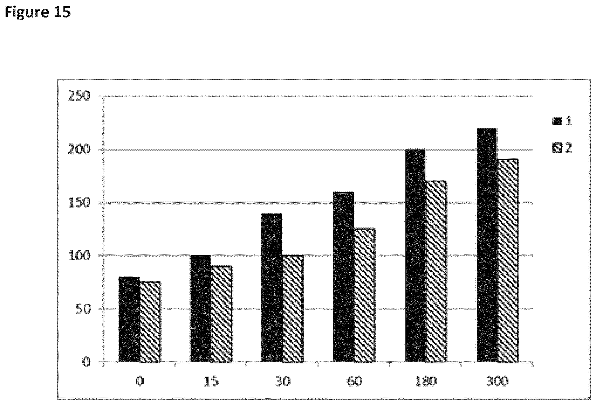

**Figure 15** demonstrates the results of drainage measurements of soap bars formula 1 (Placebo) and formula 2 (containing 2% in sum of 1-dodecanol and 2-benzylheptanol 4:1) after the treatment in the Haage foam and drainage tester. The volume of drainage of soap formula 2 (containing 1-dodecanol and 2-benzylheptanol) is much lower at every time point in comparison to soap formula 1 (Placebo, without active). The drainage volume values in ml were depicted on the y-axis. Less drainage formation indicates better foam stability and structure.

**Table 15: Composition of perfume oil 836720**

| **Ingredients** | **Amount** |
|---|---|
| ALDEHYDE C14 SO-CALLED | 2 |
| ALLYL AMYL GLYCOLATE 10% DPG | 5 |
| ANISIC ALDEHYDE PURE | 5 |
| APPLE OLIFFAC TYPE | 10 |
| Benzylacetat | 50 |
| BERGAMOT IDENTOIL® COLOURLESS | 15 |
| CANTHOXAL | 5 |
| CETALOX 10% IPM | 3 |
| CITRONELLOL 950 | 40 |
| DAMASCENONE TOTAL 1% DPG | 5 |
| DAMASCONE ALPHA 10% DPG | 5 |
| DAMASCONE DELTA 10% DPG | 2 |
| DIMETHYL BENZYL CARBINYL BUTYRATE | 2 |
| DIPROPYLENE GLYCOL | 175 |
| EBANOL | 2 |
| ETHYL DECADIENOATE TRANS CIS-2,4 10% IPM | 2 |
| FLOROSA | 5 |
| FRAMBINON® 10% DPG | 7 |
| GALAXOLIDE 50% IN IPM | 100 |
| GALBEX TYPE BASE | 1 |
| GERANYL ACETATE PURE | 2 |
| HEDIONE | 30 |
| HELIOTROPIN | 10 |
| HEXENYL ACETATE CIS-3 10% DPG | 1 |
| HEXENYL SALICYLATE CIS-3 | 5 |
| HEXYL CINNAMIC ALDEHYDE ALPHA | 70 |
| HEXYL SALICYLATE | 50 |
| HYDROXY CITRONELLAL | 10 |
| ISO E SUPER | 15 |
| ISORALDEINE 70 | 20 |
| LEAFOVERT® | 1 |
| LILIAL | 60 |
| LINALOOL | 60 |
| LINALYL ACETATE | 20 |
| LYRAL | 7 |
| MANZANATE | 2 |
| PHENOXANOL | 7 |
| PHENYLETHYL ALCOHOL | 120 |
| SANDAL MYSORE CORE | 2 |
| SANDRANOL® | 7 |
| STYRALYL ACETATE | 3 |
| TAGETES RCO 10% TEC | 2 |
| TERPINEOL PURE | 20 |
| TETRAHYDROGERANIOL 10% DPG | 5 |
| TONALIDE | 7 |
| VERTOCITRAL 10% DPG | 5 |
| VERTOFIX | 15 |
| **Total** | **1000** |

**Table 16: Composition of Mint flavour (Optamint FLO11158AA)**

| **Ingredients** | **Amount** |
|---|---|
| Spearmint oil *Mentha spicata* | 2 |
| Carvone L | 2 |
| Menthylacetate L | 3 |
| Eucalyptol | 5 |
| Menthone L/ Isomenthone D | 9 |
| Anethole from star anise | 9 |
| Peppermint oil *Mentha piperita* | 10 |
| Menthol racemic | 10 |
| Peppermint oil *Mentha arvensis* | 20 |
| Menthol L | 30 |
| **Total** | **100** |

### Formulation Examples 6 to 19

Formulations (compositions) comprising compounds according to the described invention. (Emulsions, deos, hair conditioner)
**6.** Skin lightening day cream o/w
**7:** Skin soothing lotion
**8:** Anti acne balm
**9:** Antibacterial body lotion, sprayable
**10:** Sunscreen lotion (o/w, broadband protection)
**11** w/o night cream
**12:** Anti dandruff shampoo
**13:** Deo pump spray
**14:** Barrier repair cream
**15:** Antiperspirant/deodorant roll-on
**16:** Emulsion with UV-A/B-broadband protection
**17:** Sun spray with UV-A/B-broadband protection with low oil content
**18:** Scalp soothing hair conditioner with UV-B/UV-A protection, rinse off
**19:** Hair conditioner, leave on

| **Ingredients** | **INCI** | **% BY WEIGHT / FORMULATION EXAMPLE** | | | | | | | | | | | | | |
|---|---|---|---|---|---|---|---|---|---|---|---|---|---|---|---|
| | | 6 | 7 | 8 | 9 | 10 | 11 | 12 | 13 | 14 | 15 | 16 | 17 | 18 | 19 |
| Jasmol | 2-benzlheptanol | 0.1 | 0.2 | 0.2 | 0.1 | 0.1 | 0.1 | 0.1 | 0.2 | 0.2 | 0.1 | 0.1 | 0.05 | 0.1 | 0.1 |
| 1-Dodecanol | Lauryl Alcohol | 0.4 | 0.8 | 0.8 | 0.4 | 0.4 | 0.4 | 0.4 | 0.8 | 0.8 | 0.2 | | 0.05 | 0.4 | 0.4 |
| 1,2-Decanediol | Decylene Glycol | | | 0.2 | | | | 0,4 | 0.2 | 0,2 | | | 0.1 | | |
| 2-Phenoxyethyl Alcohol | Phenoxyethanol | | | | | | | 0,4 | 0.8 | 0,8 | | | 0.2 | | |
| Abil 350 | Dimethicone | 0.5 | 2.0 | 1.0 | | | | | | 0.5 | | 0.3 | | 0.1 | |
| Allantoin | Allantoin | | 0.2 | 0.1 | | | | | | 0.25 | | | | | |
| Aloe Vera Gel Concentrate 10/1 * | Water (Aqua), Aloe Barbadensis Leaf Juice | | | 3.0 | | | 3.0 | 0,5 | | | | | | | |
| Alugel 34 TH | Aluminium Stearate | | | | | | 1.0 | | | | | | | | |
| Azelaic Acid | Azelaic Acid | | | 5.0 | | | | | | | | | | | |
| Butylene Glycol | Butylene Glycol | | | | | | | | 3,0 | | | 3.0 | | | |
| Carbopol Ultrez-10 | Carbomer | | 0.1 | | | 0.2 | | | | | | 0.2 | | | |
| Ceramide BIO* | Cetylhydroxyproline Palmitamide | | 0.1 | | | | | | | 0.5 | | | | | |
| Cetiol OE | Dicaprylyl Ether | | | 4.0 | | | | | | | | | | | |
| Cetiol SB 45 | Butyrospermum Parkii (Shea Butter) | | | 1.0 | | | | | | | | | | | |
| Citric Acid 10% sol. | Citric Acid | | 0.4 | | | | | 0.3 | 0.1 | | | 0.3 | | | |
| Comperlan 100 | Cocamide MEA | | | | | | | 0.5 | | | | | | | |
| Crinipan AD | Climbazole | | | | | | | 0.5 | | | | | | | |
| Dehyquart A CA | Cetrimonium Chloride | | | | | | | | | | | | | 0,5 | 0.2 |
| Dehyquart SP | Quaternium-52 | | | | | | | | | | | | | 4,0 | 2.0 |
| Dihydroxyacetone | Dihydroxyacetone | | | | | | | | 5.0 | | | | | | |
| Dow Corning 246 Fluid | Cyclohexasiloxane and Cyclopentasilox-ane | | | | | 2.0 | | | | | | | | | |
| Dow Corning 345 Fluid | Cyclomethicone | | | | 0.5 | | | | | | | | | | |
| D-Panthenol | Panthenol | | | 1.0 | | | | | | | | | | | |
| Dracorin® CE | Glyceryl Stearate Citrate | 2,5 | | | | | | | 5.0 | 1.5 | | | | 1.0 | 1.0 |
| Dracorin® GOC | Glyceryl Oleate Citrate. Caprylic/Capric Triglyceride | 0.5 | | | 2.0 | | | | | | | | | | |
| Drago-Beta-Glucan | Water (Aqua), Butylene Glycol. Glycerin. Avena Sativa (Oat), Kernel Extract | 0.3 | | | | | | | | | | | | | |
| Dragoderm® | Glycerin. Triticum Vulgare (Wheat) Gluten, Water (Aqua) | | | | | | | 2.0 | | | | | | | |
| Drago-Calm | Water, Glycerin. Avena Sativa (Oat) Kernel Extract | | | | 1.0 | | | | 1,0 | | | | | | 2.0 |
| Dragosan W/O P* | Sorbitan Isostearate, Hydrogenated Castor Oil, Ceresin, Beeswax (Cera Alba) | | | | | | 6.0 | | | | | | | | |
| Dragosantol® 100* | Bisabolol | 0.2 | | | 0.1 | 0.3 | 0.2 | | | | 0.1 | 0.1 | | | |
| Dragoxat® 89 | Ethylhexyl Ethylisononan-oate | | | | | | | | | 2.0 | | | | | |
| EDETA BD | Disodium EDTA | | | | | 0.1 | | | 0.1 | | | 0.1 | | 0.1 | |
| Emulsiphos® | Potassium Cetyl Phosphate, Hydrogenated Palm Glycerides | | 2.0 | 2.0 | | 1.5 | | | | 2.0 | | 1.5 | | | |
| Ethanol 96 % | Ethanol | | | | | | | | 15,0 | | 30.0 | | 13.0 | | |
| Extrapone® Green Tea GW | Glycerin. Water (Aqua). Camellia Sinensis Leaf Extract | | 0.2 | | | | | | | | | | | 0.7 | |
| Extrapone® Rosemary GW | Glycerin. Water (Aqua). Rosmarinus officinalis (Rosemary) Leaf Extract | | 0.3 | | | | | | | 0.5 | | | | | |
| Extrapone® Witch Hazel Distillate colourless | Propylene Glycol. Hamamelis Virginiana (Witch Hazel) Water, Water (Aqua). Hamamelis Virginiana (Witch Hazel) Extract | | | | | | 1.0 | | | | | | | | |
| Farnesol | Farnesol | | | | | | | | | | 0.5 | | | | |
| Perfume oil 836720 | Fragrance | 0.1 | 0,3 | 0.2 | 0,3 | 0.4 | 0,4 | 0.5 | 1,0 | 0.1 | 1,5 | 0.1 | 0,5 | 0.5 | 0,5 |
| Frescolat® MGA | Menthone Glycerol Acetal | 0.5 | | | | 0.3 | | | | | | | | | |
| Frescolat®ML cryst. | Menthyl Lactate | | | 0.8 | | | | | | | 0.2 | | | | |
| Frescolat®X-COOL | Menthyl Ethylamido Oxalate | | | | | | | | | | | 1.0 | | | |
| Genapol LRO liquid | Sodium Laureth Sulfate | | | | | | | 37.0 | | | | | | | |
| Glycerol 85 % | Glycerin | 3.0 | 2.0 | 4.0 | | 4.7 | 2.0 | | 1.5 | 3.0 | | | | | |
| Glyceryl Stearate | Glyceryl Stearate | | 2.0 | | | | | | | 2.0 | | 2.0 | 4.0 | | |
| Hydrolite®-5 | Pentylene Glycol | | | | 5.0 | | | | 3.5 | | | | | | |
| Hydroviton® 24 | Water, Glycerin, Sodium Lactate, TEA Lactate, Serine, Lactic Acid, Urea, Sorbitol, Sodium Chloride, Lauryl Diethylenediaminoglycine, Lauryl Aminopropyl-glycine, Allantoin | | | | | | | | | 1.0 | | | | | |
| Hydroviton® PLUS | Water, Pentylene Glycol, Glycerin, Fructose, Urea, Citric Acid, Sodium Hydroxide, Maltose, Sodium PCA, Sodium Chloride, Sodium Lactate, Trehalose, Allantoin, Sodium hyaluronate, Glucose | | | 1.0 | | | | | | | | | 1.0 | | |
| Irgasan DP 300 | Triclosan | | | | | | | | | | 0.3 | | | | |
| Isoadipate® | Diisopropyl Adipate | | | | | | | | | | | | 1.0 | | |
| Isodragol® | Triisononanoin | | 2.0 | | | | | | | 3.0 | | | | | |
| Isopropyl Palmitate | Isopropyl Palmitate | 4.0 | | | | | | | 4.0 | | | | | | |
| Karion F | Sorbitol | | | | | | 2.0 | | | | | | | | |
| Keltrol RD | Xanthan Gum | 0.2 | 0.1 | | | 0.2 | | | 0.3 | | | 0.2 | | | |
| Lanette 16 | Cetyl Alcohol | 1.0 | | | | | | | 1.0 | | | 1.2 | | | |
| Lanette E | Sodium Cetearyl Sulfate | | | | | | | | | | | 0.7 | | | |
| Lanette O | Cetearyl Alcohol | | 3.0 | | | 1.0 | | | | 2.0 | | | | | |
| Lara Care A-200 | Galactoarabinan | | | 0.3 | | | | | | | | | | 0.5 | 0.1 |
| Locron L | Aluminum Chlorohydrate | | | | | | | | 15.0 | | | | | | |
| Magnesium Chloride | Magnesium Chloride | | | | | | 0.7 | | | | | | | | |
| Merquat 550 | Polyquaternium-7 | | | | | | | 0.5 | | | | | | | |
| NaOH 10% sol. | Sodium Hydroxide | | | | | | | | | 0.3 | | | | | |
| Natrosol 250 HHR | Hydroxyethylcellulose | | | | | | | | | | 0.3 | | | | |
| Neo Heliopan® 357 | Butyl Methoxydibenzoylmethane | | | | | 1.0 | | | | | | | | | |
| Neo Heliopan® AP (10 % as sodium salt) | Disodium Phenyl Dibenzimidazole Tetrasulfonate | | | | | 10.0 | | | | | | 22. 0 | | | |
| Neo Heliopan® AV | Ethylhexyl Methoxy-cinnamate | 5.0 | | | | 3.0 | | | | | | | 25.0 | | |
| Neo Heliopan® E1000 | Isoamyl p-Methoxycinnamate | | | | | | | | | | | | | | |
| Neo Heliopan® HMS | Homosalate | | | | | | | | | | | 5.0 | | | |
| Neo Heliopan® Hydro (15 % as sodium salt) | Phenyl benzimidazole Sulfonic Acid | | | | | 6.7 | | | | | | | | | |
| Neo Heliopan® MBC | 4-Methylbenzylidene Camphor | | | | | 1.5 | | | | | | | 33.3 | | |
| Neo Heliopan® OS | Ethylhexyl Salicylate | | | | | 5.0 | | | | | | | | | |
| Neo PCL wssl. N | Trideceth-9. PEG-5 Ethylhexanoate, Water | | 1.0 | | | | | | | | | | | | |
| Neutral Oil | Caprylic/Capric Triglyceride | 6.0 | | | 4.0 | 2.0 | | | 6.0 | 10.0 | | 2.0 | | 1.0 | |
| Paraffin Oil | Mineral Oil | | | | 4.0 | | | | | | | | | | |
| PCL Liquid 100 | Cetearyl Ethylhexoate | 3.0 | 5.0 | | 7.0 | | 12. 0 | | 3.0 | | | 3.0 | | 0.3 | |
| PCL Solid | Stearyl Heptanoate, Stearyl Caprylate | | 2.0 | | | | | | | | | | | 3.0 | |
| Pemulen TR-2 | Acrylates/C10-30 Alkyl Acrylate Crosspolymer | | | 0.2 | 0.2 | | | | | | | | | | |
| Polymer JR 400 | Polyquaternium-10 | | | | | | | | | | | | | | 0.1 |
| Propylene Glycol | Propylene Glycol | | 5.0 | | | | | | | | | | 0.8 | | 0.8 |
| Retinyl Palmitate in Oil | Retinyl Palmitate | | | | | | 0.2 | | | | | | | | |
| Sepigel 305 | Polyacrylamide. C13-14 Isoparaffin, Laureth-7 | | | | | | | | 1.0 | | | | | | |
| Sodium Benzoate | Sodium Benzoate | 0.1 | | | | | | | | | | | | | |
| Sodium Chloride | Sodium Chloride | | | | | | | 1.0 | | | | | | | |
| Sodium Hydroxide (10% sol.) | Sodium Hydroxide | | 0.3 | 0.4 | 0.4 | | | | | | | 2.8 | | | |
| Solubilizer 611674 | PEG-40 Hydrogenated Castor Oil. Trideceth-9, Water (Aqua) | | | | | | | | | | 2.0 | | | | |
| Sun Flower Oil | Helianthus Annuus (Sunflower) Seed Oil | | | | | | 5.0 | | | | | | | | |
| Sweet Almond Oil | Prunus dulcis | | | | | | 5.0 | | | | | | | | |
| SymCalmin® | Pentylene Glycol. Butylene Glycol. Hydroxyphenyl Propamidobenzoic Acid | | 2.0 | | | | | | | 1.0 | | | | | |
| SymDeo® B125 | 2-Methyl 5-Cyclohexylpentanol | | | | | | | | | | 0.5 | | | | |
| SymDeo® MPP | Dimethyl Phenylbutanol | | | | 0.5 | | | | 1.0 | | | | | | |
| Symdiol®68 | 1,2-Hexanediol. Caprylylglycol. | | | | | | | | 1.0 | | | 0.5 | | | |
| Symdiol®68T | 1,2-Hexanediol. Caprylylglycol. Tropolone | 0.5 | | | | | | | | | | | | | |
| SymMatrix®* | Maltodextrin. Rubus Fruticosus (Blackberry) Leaf Extract | | 0.1 | | | 0.3 | 1.0 | | | | | | | | |
| SymMollient® WS | | | | | | | | | | | | | | | 1.0 |
| SymMollient®S | Cetearyl Nonanoate | | | | | | | | | | | 1.5 | | | |
| SymOcide® PS | Phenoxyethanol. Decylene Glycol. 1,2-Hexanediol | | | | | | | | | | | | | 1.0 | |
| SymRelief® 100 | Bisabolol, Zingiber Officinale (Ginger) Root Extract | | | | 0.1 | | | | | | | | | | |
| SymRepair® 100 | Hexyldecanol. Bisabolol, Cetyl-hydroxyproline Palmitamide, Stearic Acid, Brassica Campestris (Rapeseed) Sterols | | | | | | | | | 2.0 | | | | | |
| SymSave® H | Hydroxyacetophenone | | | | | | | 0.8 | | | | | | | |
| SymSitive® 1609 | Pentylene Glycol. 4-t-Butylcyclohexanol | | 1.5 | | | | | | | | | 0.5 | | | |
| SymSol®PF3 * | Water, Pentylene Glycol, Sodium Lauryl Sulfoacetate, Sodium Oleoyl Sarcosinate, Sodium Chloride, Disodium Sulfoacetate, Sodium Oleate, Sodium Sulfate | | | | | | | | | | | | | | 1.5 |
| SymTriol® | Caprylyl Glycol, 1,2-Hexanediol, Methyl benzyl Alcohol | | | | | | | | | | | | | | 1.0 |
| SymVital® AgeRepair | Zingiber Officinale (Ginger) Root Extract | 0.1 | | | | | 0.1 | | | | | | | | |
| SymWhite® 377 | Phenylethyl Resorcinol | 0.5 | | | | | | | | | | 0.5 | | | |
| Tego Betain L7 | Cocamidopropyl Betaine | | | | | | | 6.0 | | | | | 1.0 | | |
| Tegosoft PC 31 | Polyglyceryl 3-Caprate | | | | | | | | | 0.3 | | | | | |
| Tegosoft TN | C12-15 Alkyl Benzoate | | | 5.0 | | 5.0 | | | | | | | | | |
| Tocopherol Acetate | Tocopheryl Acetate | | | 0.5 | | 0.5 | 3.0 | | | 0.3 | | 0.5 | | | |
| Triethanolamine. 99% | Triethanolamine | | | | | 0.5 | | | | | | | | | |
| Water (demineralized) | Water (Aqua) | ad 100 | | | | | | | | | | | | | |
| Zirkonal L 450 | Aluminium Zirconium Pentachloro-hydrate (40 % aqueous solution) | | | | | | | | | | 37.0 | | | | |

### Formulation examples 20 to 35

### Example 20: Syndet antimicrobial soap bar

| **Ingredients** | **INCI Name** | **% by weight** | % **by weight** |
|---|---|---|---|
| Zetesap 813 A | Disodium Lauryl Sulfosuccinate, Sodium Lauryl Sulfate, Corn Starch, Cetearyl Alcohol, Paraffin, Titanium Dioxide | 91.0 | 91.5 |
| Amphotensid GB 2009 | Disodium Cocoamphodiacetate | 6.0 | 6.0 |
| Allantoin | Allantoin | 1.0 | 1.0 |
| Fragrance | Fragrance | 1.0 | 1,5 |
| Thymol | Thymol | 0.05 | 0.1 |
| Jasmol | 2-benzlheptanol | 0.1 | 0.1 |
| 1-Dodecanol | Lauryl Alcohol | 0.4 | 0.4 |
| 1,2-Decanediol | Decylene Glycol | 0.1 | |
| 2-Phenoxyethyl Alcohol | Phenoxyethanol | 0.4 | |

### Example 21: Syndet antimicrobial soap bar with Triclocarban

| **Ingredients** | **INCI Name** | **% by weight** | **% by weight** |
|---|---|---|---|
| Fenopon AC-78 | Sodium Cocoyl Isethionate | 20.0 | 20.0 |
| Natriumlaurylsulfoacetate | Sodium Lauryl Sulfoacetate | 16.0 | 16.0 |
| Paraffin | Paraffin | 19.0 | 19.0 |
| Wax, microcrystalline | Microcrystalline Wax | 1.0 | 1.0 |
| Corn Starch | Corn Starch | 8.0 | 8.0 |
| Coconut acid | Coconut acid | 2.0 | 2.0 |
| Lauric acid diethanol amide | Lauramide DEA | 2.0 | 2.0 |
| Dextrin | Dextrin | 21.0 | 21.0 |
| Lactic acid, 88% | Lactic Acid | 1.0 | 1.0 |
| Disinfecting agent | Triclocarban | 0.3 | 0.1 |
| Thymol | Thymol | 0.05 | 0.15 |
| Water | Water | Ad 100 | Ad100 |
| Fragrance | Fragrance | 1.0 | 1.0 |
| Jasmol | 2-benzlheptanol | 0.1 | 0.1 |
| 1-Dodecanol | Lauryl Alcohol | 0.4 | 0.4 |
| 1,2-Decanediol | Decylene Glycol | - | 0.1 |
| 2-Phenoxyethyl Alcohol | Phenoxyethanol | - | 0.4 |

### Example 22: Antimicrobial toilet soap bar

| **Ingredients** | **% by weight** | **% by weight** |
|---|---|---|
| Sodium soap from tallow | 60.0 | 60.0 |
| Sodium soap from palm oil | 27.0 | 27.0 |
| Glycerol | 2.0 | 2.0 |
| Sodium Chloride | 0.5 | 0.5 |
| 1-Hydroxyethane-1,1-diphosphonic acid, tetrasodium salt | 0.3 | 0.3 |
| Alpha-Tocopherol | 0.1 | 0.1 |
| Pigment Yellow 1 | 0.02 | 0.02 |
| Water | Ad 100 | Ad 100 |
| Fragrance | 3.0 | 3.0 |
| Thymol | 0.05 | 0.1 |
| Jasmol (2-benzypheptanol) | 0.2 | 0.1 |
| 1-Dodecanol (Lauryl Alcohol) | 0.8 | 0.4 |
| 1,2-Decanediol (Decylene Glycol) | - | 0.1 |
| 2-Phenoxyethyl Alcohol (Phenoxyethanol) | - | 0.4 |

### Example 23: Antimicrobial curd soap bar

| **Ingredients** | **% by weight** | **% by weight** |
|---|---|---|
| Sodium soap from tallow | 60.0 | 60.0 |
| Sodium soap from palm oil | 10.0 | 10.0 |
| Sodium Chloride | 0.5 | 0.5 |
| Ethylendiamin tetra acetic acid, tetrasodium salt | 0.3 | 0.3 |
| Water | Ad 100 | Ad 100 |
| Thymol | 0.05 | 0.15 |
| Fragrance | 2.5 | 2.5 |
| Jasmol (2-benzypheptanol) | 0.2 | 0.1 |
| 1-Dodecanol (Lauryl Alcohol) | 0.8 | 0.4 |
| 1,2-Decanediol (Decylene Glycol) | - | 0.1 |
| 2-Phenoxyethyl Alcohol (Phenoxyethanol) | - | 0.4 |

### Example 24: Liquid soap, transparent

| **Ingredients** | **INCI Name** | **% by weight** | **% by weight** |
|---|---|---|---|
| Tagat O 2 | PEG-20 Glyceryl Oleate | 2.5 | 2.5 |
| Cocosfettsäure diethanolamid | Cocamide DEA | 5.0 | 5.0 |
| Abil B 8842 | Cyclomethicone | 0.5 | 0.5 |
| Sodium laurylethersulfate, 28% | Sodium Laureth Sulfate | 35.0 | 35.0 |
| Tego-Betaine L7 | Cocamidopropyl Betaine | 5.0 | 5.0 |
| Soap, 25% | Coconut acid, Potassium salt, Potassium Oleate | 20.0 | 20.0 |
| Water | Water | Ad 100 | Ad 100 |
| Preservative | DMDM Hydantoin | 0.1 | 0.05 |
| Fragrance | Fragrance | 0.4 | 0.4 |
| Jasmol | 2-benzlheptanol | 0.2 | 0.1 |
| 1-Dodecanol | Lauryl Alcohol | 0.8 | 0.4 |
| 1,2-Decanediol | Decylene Glycol | 0.2 | 0.1 |
| 2-Phenoxyethyl Alcohol | Phenoxyethanol | 0.8 | - |

### Example 25: Syndet soap, liquid

| **Ingredients** | **INCI Name** | **% by weight** | **% by weight** |
|---|---|---|---|
| Elfan OS 46 | Sodium Olefin C14-C16 Sulfonate | 35.5 | 35.5 |
| Armoteric LB | Lauryl Betaine | 8.0 | 8.0 |
| Elfan SG | | 10.0 | 10.0 |
| Elfacos GT 282 L | Talloweth-60 Myristyl Glycol | 3.0 | 3.0 |
| PCL-Liquid 100 | Cetearyl Ethylhexanoate | 4.0 | 4.0 |
| Water | Water | Ad 100 | Ad 100 |
| SymSave H | Hydroxyacetophenone | 0.8 | 0.8 |
| Fragrance | Fragrance | 0.4 | 0.4 |
| Jasmol | 2-benzlheptanol | 0.2 | 0.1 |
| 1-Dodecanol | Lauryl Alcohol | 0.8 | 0.4 |
| 1,2-Decanediol | Decylene Glycol | 0.2 | 0.1 |
| 2-Phenoxyethyl Alcohol | Phenoxyethanol | 0.8 | - |

### Example 26: Body wash

| **Ingredients** | **INCI Name** | **% by weight** | **% by weight** |
|---|---|---|---|
| Lumerol K 28 | Disodium Laureth Sulfosuccinate, Cocamidopropyl Betaine, Magnesium Lauryl Sulfate | 33.0 | - |
| Amphotensid B 4 | Cocamidopropyl Betaine | 10.0 | - |
| Perlglanzmittel GM 4055 | MIPA-Pareth-25 Sulfate, Glycol Stearate | 4,0 | - |
| Sodium Chloride | Sodium Chloride | 2.0 | - |
| Avocado oil | Persea Gratissima (Avocado) Oil | 3.0 | 3.0 |
| SymSave H | Hydroxyacetophenone | 0.8 | 0.8 |
| Water | Water | Ad 100 | Ad 100 |
| Fragrance | Fragrance | 0.5 | - |
| Jasmol | 2-benzlheptanol | 0.2 | 0.1 |
| 1-Dodecanol | Lauryl Alcohol | 0.8 | 0.4 |
| 1,2-Decanediol | Decylene Glycol | 0.2 | 0.1 |
| 2-Phenoxyethyl Alcohol | Phenoxyethanol | 0.8 | - |

### Example 27: Body wash with Triclosan

| **Ingredients** | **INCI Name** | **% by weight** | **% by weight** |
|---|---|---|---|
| Texapon N 25 | Sodium Laureth Sulfate | 37.5 | - |
| Lamepon S | Potassium Cocoyl Hydrolyzed Collagen | 28.0 | - |
| Lamesoft LMG | Hydrogenated Tallow Glycerides, TEA-Cocoyl Hydrolyzed Collagen | 5.0 | - |
| Lamesoft 156 | Glyceryl Laurate, TEA-Cocoyl Hydrolyzed Collagen | 5.0 | - |
| Sodium Chloride | Sodium Chloride | 1.7 | - |
| Irgasan DP 300 | Triclosan | 0.5 | 0.5 |
| SymSave H | Hydroxyacetophenone | 0.8 | 0.8 |
| Water | Water | Ad 100 | Ad 100 |
| Fragrance | Fragrance | 0.3 | - |
| Jasmol | 2-benzlheptanol | 0.05 | 0.1 |
| 1-Dodecanol | Lauryl Alcohol | 0.2 | 0.4 |
| 1,2-Decanediol | Decylene Glycol | 0.04 | 0.1 |
| 2-Phenoxyethyl Alcohol | Phenoxyethanol | 0.2 | - |

### Example 28: Intimate wash

| **Ingredients** | **INCI Name** | **% by weight** | **% by weight** |
|---|---|---|---|
| Tegobetaine HS | Cocamidopropyl Betaine, Glyceryl Laurate | 15.0 | 15.0 |
| Tagat L 2 | PEG-20 Glyceryl Laurate | 2.0 | 2.0 |
| Arlacide G | Chlorhexidine Digluconate | 0.1 | 0.1 |
| Rewoquat B 50 | Benzalkonium Chloride | 0.1 | 0.1 |
| Lactic Acid, 80% | Lactic Acid | 0.1 | 0.1 |
| euxyl® K700 | Potassium Sorbate, Benzyl Alcohol, Phenoxyethanol | 0.3 | 0.5 |
| Water | Water | Ad 100 | Ad 100 |
| Fragrance | Fragrance | 0.2 | 0.2 |
| Jasmol | 2-benzlheptanol | 0.05 | 0.1 |
| 1-Dodecanol | Lauryl Alcohol | 0.2 | 0.4 |
| 1,2-Decanediol | Decylene Glycol | 0.04 | 0.1 |
| 2-Phenoxyethyl Alcohol | Phenoxyethanol | 0.2 | - |

### Example 29: Bar Soap

| **Ingredients** | **INCI** | **% by weight** | **% by weight** |
|---|---|---|---|
| Deionized water | Water | 2.5 | 2.5 |
| Soap bases mix | Sodium tallowates / palmitates | 95.5 | 95.5 |
| Titanium dioxide | Titanium dioxide | 1.0 | 1.0 |
| Fragrance | Fragrance | 0.5 | 0.5 |
| Jasmol | 2-benzlheptanol | 0.05 | 0.1 |
| 1-Dodecanol | Lauryl Alcohol | 0.2 | 0.4 |
| 1,2-Decanediol | Decylene Glycol | 0.04 | 0.1 |
| 2-Phenoxyethyl Alcohol | Phenoxyethanol | 0.2 | - |

### Example 30: Liquid Soap

| **Ingredients** | **INCI** | **% by weight** | **% by weight** |
|---|---|---|---|
| Deionized water | Water | 2.0 | 2.0 |
| Soap bases mix | Sodium tallowates / palmitates | 95.8 | 95.5 |
| Titanium dioxide | Titanium dioxide | 1.0 | 1.0 |
| Fragrance | Fragrance | 0.5 | 0.5 |
| Jasmol | 2-benzlheptanol | 0.05 | 0.1 |
| 1-Dodecanol | Lauryl Alcohol | 0.2 | 0.4 |
| 1,2-Decanediol | Decylene Glycol | 0.04 | 0.1 |
| 2-Phenoxyethyl Alcohol | Phenoxyethanol | 0.2 | - |

### Example 31: Shampoo

| **Ingredients** | **% by weight** | **% by weight** |
|---|---|---|
| Sodium lauryl ether sulfate (e.g. Texapon NSO) | 12 | 12 |
| Cocamidopropyl betaine (e.g. Dehyton K) | 2 | 2 |
| Sodium chloride | 1.4 | 1.4 |
| Citric acid | 1.3 | 1.3 |
| Phenoxyethanol, 4-hydroxy acetophenone | 0.5 | 0.5 |
| Fragrance | 0.3 | 0.3 |
| Jasmol(2-benzlheptanol) | 0.1 | 0.1 |
| 1-Dodecanol (Lauryl Alcohol) | 0.4 | 0.4 |
| 1,2-Decanediol (Decylene Glycol) | - | 0.1 |
| 2-Phenoxyethyl Alcohol (Phenoxyethanol) | 0.4 | 0.2 |
| Panthenol | 0.50 | 0.50 |
| Water (Aqua) | Ad 100 | Ad 100 |
| Polyquaternium-10 | 0.40 | 0.40 |

### Example 32: Shower gel

| **Ingredients** | **INCI** | **% by weight** | **% by weight** |
|---|---|---|---|
| Deionized water | Water | Ad 100 | Ad 100 |
| Plantacare PS 10 | Sodium Laureth Sulfate, Lauryl Glucoside | 20.0 | 20.0 |
| SymSave H | Hydroxyacetophenone | 0.6 | 0.6 |
| Sodium chloride | Sodium Chloride | 1.4 | 1.4 |
| Citric acid monohydrate crystalline | Citric Acid | 1.3 | 1.3 |
| Fragrance | Fragrance | 0.6 | 0.6 |
| Jasmol | 2-benzlheptanol | 0.1 | 0.1 |
| 1-Dodecanol | Lauryl Alcohol | 0.4 | 0.4 |
| 1,2-Decanediol | Decylene Glycol | - | 0.2 |
| 2-Phenoxyethyl Alcohol | Phenoxyethanol | 0.4 | 0.2 |

### Example 33: Shaving foam

| **Ingredients** | **% by weight** | **% by weight** |
|---|---|---|
| Dem. Water | Ad100 | Ad100 |
| Triethanolamine | 4.0 | 4.0 |
| Edenor L2 SM (Stearinic acid, Palmitinic acid) (Cognis) | 5.3 | 5.3 |
| Laureth-23 | 3.0 | 3.0 |
| Stearylalcohol | 0.5 | 0.5 |
| euxyl® K220 (Methylisothiazolinone, Ethylhexylglycerol) | 0.8 | 0.8 |
| Jasmol (2-benzlheptanol) | 0.05 | 0.1 |
| 1-Dodecanol (Lauryl Alcohol) | 0.2 | 0.4 |
| 1,2-Decanediol (Decylene Glycol | 0.04 | 0.1 |
| 2-Phenoxyethyl Alcohol (Phenoxyethanol) | 0.2 | - |
| Sodium lauryl sulfate | 3.0 | 3.0 |
| Extrapone Seaweed (water, propylene glycol, potassium iodide, Fucus Vesiculosus Extract) | 1.0 | 1.0 |
| Dragosantol (Bisabolol, Farnesol) | 0.1 | 0.1 |
| Fragrance | 1.0 | 1.0 |
| propane, butane 4,2 Bar | 4.0 | 4.0 |

### Example 34: Deodorant stick

| **Ingredients** | **% by weight** | **% by weight** |
|---|---|---|
| Sodium stearate | 8.00 | 8.00 |
| PPG-3 Myristyl ether | 70.00 | 70.00 |
| 1,2-propylene glycol | 10.00 | 10.00 |
| 1,1-dimethyl-3-phenylpropanol | 0.20 | 0.25 |
| 2-butyloctanoic acid | 0.20 | 0.20 |
| Fragrance | 0.60 | 0.6 |
| Water | Ad 100 | Ad 100 |
| Jasmol (2-benzlheptanol) | 0.1 | 0.1 |
| 1-Dodecanol (Lauryl Alcohol) | 0.4 | 0.4 |
| 1,2-Decanediol (Decylene Glycol) | - | 0.1 |
| 2-Phenoxyethyl Alcohol (Phenoxyethanol) | - | 0.4 |

### Example 35: Zirconium suspensoid antiperspirant stick

| **Ingredients** | **INCI** | **% by weight** | **% by weight** |
|---|---|---|---|
| PCL Liquid 100 | Cetearyl ethylhexanonate | to 100 | to 100 |
| Silicone Fluid 345 | Cyclomethicone | 10.00 | 10.00 |
| CRODACOL C90 | Cetyl Alcohol | 8.00 | 8.00 |
| SYNCROWAX HGLC | C18-36 Triglyceride | 8.00 | 8.00 |
| CRODAMOL PTC | Pentaerythritol Tetracaprylate/Caprate | 5.00 | 5.00 |
| SYNCROWAX HRC | Tribehenin | 4.00 | 4.00 |
| VOLPO N5 | Oleth-5 | 1.00 | 1.00 |
| Titanium Dioxide | | 1.00 | 1.00 |
| Rezal 36GP | Aluminium Tetrachlorohydrex GLY | 20.00 | 20.00 |
| Dry Flo C | Aluminium Starch Octenyl Succinate | 22.50 | 22.50 |
| Preservative | Phenoxyethanol | 0.8 | 0.8 |
| Fragrance | Fragrance | 0.60 | 0.6 |
| Jasmol | 2-benzlheptanol | 0.1 | 0.1 |
| 1-Dodecanol | Lauryl Alcohol | 0.4 | 0.4 |
| 1,2-Decanediol | Decylene Glycol | - | 0.2 |
| 2-Phenoxyethyl Alcohol | Phenoxyethanol | 0.4 | 0.2 |

## Claims

1. A mixture comprising or consisting of
a) at least one phenyl alkanol selected from the group consisting of 2-benzylheptanol (2-benzylheptan-1-ol), 2-methyl 5-phenylpentanol, dimethyl phenylbutanol (2-methyl-4-phenylbutan-2-ol), dimethyl phenylpropanol (2,2-dimethyl-3-phenylpropanol), 3-methyl-4-phenylbutan-2-ol (2-methyl-4-phenyl-2-butanol) or a mixture thereof,
b) at least one primary, monohydric, linear alcohol with 8 to 12 carbon atoms,
and optionally
c) one or more diols.

2. The mixture of claim 1, wherein the primary, monohydric, linear alcohol (compound b) is selected from the group consisting of undecan-1-ol, decan-1-ol, nonan-1-ol, octan-1-ol, 1-dodecanol or a mixture thereof.

3. The mixture according to any claims 1 or 2, wherein the diol (compound c) is selected from the group consisting of 1,2-pentanediol, 1,2-hexanediol, 1,2-octanediol, 1,2-decanediol, 1,2-dodecanediol, glyceryl monocaprylate (2,3-dihydroxy-propyl octanoate), glyceryl monocaprate (2,3-dihydroxypropyl decanoate), glyceryl monolaurate (2,3-Dihydroxypropyl dodecanoate), ethylhexylglycerin (3-[(2-ethylhexyl)oxy]-1,2-propanediol) or a mixture thereof.

4. The mixture according to any claims 1 to 3, wherein compound a) is 2-benzylheptanol (2-benzylheptan-1-ol), compound b) is 1-dodecanol and compound c) if present, is 1,2-decanediol.

5. The mixture according to any claims 1 to 4, wherein compound c) is a mixture of at least two different 1,2-alkane diols selected from:
e) 1,2-pentanediol and 1,2- octanediol,
f) 1,2-pentanediol and 1,2- decanediol,
g) 1,2-hexanediol and 1,2- octanediol,
h) 1,2-hexanediol and 1,2- decanediol.

6. The mixture according to any claims 1 to 5, wherein the amounts of
i) compound a) is from 1% by weight to 50% by weight,
ii) compound b) is from 50% by weight to 99% by weight,
iii) compound c), if present is from 1% by weight to 50% by weight,
on condition that the amounts add to 100% b.w.

7. Use of a mixture as defined in any preceding claims 1 to 6 to improve the stability of a cosmetic or household formulation.

8. Use according to claim 7, wherein the stability improvement is selected from
(v) emulsion stability,
(vi) foam and viscosity stability,
(vii) solubility of lipophilic components,
(viii) soap bar stability.

9. The use of claim 7 and/or 8, wherein compound a) is 2-benzylheptanol (2-benzylheptan-1-ol) and compound b) is 1-dodecanol.

10. A deodorant preparation comprising or consisting of
viii) from 0 to 90% b.w. ethyl alcohol,
ix) from 0.2 to 3% b.w. PEG 40 hydrogenated castor oil,
x) from 0 to 2% b.w. fragrance,
iv) from 0 to 20% b.w. glycerine,
xi) from 0 to 30% b.w. aluminum chlorohydrate,
xii) from 0 to 80% b.w. cyclomethicone,
xiii) from 0 to 1% b.w. phenoxyethanol,
xiv) from 0 to 5% b.w.fatty acid ester or caprylic capric triglycerides
ix) from 0 to 1% b.w. ethylhexyl glycerine,
x) from 0.1 to 1% b.w. of a mixture as defined in any claims 1 to 8,
xi) water (deionized)
on condition that the amounts add - optionally together with additional ingredients - to 100% b.w.

11. A shampoo preparation comprising or consisting of
i) from 5 to 25% b.w. sodium lauryl ether sulfate,
ii) from 1 to 5% b.w. cocamidopropyl betaine,
iii) from 0 to 5% b.w. plant oil,
iv) from 0 to 10% b.w. fatty acid ester,
v) from 0 to 3% b.w. sodium chloride,
vi) from 0 to 2% b.w. citric acid,
vii) from 0 to 2% b.w. perfume oil,
viii) from 0 to 1.5% b.w. phenoxyethanol, 4-hydroxyacetophenone,
ix) from 0.1 to 2% b.w. of a mixture as defined in any claims 1 to 8,
x) water (deionized),
on condition that the amounts add - optionally together with additional ingredients - to 100% b.w.

12. A soap bar preparation comprising or consisting of
iii) from 50 to 80% b.w. sodium soap from tallow
iv) from 0 to 40% b.w. sodium soap from palm oil
viii) from 0 to 10% b.w. glycerol
ix) from 0 to 2% b.w. sodium chloride
x) from 0 to 0.5% b.w. 1-hydroxyethane-1,1-diphosphonic acid, tetrasodium salt
xi) from 0 to 0.5% b.w. ethylendiamin tetra acetic acid tetrasodium salt
xii) from 0 to 3% b.w. fragrance
xiii) from 0.2 to 2% b.w. mixture according to the invention,
xiv) water (deionized)
on condition that the amounts add - optionally together with additional ingredients - to 100% b.w.

13. A method of
v) stabilizing an emulsion against separation,
vi) reducing the size of water or oil droplets in an emulsion,
vii) improving foam stability and viscosity of a formulation,
viii) preventing soap cracking
by adding of a working amount of a mixture according to any claims 1 to 6 to a cosmetic or household formulation.

## Patentansprüche

1. Eine Mischung, umfassend oder bestehend aus
(a) mindestens ein Phenylalkanol, ausgewählt aus der Gruppe bestehend aus 2-Benzylheptanol (2-Benzylheptan-1-ol), 2-Methyl-5-Phenylpentanol, Dimethylphenylbutanol (2-Methyl-4-phenylbutan-2-ol), Dimethylphenylpropanol (2,2-Dimethyl-3-phenylpropanol), 3-Methyl-4-phenylbutan-2-ol (2-Methyl-4-phenyl-2-butanol) oder einem Gemisch davon,
(b) mindestens einen primären, einwertigen, linearen Alkohol mit 8 bis 12 Kohlenstoffatomen,
und optional
(c) ein oder mehrere Diole.

2. Mischung nach Anspruch 1, wobei der primäre, einwertige, lineare Alkohol (Verbindung b) ausgewählt ist aus der Gruppe bestehend aus Undecan-1-ol, Decan-1-ol, Nonan-1-ol, Octan-1-ol, 1-Dodecanol oder einem Gemisch davon.

3. Mischung nach einem der Ansprüche 1 oder 2, wobei das Diol (Verbindung c) ausgewählt ist aus der Gruppe bestehend aus 1,2-Pentandiol, 1,2-Hexandiol, 1,2-Octandiol, 1,2-Decandiol, 1,2-Dodecandiol, 1,2-Dodecandiol, Glycerylmonocaprylat (2,3-Dihydroxy-Propyl-Octanoat), Glycerylmonocaprat (2,3-Dihydroxypropyldecanoat), Glycerylmonolaurat (2,3-Dihydroxypropyldodecanoat), Ethylhexylglycerin (3-[(2-Ethylhexyl)oxy]-1,2-propandiol) oder eine Mischung davon.

4. Mischung nach den Ansprüchen 1 bis 3, wobei die Verbindung a) 2-Benzylheptanol (2-Benzylheptan-1-ol) ist, die Verbindung b) 1-Dodecanol ist und die Verbindung c), falls vorhanden, 1,2-Decandiol ist.

5. Mischung nach den Ansprüchen 1 bis 4, wobei Verbindung c) ein Gemisch aus mindestens zwei verschiedenen 1,2-Alkandiolen ist, ausgewählt aus:
(a) 1,2-Pentandiol und 1,2-Octandiol,
(b) 1,2-Pentandiol und 1,2-Decandiol,
(c) 1,2-Hexandiol und 1,2-Octandiol,
(d) 1,2-Hexandiol und 1,2-Decandiol.

6. Mischung nach den Ansprüchen 1 bis 5, wobei die Mengen von
(i) Verbindung a) zwischen 1 Gewichtsprozent und 50 Gewichtsprozent liegt,
(ii) Verbindung b) von 50 Gew.-% bis 99 Gew.-% beträgt,
(iii) Verbindung c), falls vorhanden, von 1 Gew.-% bis 50 Gew.-%,
unter der Bedingung, dass die Beträge 100 % des Bruttoinlandsprodukts betragen.

7. Verwendung einer Mischung, wie in den vorangegangenen Ansprüchen 1 bis 6 definiert, zur Verbesserung der Stabilität einer Kosmetik- oder Haushaltsformulierung.

8. Verwendung nach Anspruch 7, wobei die Stabilitätsverbesserung ausgewählt ist aus
(i) Stabilität der Emulsion,
(ii) Schaum- und Viskositätsstabilität,
(iii) Löslichkeit der lipophilen Komponenten,
(iv) Stabilität des Seifenstücks.

9. Verwendung nach Anspruch 7 und/oder 8, wobei Verbindung a) 2-Benzylheptanol (2-Benzylheptan-1-ol) und Verbindung b) 1-Dodecanol ist.

10. Deodorantzubereitung, umfassend oder bestehend aus
(i) von 0 bis 90 Gew.-% Ethylalkohol,
(ii) von 0,2 bis 3 Gew.-% PEG 40 hydriertes Rizinusöl,
(iii) von 0 bis 2 Gew.-% Duftstoff,
(iv) von 0 bis 20 Gew.-% Glyzerin,
(v) von 0 bis 30 Gew.-% Aluminiumchlorhydrat,
(vi) von 0 bis 80 Gew.-% Cyclomethicon,
(vii) von 0 bis 1 Gew.-% Phenoxyethanol,
(viii) von 0 bis 5 Gew.-% Fettsäureester oder Caprylcaprinsäuretriglyceride
(ix) von 0 bis 1 Gew.-% Ethylhexylglycerin,
(x) von 0,1 bis 1 Gew.-% einer Mischung gemäß der Definition in den Ansprüchen 1 bis 8,
(xi) Wasser (entionisiertes)
unter der Bedingung, dass sich die Mengen - optional zusammen mit zusätzlichen Inhaltsstoffen - auf 100 Gew.-% addieren.

11. Shampoo-Zubereitung, umfassend oder bestehend aus
(i) von 5 bis 25 Gew.-% Natriumlaurylethersulfat,
(ii) von 1 bis 5 Gew.-% Cocamidopropylbetain,
(iii) von 0 bis 5 Gew.-% Pflanzenöl,
(iv) von 0 bis 10 Gew.-% Fettsäureester,
(v) von 0 bis 3 Gew.-% Natriumchlorid,
(vi) von 0 bis 2 Gew.-% Zitronensäure,
(vii) von 0 bis 2 Gew.-% Parfümöl,
(viii) von 0 bis 1,5 Gew.-% Phenoxyethanol, 4-Hydroxyacetophenon,
(ix) von 0,1 bis 2 Gew.-% einer Mischung gemäß der Definition in den Ansprüchen 1 bis 8,
(x) Wasser (deionisiert),
unter der Bedingung, dass sich die Mengen - optional zusammen mit zusätzlichen Inhaltsstoffen - auf 100 Gew.-% addieren.

12. Seifenstückzubereitung, umfassend oder bestehend aus
(i) von 50 bis 80 Gew.-% Natriumseife aus Talg
(ii) von 0 bis 40 Gew.-% Natriumseife aus Palmöl
(iii) von 0 bis 10 Gew.-% Glycerin
(iv) von 0 bis 2 Gew.-% Natriumchlorid
(v) von 0 bis 0,5 Gew.-% 1-Hydroxyethan-1,1-diphosphonsäure, Tetranatriumsalz
(vi) von 0 bis 0,5 Gew.-% Ethylendiamintetraessigsäure-Tetranatriumsalz
(vii) von 0 bis 3 Gew.-% Duftstoff
(viii) von 0,2 bis 2 Gew.-% Mischung gemäß der Erfindung,
(ix) Wasser (entionisiertes)
unter der Bedingung, dass sich die Mengen - optional zusammen mit zusätzlichen Inhaltsstoffen - auf 100 Gew.-% addieren.

13. Eine Methode zur
(i) Stabilisierung einer Emulsion gegen Trennung,
(ii) Verringerung der Größe von Wasser- oder Öltröpfchen in einer Emulsion,
(iii) Verbesserung der Schaumstabilität und Viskosität einer Formulierung,
(iv) Verhinderung von Seifenrissen
durch Zugabe einer Arbeitsmenge einer Mischung nach den Ansprüchen 1 bis 6 zu einer Kosmetik- oder Haushaltsformulierung.

## Revendications

1. Un mélange comprenant ou consistant en
(a) au moins un phénylalcanol choisi dans le groupe constitué par le 2-benzylheptanol (2-benzylheptan-1-ol), le 2-méthyl 5-phénylpentanol, le diméthylphénylbutanol (2-méthyl-4-phénylbutan-2-ol), le diméthylphénylpropanol (2,2-diméthyl-3-phénylpropanol), le 3-méthyl-4-phénylbutan-2-ol (2-méthyl-4-phényl-2-butanol) ou un mélange de ceux-ci,
(b) au moins un alcool primaire, monohydrique et linéaire, comportant 8 à 12 atomes de carbone,
et le cas échéant
(c) un ou plusieurs diols.

2. Le mélange selon la revendication 1, **caractérisé en ce que** l'alcool primaire, monohydrique et linéaire (composé b) est choisi dans le groupe constitué par l'undécan-1-ol, le décan-1-ol, le nonan-1-ol, l'octan-1-ol, le 1-dodécanol ou un mélange de ceux-ci.

3. Le mélange selon l'une des revendications 1 ou 2, dans lequel le diol (composé c) est choisi dans le groupe constitué par le 1,2-pentanediol, le 1,2-hexanediol, le 1,2-octanediol, le 1,2-décanediol, le 1,2-dodécanediol, le monocaprylate de glycéryle (2,3-dihydroxy-propyl octanoate), le monocaprate de glycéryle (2,3-dihydroxypropyl décanoate), le monolaurate de glycéryle (2,3-dihydroxypropyl dodécanoate), l'éthylhexylglycérine (3-[(2-éthylhexyl)oxy]-1,2-propanediol) ou un mélange de ceux-ci.

4. Le mélange selon les revendications 1 à 3, dans lequel le composé a) est le 2-benzylheptanol (2-benzylheptane-1-ol), le composé b) est le 1-dodécanol et le composé c), s'il est présent, est le 1,2-décanediol.

5. Le mélange selon les revendications 1 à 4, dans lequel le composé c) est un mélange d'au moins deux 1,2-alcanediols différents choisis parmi :
(a) le 1,2-pentanediol et le 1,2-octanediol,
(b) le 1,2-pentanediol et 1,2-décanediol,
(c) le 1,2-hexanediol et 1,2-octanediol,
(d) le 1,2-hexanediol et 1,2-décanediol.

6. Le mélange selon les revendications 1 à 5, dans lequel les quantités de
(i) le composé a) est de 1 % en poids à 50 % en poids,
(ii) le composé b) est de 50 % en poids à 99 % en poids,
(iii) le composé c), s'il est présent, est de 1 % en poids à 50 % en poids,
à condition que les montants s'additionnent à 100 % de la masse corporelle.

7. Utilisation d'un mélange tel que défini dans les revendications 1 à 6 précédentes pour améliorer la stabilité d'une formulation cosmétique ou domestique.

8. L'utilisation selon la revendication 7, dans laquelle l'amélioration de la stabilité est choisie parmi
(i) la stabilité de l'émulsion,
(ii) la stabilité de la mousse et de la viscosité,
(iii) la solubilité des composants lipophiles,
(iv) la stabilité du pain de savon.

9. L'utilisation selon la revendication 7 et/ou 8, dans laquelle le composé a) est le 2-benzylheptanol (2-benzylheptan-1-ol) et le composé b) est le 1-dodécanol.

10. Préparation déodorante comprenant ou consistant en
(i) de 0 à 90% en poids d'alcool éthylique,
(ii) de 0,2 à 3 % en poids d'huile de ricin hydrogénée PEG 40,
(iii) de 0 à 2 % de parfum en poids,
(iv) de 0 à 20 % en poids de glycérine,
(v) de 0 à 30 % en poids de chlorhydrate d'aluminium,
(vi) de 0 à 80 % de cyclométhicone en poids corporel,
(vii) de 0 à 1 % en poids de phénoxyéthanol,
(viii) de 0 à 5% en poids d'esters d'acides gras ou de triglycérides capriques capryliques
(ix) de 0 à 1 % en poids d'éthylhexyl glycérine,
(x) de 0,1 à 1% en poids d'un mélange tel que défini dans les revendications 1 à 8,
(xi) de l'eau (déionisée)
à condition que les quantités s'ajoutent - éventuellement avec des ingrédients supplémentaires - à 100 % en poids.

11. Préparation de shampoing comprenant ou consistant en
(i) de 5 à 25% en poids de lauryléthersulfate de sodium,
(ii) de 1 à 5 % en poids de cocamidopropylbétaïne,
(iii) de 0 à 5 % en poids d'huile végétale,
(iv) de 0 à 10 % en poids d'ester d'acide gras,
(v) de 0 à 3 % en poids de chlorure de sodium,
(vi) de 0 à 2 % en poids d'acide citrique,
(vii) de 0 à 2 % en poids d'huile de parfum,
(viii) de 0 à 1,5% en poids de phénoxyéthanol, 4-hydroxyacétophénone,
(ix) de 0,1 à 2% en poids d'un mélange tel que défini dans les revendications 1 à 8,
(x) de l'eau (déionisée),
à condition que les quantités s'ajoutent - éventuellement avec des ingrédients supplémentaires - à 100 % en poids.

12. Préparation de savon comprenant ou consistant en
(i) de 50 à 80% en poids de savon de sodium de suif
(ii) de 0 à 40% en poids de savon de sodium provenant de l'huile de palme
(iii) de 0 à 10% en poids de glycérol
(iv) de 0 à 2% en poids de chlorure de sodium
(v) de 0 à 0,5% en poids d'acide 1-hydroxyéthane-1,1-diphosphonique, sel tétrasodique
(vi) de 0 à 0,5% en poids de sel tétrasodique de l'acide éthylènediaminetétra-acétique
(vii) de 0 à 3 % de parfum en p.c.
(viii) de 0,2 à 2% de mélange en poids selon l'invention,
(ix) de l'eau (déionisée)
à condition que les quantités s'ajoutent - éventuellement avec des ingrédients supplémentaires - à 100 % en poids.

13. Une méthode de
(i) la stabilisation d'une émulsion contre la séparation,
(ii) la réduction de la taille des gouttelettes d'eau ou d'huile dans une émulsion,
(iii) améliorer la stabilité de la mousse et la viscosité d'une formulation,
(iv) la prévention de la fissuration du savon
en ajoutant une quantité de travail d'un mélange selon les allégations 1 à 6 à une formulation cosmétique ou ménagère.
